(19) Europäisches Patentamt
European Patent Office
Office européen des brevets

(11) Publication number: **0 396 841 B1**

(12) **EUROPEAN PATENT SPECIFICATION**

(45) Date of publication of patent specification: **02.02.94**

(51) Int. Cl.[5]: **C07C 69/65**, C07C 69/732, C07C 233/09, C07C 235/28, C07C 259/06, C07C 57/52, C07C 257/04, A61K 31/23, A61K 31/20

(21) Application number: **89401244.2**

(22) Date of filing: **02.05.89**

# (54) Fluorinated arachidonic acid derivatives.

(43) Date of publication of application:
**14.11.90 Bulletin 90/46**

(45) Publication of the grant of the patent:
**02.02.94 Bulletin 94/05**

(84) Designated Contracting States:
**AT BE CH DE ES FR GB GR IT LI LU NL SE**

(56) References cited:
**EP-A- 0 104 468**
**US-A- 3 057 893**

**CHEMICAL AND PHARMACEUTICAL BULLETIN, vol. 35, no. 4, 1987, pages 1666-1669, Tokyo, JP; T. TAGUCHI et al: "Synthesis of 5-fluoroarachidonic acid and its biotransformation to 5-fluoro-12-hydroxyeicosateraenoic acid" & JP-A- 61 277 246**

(73) Proprietor: **MERRELL DOW PHARMACEUTICALS INC.**
**2110 East Galbraith Road**
**Cincinnati Ohio 45215-6300(US)**

(72) Inventor: **Ducep, Jean Bernard**
**29 rue des Alpes**
**F-68280 Sundhoffen(FR)**
Inventor: **Nave, Jean-François**
**4, rue de la Tanche**
**F-67000 Strasbourg(FR)**
Inventor: **Jacobi, Detlef**
**Gustav-Weisstrasse 3**
**D-7640 Kehl am Rhein(DE)**

(74) Representative: **Gillard, Marie-Louise et al**
**Cabinet Beau de Loménie**
**158, rue de l'Université**
**F-75340 Paris Cédex 07 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, vol. 107, no. 7, 17 August 1987, page 685, abstract no. 58491e, Columbus, Ohio, USA; Y. KOBAYASHI et al: "Vinyl fluoride derivatives" & JP-A-61 271 234

CHEMICAL ABSTRACTS, vol. 109, no. 25, 19 December 1988, page 822, abstract no. 230631e, Columbus, Ohio, USA; Y. KOBAYASHI et al: "Preparation of fluoroarachidonic acid derivatives as cyclooxygenase and lipoxygenase inhibitors" & JP-A-63 48 243

CHEMICAL ABSTRACTS, vol. 108, no. 21, 23 May 1988, page 675, abstract no. 186433v, Columbus, Ohio, USA; Y. KOBAYASHI et al: "Preparation of 12-fluoroarachidonic acid derivatives as cyclooxygenase and 5-lipoxygenase inhibitors" &JP-A-62 209 043

ARCHIVES OF BIOCHEMISTRY AND BIOPHYSICS, vol. 263, no. 1, 15 May 1988, pp. 178-190; Y. TANAKA et al: "Biosynthesis of 20,20,20-trifluoroleukotriene B4 from 20,20,20-trifluoroarachidonic acid: A metabolically stable analog of leukotriene B4 and its application to a study of stimulation of leukotriene B4 synthesis by immunoglobulin G 1,2"

## Description

This invention relates to certain fluorinated arachidonic acid derivatives and their pharmaceutical uses.

Lipoxygenases, which are found in various mammalian tissues including the lung, mast cells, platelets, and white cells, are enzymes which oxidize arachidonic acid into hydroperoxyeicosatetraenoic acids (HPETEs) which are in turn reduced to the corresponding hydroxyeicosatetra-enoic acids (HETEs). The lipoxygenases are classified according to the position in the arachidonic acid which is oxygenated. Platelets metabolize arachidonic acid to 12-HETE via a 12-lipoxygenase, while polymorphonuclear leukocytes contain 5- and 15-lipoxygenases which oxidize arachidonic acid to 5-HPETE and 15-HPETE, respectively.

5-HPETE is the precursor of leukotriene $A_4$, an unstable precursor of two distinct groups of leukotrienes. The first of these are the peptido-lipid leukotrienes $LTC_4$ and $LTD_4$ formed sequentially by reaction of $LTA_4$ with glutathione followed by reaction with $\gamma$-glutamyl trans-peptidase to form the cysteinyl-glycine adduct. These compounds account for the biologically active material known as the slow reacting substances of anaphylaxis (SRS-A).

These leukotrienes are potent smooth muscle contracting agents, particularly effective on smooth muscle but also on other tissues. They also promote mucous production, modulate vascular permeability changes and are potent inflammatory agents in human skin. The leukotrienes are potent spasmogens of human trachea, bronchus and lung parenchymal strips. Administered as an aerosol to normal volunteers, leukotrienes have been found to be about 3800 times more potent than histamine itself. *In vitro* studies have shown that antigen challenge of human lung or human mast cells results in the production and release of significant amounts of leukotrienes. For these reasons leukotrienes are thought to be major contributors of the symptoms of asthma and anaphylaxis. The most important compound of the second group of leukotrienes is leukotriene $B_4$, a dihydroxy fatty acid. This compound is a potent chemotactic agent for neutrophils and in addition may modulate a number of other functions of these cells. It also affects other cell types such as lymphocytes and, for example, is thought to inhibit the phytohemagglutinin-induced elaboration of leukocyte inhibitory factor in T-lymphocytes. Leukotriene $B_4$ is also a potent hyperalgesic agent *in vivo* and can modulate vascular permeability changes through a neutrophil-dependent mechanism.

Psoriasis is a human skin disease which affects from about 2 to 6 per cent of the population but fully adequate therapy remains unavailable. One of the earliest events in the development of psoriatic lesions is the recruitment of leukocytes to the skin site. In human psoriatic skin, abnormally high levels of free arachidonic acid and lipoxygenase products are found. Among these, leukotriene $B_4$ has been identified in blister fluid from human psoriatic skin, and when injected into human skin, leukotriene $B_4$ induces a pronounced accumulation of neutrophils at the site of injection. Moreover in humans with stable psoriasis, intralesional injection of 15-(S)-HETE, an inhibitor of 5- and 12-lipoxygenases, produces considerable improvement of psoriatic plates.

The leukotrienes are important mediators of inflammatory diseases through their ability to modulate leukocyte and lymphocyte functions. The presence of the leukotrienes is thought to be responsible for many of the symptoms observed in allergy and rheumatoid arthritis patients.

It has been shown that 12-fluoro and 16-fluoroarachidonic acid derivatives are 5-lipoxygenase inhibitors (See Chem. Abs., 1988, 108 (21), 186 433 v and Chem. Abs., 1988, 109 (25), 230 631 e). The preparation of a family compound, namely 5-fluoroarachidonic acid, is described in Chem. Pharm. Bull., 1987, 35(4), 1666-1669.

Applicants have now discovered a novel class of fluorinated arachidonic acid derivatives which are potent inhibitors of 5-lipoxygenase, the enzyme responsible for the conversion of arachidonic acid to the leukotrienes. These new compounds are useful as antiallergic and anti-inflammatory agents in the treatment of asthma, anaphylaxis, allergy, rheumatoid arthritis, psoriasis, and cardiovascular diseases..

SUMMARY OF THE INVENTION

Fluorinated arachidonic derivatives of formula 1:

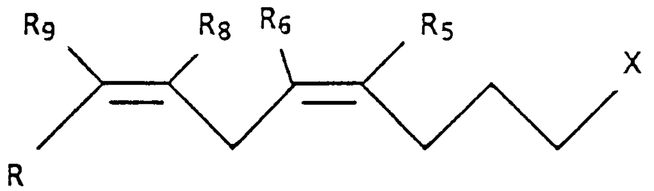

1

wherein

one of $R_5$ and $R_6$ is a fluoro group and the other is a hydrogen or both $R_5$ and $R_6$ individually are a hydrogen;

one of $R_8$ and $R_9$ is a fluoro group and the other is a hydrogen;

X is a C(O)OR′ group wherein R′ is a hydrogen, a straight chain ($C_1$-$C_6$)alkyl group, or

X is a group of the formula $-C(O)OCH_2CH(OR'')CH_2(OR'')$ wherein R″ is a long chain fatty acid residue and wherein $R'''$ is a hydrogen or a long chain fatty acid residue, or

X is a $-C(O)NH_2$ or -C(O)NH(OH) group, or

X is a 1H-tetrazol-5-yl group; and

R is a group of one of the structural formulae

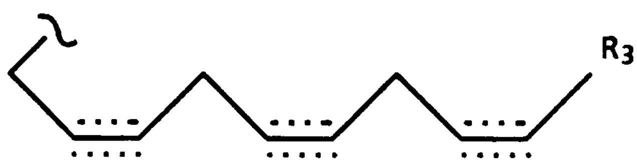

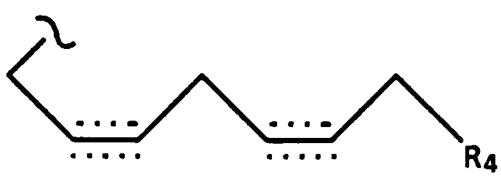

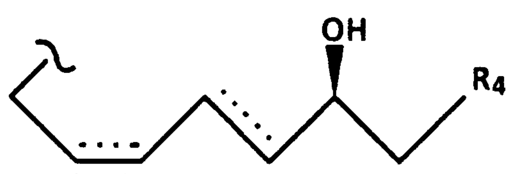

wherein

$R_3$ is a hydrogen or a straight chain ($C_1$-$C_4$)alkyl and $R_4$ is a hydrogen or a straight chain ($C_1$-$C_6$)alkyl and wherein a dotted line indicates an optional double or triple bond

and the pharmaceutically acceptable salts thereof are 5-lipoxygenase inhibitors which have the useful pharmacologic activity as antiallergy and anti-inflammatory agents and are useful for treating, for example, asthma, anaphylaxis, allergy, rheumatoid arthritis, psoriasis, and cardiovascular diseases.

DETAILED DESCRIPTION OF THE INVENTION

The compounds of this invention may be described as 8-fluoro-, 5,8-difluoro-, 9-fluoro-, and 5,9-difluoro-, and 6,9-difluoro- arachidonic acid derivatives.

The R groups of the compounds of this invention may contain one or more double bonds. Any double bonds in the R group of this invention must have the cis configuration except for the double bond at the 13,14 position of the hydroxylated R groups which must be of the trans configuration. Moreover the carbon atom to which the hydroxy group is attached in the hydroxylated R groups, the 15 carbon atom, is chiral. Of those compounds having a hydroxylated R group, applicants prefer those with the S configuration at the carbon atom bearing the hydroxy group.

As is true with many classes of pharmacologically active compounds, certain subclasses are preferred. In the compounds of this invention those of formula 1 wherein X is a $CO_2H$ group and wherein X is a group of the formula $-C(O)OCH_2CH(OR'')CH_2(OR''')$ wherein R'' is a long chain fatty acid residue and wherein R''' is a hydrogen or a long chain fatty acid residue are preferred. Also preferred are those compounds of formula 1 wherein the R group is hydroxylated, especially those hydroxylated R groups having two double bonds. Additionally preferred are those R groups wherein $R_3$ is an ethyl group, especially those having two or three double bonds and which correspond to 5,8,11,14-eicosatetraenoic acid and 5,8,11,14,17-eicosapentaenoic acid.

Those compounds of this invention wherein X is a group of the formula $-C(O)OCH_2CH(OR'')CH_2(OR''')$ are analogs of the naturally occuring arachidonic acid containing lipids from which arachidonic acid is released in mammals. The groups R'' and R''' can be long chain fatty acid residues. Suitable long chain fatty acid residues are those of the naturally occurring saturated and unsaturated fatty acids as well as analogs of these naturally occurring fatty acids. The carbon chains of the naturally occurring fatty acids are usually unbranched, usually contain an even number of carbon atoms, and usually any double bonds are of the cis configuration. Additionally the double bonds of the naturally occurring unsaturated fatty acids are never conjugated. However, the long chain fatty acids of this invention may be branched, may contain an odd number of carbon atoms, may contain conjugated double bonds, and may have trans configuration. Examples of suitable fatty acids are butyric, caproic, caprylic, capric, lauric, myristic, palmitic, stearic, arachidic, lignoceric, oleic, palmit-oleic, linoleic, $\gamma$-linolenic, linolenic, arachidonic 5,8,11,14,17-eicosapentaenoic acids.

The 8-fluoro and 5,8-difluoroarachidonic acid derivatives, that is those compounds of formula 1 wherein X is a COOH group and $R_8$ is a fluoro group, can be prepared by the oxidation of an aldehyde of formula 2

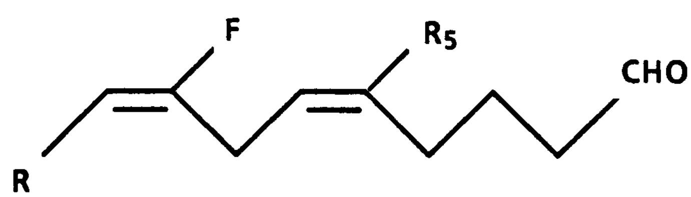

2

wherein R and $R_5$ are as defined in formula 1. The oxidation can be accomplished by, for example, adding an excess of Jones Reagent to a cooled (0 °C) acetone solution of the aldehyde. The reaction mixture is then allowed to react for about 10 to 30 minutes. Isopropanol is then added to consume excess Jones Reagent and the acetone solvent is removed by evaporation on the rotary evaporator. The residue is then mixed with water and the water mixture is extracted with ethyl acetate. After concentration of the ethyl acetate extracts, flash chromatography on silica gel eluting with a mixture of ethyl acetate and benzene (15:85) results in the isolation of the desired carboxylic acid.

The formula 2 aldehydes are prepared by treatment of a bromo or chloro derivative of formula 3

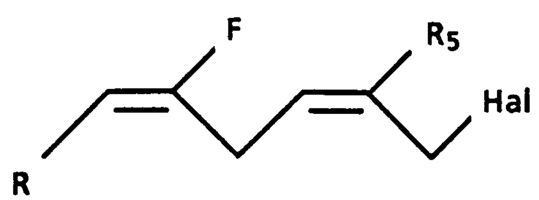

3

wherein Hal is a chloro or bromo group and R and $R_5$ are as defined in formula 1. This can be accomplished by treating a solution of N-allyl-N,N',N''-pentamethylphosphoramide in tetrahydrohydrofuran (THF) with one equivalent of n-butyllithium while maintaining a temperature of about -78 °C for about 1 hour until anion formation is complete. The formula 3 halide is then added to the solution of anion and the temperature is allowed to rise to about 0 °C. The reaction is complete in about 2 hours and the resulting condensation product of formula 3a

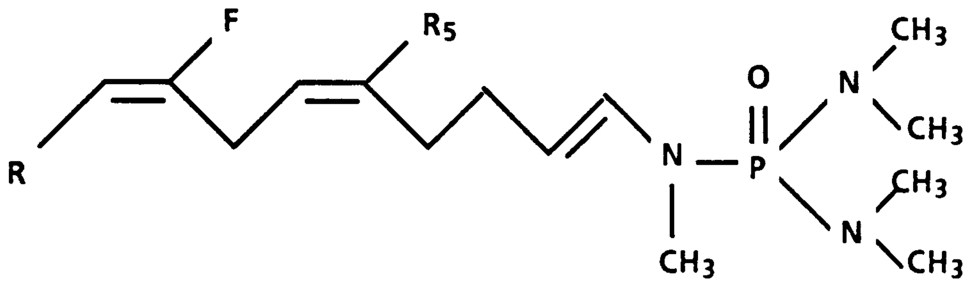

3a

wherein R and $R_5$ are as defined in formula 1, in an ether solvent such as THF or diethyl ether is then subjected to acid catalyzed hydrolysis employing a mild acid such as a dilute mineral acid such as dilute hydrochloric acid. The hydrolysis is complete in about 1 to 2 hours at room temperature. Isolation by solvent removal and purification by chromatography on, for example, silica gel eluting with a 25:75 mixture of ethyl acetate and hexane afforded purified product.

The halide of formula 3 is prepared from the allylic alcohol of formula 4

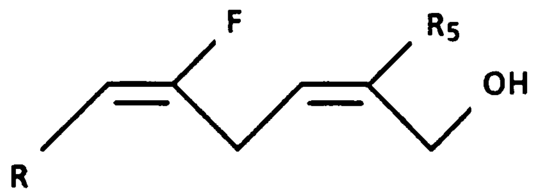

4

wherein R and $R_5$ are as defined in formula 1 in any suitable art known procedure. Applicants have prepared the halide derivatives of formula 3 by treatment of the formula 4 alcohol with a slight (e.g. 20%) excess of 1-bromo-N,N,2-trimethylpropenylamine in a cooled (0 °C) methylene chloride solution. The formula 3 halide derivatives can also be prepared by stepwise conversion of the alcohol, 4, to its mesylate derivative by treatment with methanesulfonic acid chloride in the presence of a proton acceptor such as pyridine or triethylamine. Subsequent treatment of the mesyl derivative with a source of bromide or chloride ion such as a brominated or chlorinated ion exchange resin, for example, Amberlyst® A26, $Br^-$ or $Cl^-$ form, results in the desired halide. The halogenation reaction utilizing Amberlyst® A26 resin will take from 8 to 24 hours when performed in refluxing benzene.

The alcohol of formula 4 is prepared by reduction of the corresponding carboxylic ester of formula 5

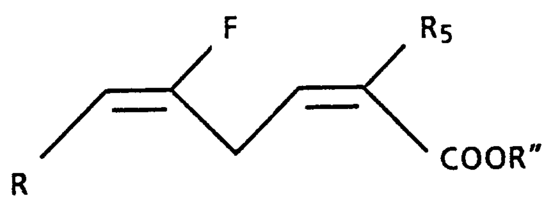

5

wherein R and $R_5$ are as defined in formula 1 and R'' is an alkyl or benzyl group, for example, an ethyl group. The reduction can be carried out in any conventional manner readily known by those skilled in the

art for reducing an ester in the presence of an olefinic bond. Applicants have performed this reduction by treating a solution of an ethyl ester of formula 5 (R'' is ethyl) in an ethereal solvent such as THF with an aluminum hydride reducing agent such as diisobutylaluminum hydride (DIBAL). Such a reaction is typically carried out by adding a solution of DIBAL in hexane to a solution of the ester in the ethereal solvent. After stirring for from about 15 minutes to about 1 hour, preferably about 30 minutes, the reaction is allowed to continue at room temperature for from about 6 to about 24 hours. Addition of methanol to destroy excess reducing agent and ammonium chloride to precipitate the aluminum salts gives a solution of the reduced product. The product is isolated after solvent removal and is purified by flash chromatography on silica gel eluting with, for example, an 8:2 mixute of hexane and ethyl acetate.

The formula 5 ester is prepared from the formula 6 aldehyde

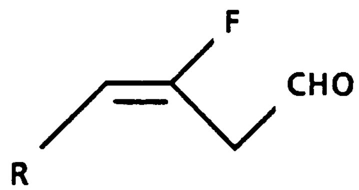

6

wherein R is as defined in formula 1 by reaction with the ylid of triethylphosphono fluoroacetate.

The ylid is formed from the optionally fluorinated phosphonate in the usual way by treatment of the phosphonate with about one molar equivalent of a strong, organic base, preferably lithium diisopropylamide (LDA) formed *in situ* by the reaction of n-butyl lithium and diisopropylamine, at low temperature, typically from about -78 °C to about -25 °C, in a suitable solvent, preferably a solvent or solvent combination known to promote the Wittig reaction such as tetrahydrofuran (THF). Hexamethylphosphorictriamide (HMPA), which is known to promote the Wittig reaction by forming a chelate with the lithium counterion, can advantageously be added. The solution of ylid is then allowed to warm slightly to from about -30 °C to about 0 °C and the appropriate aldehyde is added, preferably dropwise, and allowed to react until formation of the desired condensation product of formula 5 is formed. The product can be isolated by quenching the reaction with a saturated, aqueous solution of ammonium chloride and subsequent removal of the organic solvent by evaporation with a rotary evaporator. The mixture is then extracted with diethyl ether to give the isolated product upon evaporation of the ether solvent. The crude product can be purified by, for example, flash chromatography on silica gel eluting with a mixture of hexane and benzene (9:1).

The structure 6 aldehyde is in turn prepared from the appropriate dithiane of structure 7

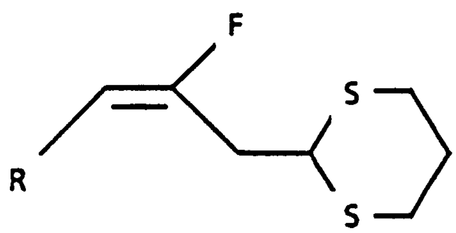

7

wherein R is as defined in formula 1 by hydrolysis in the usual manner. The dithiane is prepared from the appropriate bromo or chloro of structure 8

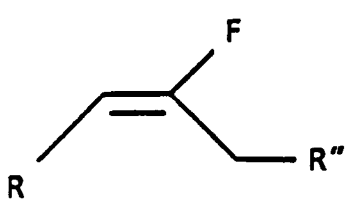

8, R'' = Hal
8a, R'' = OH

wherein R is as defined in formula 1 and wherein Hal is a bromo or chloro group by reaction with the anion of 1,3-dithiane formed by treatment with n-butyl lithium in cooled (i.e. -30 ° C) tetrahydrofuran.

The formula 8 halide is prepared from the corresponding alcohol of formula 8a in any suitable art-known manner. Applicants have transformed the formula 8a alcohol to the formula 8 halide by reaction with one equivalent of 1-bromo-N,N,2-trimethylpropenylamine in a cooled (0 ° C) methylene chloride solution. Alternatively, the formula 7 dithiane derivative can be prepared from an activated derivative of the formula 8a alcohol such as the mesyl or tosyl derivative of the alcohol.

The formula 8a alcohol can be prepared by reduction of the appropriate ester of formula 9

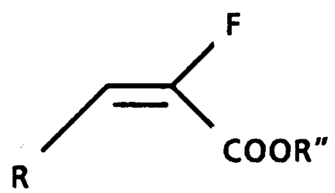

9

wherein R as defined for formula 1 and wherein R″ is a lower alkyl, phenyl, or benzyl group. This reduction can be accomplished in any suitable manner such as by treating a solution of an ethyl ester of formula 9 (R″ = ethyl) in an ethereal solvent such as THF with an aluminum hydride reducing agent such as diisobutylaluminum hydride (DIBAL). Such a reaction is typically carried out by adding a solution of DIBAL in hexane to a solution of the ester in the ethereal solvent. After stirring for from about 15 minutes to about 1 hour, preferably about 30 minutes, the reaction is allowed to continue at room temperature for from about 6 to about 24 hours. Addition of methanol to destroy excess reducing agent and ammonium chloride to precipitate the aluminum salts gives a solution of the reduced product. The product is isolated after solvent removal and is purified by flash chromatography on silica gel eluting with, for example, an 8:2 mixture of hexane and ethyl acetate.

The formula 9 ester is prepared by the condensation of the ylid of the fluorinated phosphonate $(C_2H_5O)_2P(O)CHFCO_2(C_2H_5)$ with an aldehyde of the formula RCHO wherein R is also as defined as above in formula 1. The ylid is formed from the fluorinated phosphonate in the usual way by treatment of the phosphonate with about one molar equivalent of a strong, organic base, preferably lithium diisopropylamide (LDA) formed *in situ* by the reaction of n-butyl lithium and diisopropylamine, at low temperature, typically from about -78 ° C to about -25 ° C, in a suitable solvent, preferably a solvent or solvent combination known to promote the Wittig reaction such as tetrahydrofuran (THF). Hexamethylphosphorictriamide (HMPA), which is known to promote the Wittig reaction by forming a chelate with the lithium counterion, can advantageously be added. The solution of ylid is then allowed to warm slightly to from about -30 ° C to about 0 ° C and the appropriate aldehyde is added, preferably dropwise, and allowed to react until formation of the desired condensation product of formula 9 is formed. The product can be isolated by quenching the reaction with a saturated, aqueous solution of ammonium chloride and subsequent removal of the organic solvent by evaporation with a rotary evaporator. The mixture is then extracted with diethyl ether to give the isolated product upon evaporation of the ether solvent. The crude product can be purified by, for example, flash chromatography on silica gel eluting with a mixture of hexane and benzene (9:1).

The aldehydes of the formula RCHO, i.e., R is a $C_{11}$ carbon chain used to prepare the compounds of this invention can be easily prepared from readily available materials, for example, from the corresponding alcohols by simple oxidation using pyridinium chlorochromate or Collin's reagent in methylene chloride. Many of the alcohols and aldehydes are known. 6-Dodecyn-1-ol is known from J. Chem. Soc., 4363 (1963); (Z)-6-Dodecenal is known from United States Patent Number 4,239,756, granted December 1980; (Z,Z)-3,6-dodecedienal is known from Agric. Biol. Chem., 41, 1481 (1977); and 1-hydroxy-3,6,9-dodecatriyne and (Z,Z,Z)-1-hydroxy-3,6,9-dodecatriene are known from Tetrahedron Letters, 22, 4729 (1981). Olefinic alcohols having the (Z) configuration, for example, can be prepared by Nickel boride with ethylene diamine in methanol or ethanol hydrogenation of the corresponding acetylenic alcohols by the procedure of C. A. Brown and V. K. Ahuja, Chemical Comm. 553 (1973).

The optionally active aldehyde (25) required to prepare those compound of formula 1 wherein the R group has the structural formula:

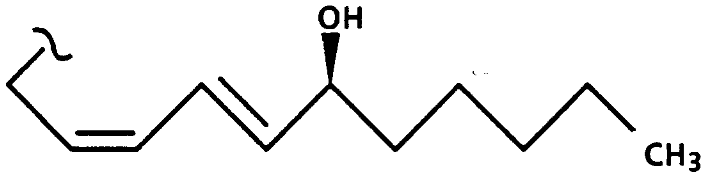

can be prepared from D-arabinose as illustrated in Scheme 3.

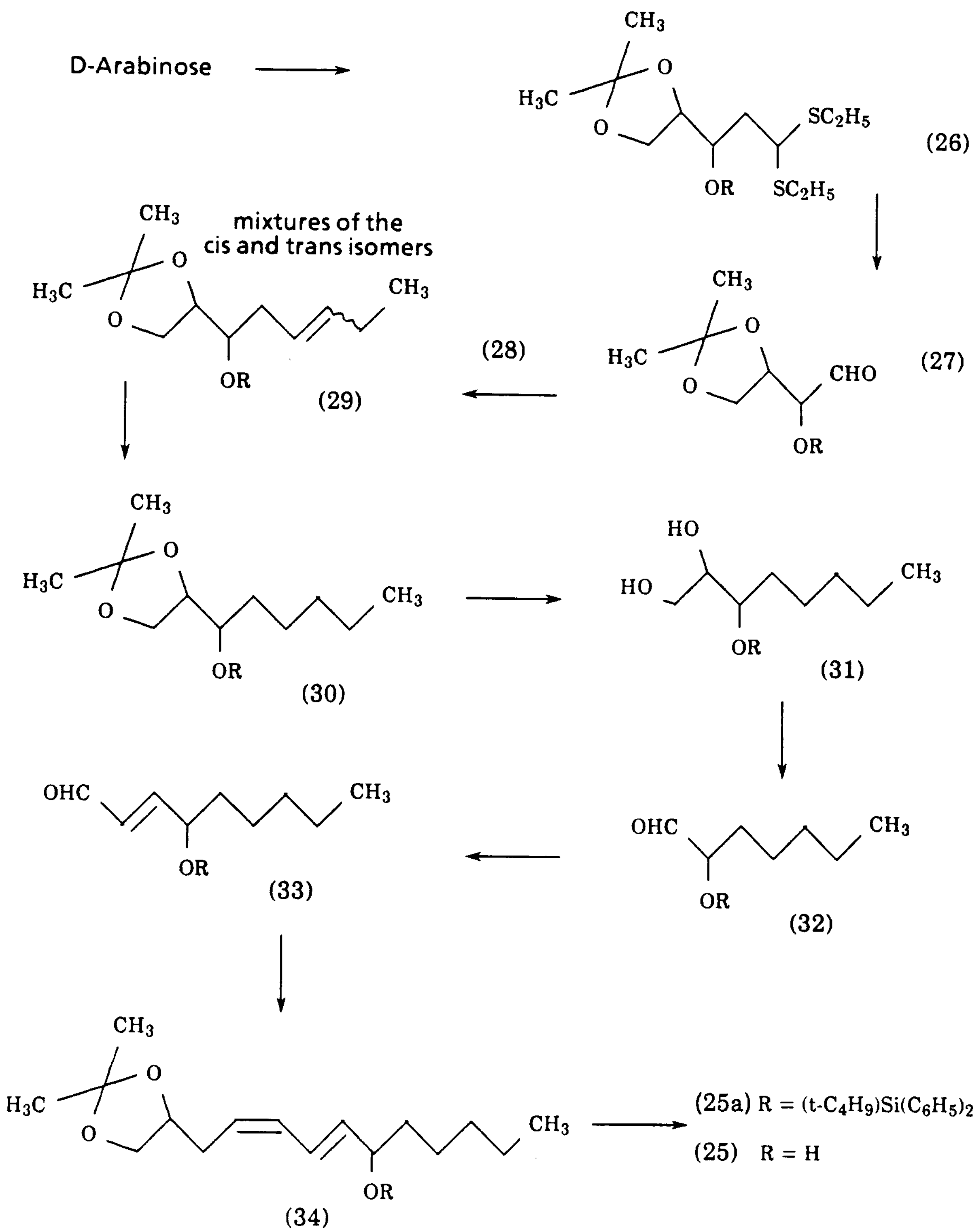


**SCHEME 3**

The thioacetal (26) is first prepared from D-arabinose by the procedure described by M. Wong and G. Gray, J. Amer. Chem. Soc. 100, 3548 (1978). The silyloxy aldehyde (27) is then prepared by reaction of the dithioacetal (26) with mercuric oxide and calcium carbonate in refluxing aqueous acetonitrile. The silyloxy aldehyde (27) is then reacted with the ylid of n-propylbromide and triphenylphosphine (28) formed in the usual manner by reaction with a strong base such as n-butyllithium and potassium t-butoxide in a solvent such as tetahydrofuran. The resulting silyloxyolefin (29) is reduced catalytically with, for example, molecular hydrogen and a palladium on carbon catalyst in ethyl acetate to form the silyloxy compound (30). The

silyloxy compound (30) is converted to the unsaturated aldehyde (33) by the procedure described in G. Just and Z. Wang, Tet. Lett. 26, 2993 (1985) via the diol (31) and the aldehyde (32). Reaction of the unsaturated aldehyde (33) with the ylid of the acetone ketal of 3,4-dihydroxy-iodobutane described by P. DeClercy and R. Mijnheen, Bull. Soc. chem. Belg. 87, 495 (1978) in the usual manner results in the diolefinketal (34). Hydrolysis and sodium metaperiodate oxidation in a manner analogous to that described for the conversion of (30) to (32) give the silyl ether derivative (25a) which upon removal of the silyl group in the usual manner such as by treatment with fluoride ion gives the desired diunsaturated aldehyde (25) wherein the carbon atom bearing the hydroxy group is of the S configuration. Modification of this procedure or chemical modification of the diunsaturated aldehyde can give the other required optically active aldehydes.

In the preparation of the 8-fluoro compounds embraced by formula 1, the synthesis is affected by the following reaction scheme using compounds 6 as starting materials.

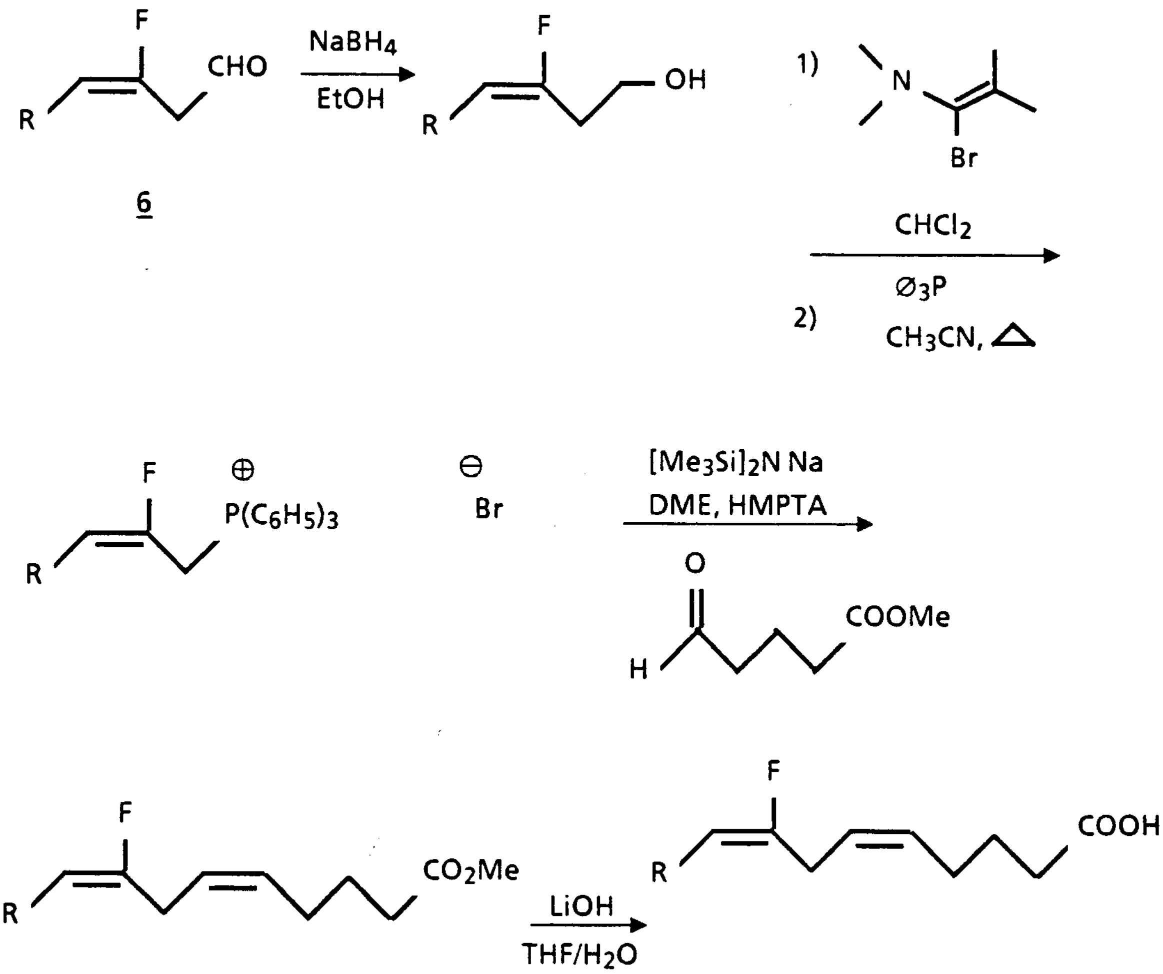


The 5,9-difluoroarachidonic acid derivatives, that is those compounds of formula 1 wherein X is a COOH group and $R_5$ and $R_9$ are both a fluoro group, can be prepared by the oxidation of the corresponding alcohol of formula 10

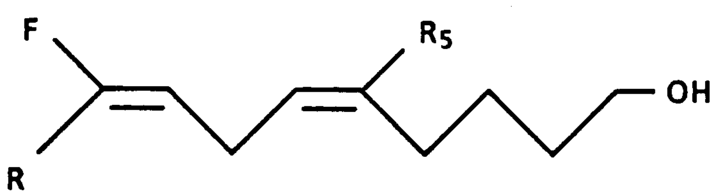

10

wherein R and $R_5$ are as defined in formula 1. Such an oxidation can be accomplished by, for example, treatment of the alcohol with the Jones Reagent. Excess Jones Reagent is then added to a cooled (0 °C) acetone solution of the alcohol and the reaction mixture is then allowed to react for about 10 to 30 minutes. Isopropanol is then added to consume excess Jones Reagent and the acetone solvent is removed by evaporation on the rotary evaporator. The residue is then mixed with water and the water mixture is extracted with ethyl acetate. After concentration of the ethyl acetate extracts, flash chromatography on silica gel eluting with a mixture of ethyl acetate and benzene (15:85) results in the isolation of the desired carboxylic acid.

The formula 10 alcohol is in turn prepared from the silylated halide of formula 11

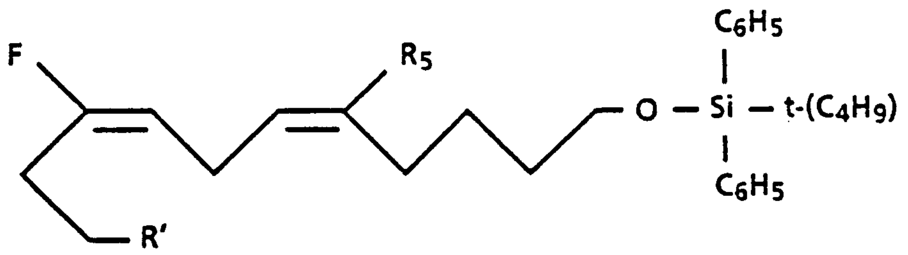

11, R' = Hal
11a, R' = OH

wherein $R_5$ is as defined in formula 1 and Hal is a chloro or preferably a bromo group. The formula 11 halide is first reacted with triphenylphosphine in the usual manner to form the triphenylphosphonium salt. The ylid is formed from the corresponding phosphonium salt in the usual way by treatment of the phosphonium salt with about one molar equivalent of a strong, organic base, preferably lithium diisopropylamide (LDA) formed *in situ* by the reaction of n-butyl lithium and diisopropylamine, at low temperature, typically from about -78 °C to about -25 °C, in a suitable solvent, preferably a solvent or solvent combination known to promote the Wittig reaction such as tetrahydrofuran (THF). Hexamethylphosphorictriamide (HMPA), which is known to promote the Wittig reaction by forming a chelate with the lithium counterion, can advantageously be added. The solution of ylid is then allowed to warm slightly to from about -30 °C to about 0 °C and the appropriate aldehyde is added, preferably dropwise, and allowed to react until formation of the desired condensation product is formed. The product can be isolated by quenching the reaction with a saturated, aqueous solution of ammonium chloride and subsequent removal of the organic solvent by evaporation with a rotary evaporator. The mixture is then extracted with diethyl ether to give the isolated product upon evaporation of the ether solvent. The crude product can be purified by, for example, flash chromatography on silica gel eluting with a mixture of hexane and benzene (9:1). Removal of the diphenyl-t-butyl silyl protecting group in the usual manner such as by treatment with fluoride ion results in the desired formula 10 alcohol.

The formula 11 silylated halide is prepared from the corresponding alcohol of formula 11a wherein $R_5$ is as defined in formula 1. Applicants have transformed the alcohol to the halide by reaction with one equivalent of 1-bromo-N,N,2-trimethylpropenylamine in a cooled (0 °C) methylene chloride solution. The formula 11a alcohol is prepared from the corresponding halide of formula 12

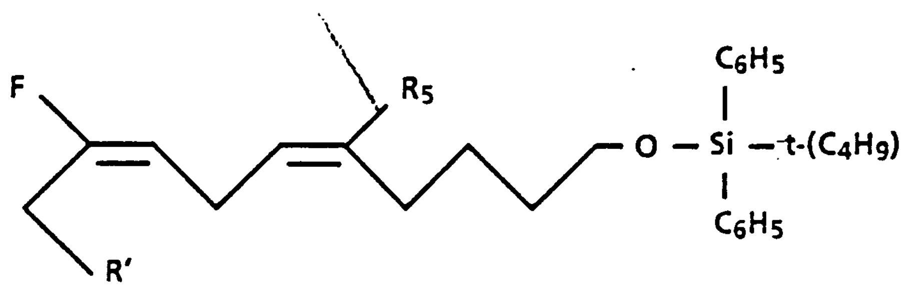

12, R' = Hal
12a, R' = OH

wherein $R_5$ is as defined in formula 1 and wherein Hal is a chloro group or preferably a bromo group. The formula 12 halide is treated with the anion of 1,3-dithiane formed by reaction of 1,3-dithiane with n-butyl lithium in cooled (i.e. -30 °C) tetrahydrofuran to produce an intermediate dithialane compound upon hydrolysis in the usual manner such as by addition of the dithialane derivative to a suspension of one equivalent of trimethyloxonium tetrafluoroborate in methylene chloride. After reaction for about one hour at

room temperature, two equivalents of calcium carbonate in aqueous acetone suspension are added and allowed to react overnight. The intermediate aldehyde is isolated and reduced with, for example, sodium borohydride in the usual manner to yield the desired alcohol of formula 11a.

The formula 12 halide is prepared from the corresponding alcohol of formula 12a wherein $R_5$ is as defined in formula 1 in any suitable art known procedure. Applicants have prepared the formula 12 bromide by treatment of the alcohol with one equivalent of 1-bromo-N,N-2-trimethylpropenylamine in a cooled (0 °C) methylene chloride solution. Alternatively the formula 12 halide can be prepared by stepwise conversion of the formula 12a alcohol to its mesylate (or tosyl) derivative by treatment with methanesulfonic acid chloride in the presence of a proton acceptor such as pyridine or triethylamine. Subsequent treatment of the mesyl derivative with a source of bromide ion such as a brominated ion exchange resin, for example, Amberlyst® A26, $Br^-$ form, results in the desired bromide 12. The bromination reaction utilizing Amberlyst® A26 resin will take from 8 to 24 hours when performed in refluxing benzene. Alternatively, the mesyl (or tosyl) derivative can be utilized directly in the reaction with the anion of 1,3-dithiane to produce the dithiane intermediate described above for the preparation of the formula 11 alcohol.

The formula 12a alcohol is prepared from the ester of formula 13

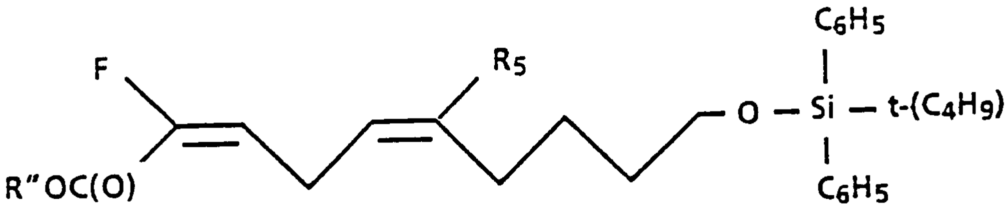

13

wherein $R_5$ is as defined in formula 1 and wherein R" is a lower alkyl, phenyl, or benzyl group such as an ethyl group. This reduction can be accomplished in any suitable manner such as by treating a solution of an ethyl ester of formula 13 (R" = ethyl) in an ethereal solvent such as THF with an aluminum hydride reducing agent such as diisobutylaluminum hydride (DIBAL). Such a reaction is typically carried out by adding a solution of DIBAL in hexane to a solution of the ester in the ethereal solvent. After stirring for from about 15 minutes to about 1 hour, preferably about 30 minutes, the reaction is allowed to continue at room temperature for from about 6 to about 24 hours. Addition of methanol to destroy excess reducing agent and ammonium chloride to precipitate the aluminum salts gives a solution of the reduced product. The product is isolated after solvent removal and is purified by flash chromatography on silica gel eluting with, for example, an 8:2 mixture of hexane and ethyl acetate.

The formula 13 ester is in turn prepared by reacting the formula 14 aldehyde

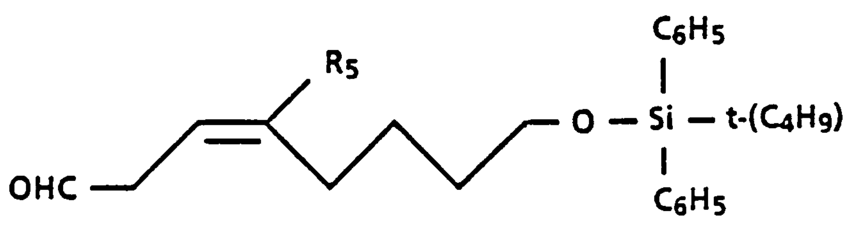

14

wherein $R_5$ is as defined in formula 1 with the ylid of the fluorinated phosphonate ester $(C_2H_5O)_2P(O)$-$CHFCO_2C_2H_5$. The ylid is formed from the corresponding phosphonium salt in the usual way by treatment of the phosphonium salt with about one molar equivalent of a strong, organic base, preferably lithium diisopropylamide (LDA) formed *in situ* by the reaction of n-butyl lithium and diisopropylamine, at low temperature, typically from about -78 °C to about -25 °C, in a suitable solvent, preferably a solvent or solvent combination known to promote the Wittig reaction such as tetrahydrofuran (THF). Hexamethylphosphorictriamide (HMPA), which is known to promote the Wittig reaction by forming a chelate with the lithium counterion, can advantageously be added. The solution of ylid is then allowed to warm slightly to from about -30 °C to about 0 °C and the appropriate aldehyde is added, preferably dropwise, and allowed to react until formation of the desired condensation product of structure 13 is formed. The product can be

isolated by quenching the reaction mixture with a saturated, aqueous solution of ammonium chloride and subsequent removal of the organic solvent by evaporation with a rotary evaporator. The mixture is then extracted with diethyl ether to give the isolated product upon evaporation of the ether solvent. The crude product can be purified by, for example, flash chromatography on silica gel eluting with a mixture of hexane and benzene (9:1).

The formula 14 aldehydes are prepared by addition of the 1,3-dithialane derivative of formula 15 to a suspension of one equivalent of trimethyloxonium tetrafluoroborate in methylene chloride. After reaction for about one hour at room temperature, two equivalents of calcium carbonate in aqueous acetone suspension are added and allowed to react overnight. The aldehyde is isolated by, for example, filtration and solvent removal.

The silylated dithialanes of formula 15 wherein $R_5$ is a fluoro group are prepared from the dithianyl alcohol of formula 19 as outlined in scheme 1.

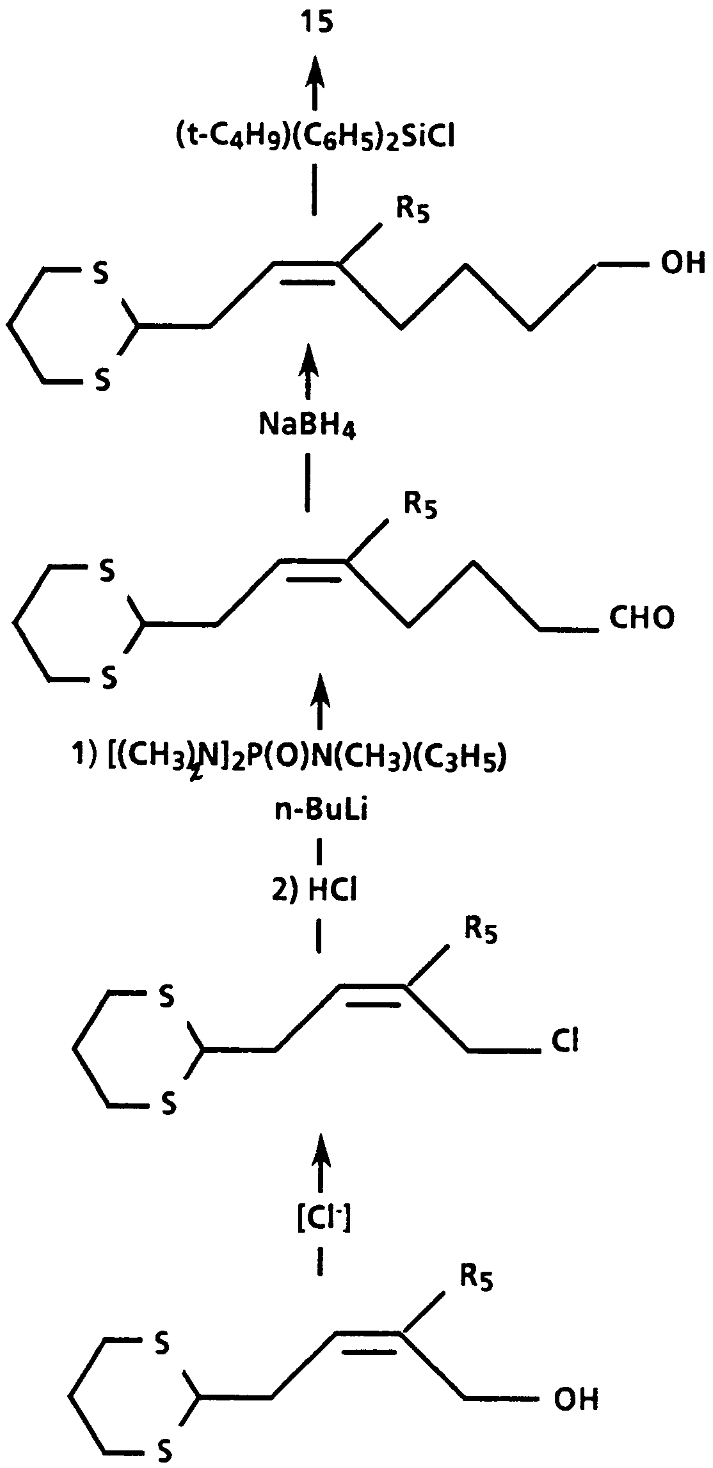


16

17

18

19

SCHEME 1

The hydroxy group of the formula 19 butene is converted to a chloro group by reaction with, for example, 1-chloro-N,N,2-trimethylpropeneylamine. The chlorinated compound of formula 18 wherein $R_5$ is as defined in formula 1 is transformed into the aldehyde of formula 17 by reaction with N-allyl-N,N',N''-pentamethylphosphoramide in tetrahydrofuran. Reduction in the usual manner with sodium borohydride results in formation of the alcohol of formula 16. Treatment of the alcohol with t-butyldiphenylsilyl chloride in the presence of a proton acceptor gives the desired compound of formula 15. The hydroxy dithialane of formula 19 is prepared from a chloro tetrahydropyranyloxy butene of formula 21

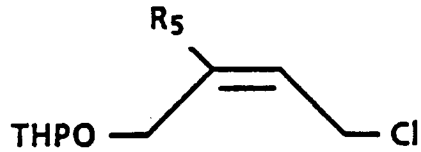

21

wherein $R_5$ is a defined in formula 1. The dithialane derivative of formula 20

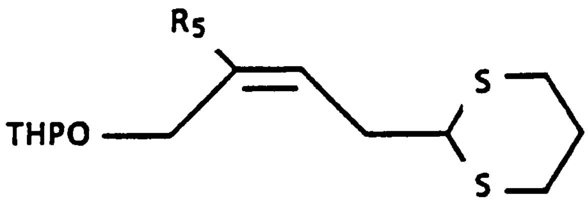

20

is first prepared by reaction of the formula 21 chloro derivative with the anion of 1,3-dithiane formed by reaction with n-butyl lithium in cooled (i.e. -30 ° C) tetrahydrofuran.

Subsequent removal of the THP protecting group by treatment with methanol and pyridinium para-toluene sulfonate (PPTS) catalyst results in the desired formula 19 alcohol. The compound of formula 21 is readily prepared from the optionally fluorinated maleic acid, formula 24, as illustrated in scheme 2.

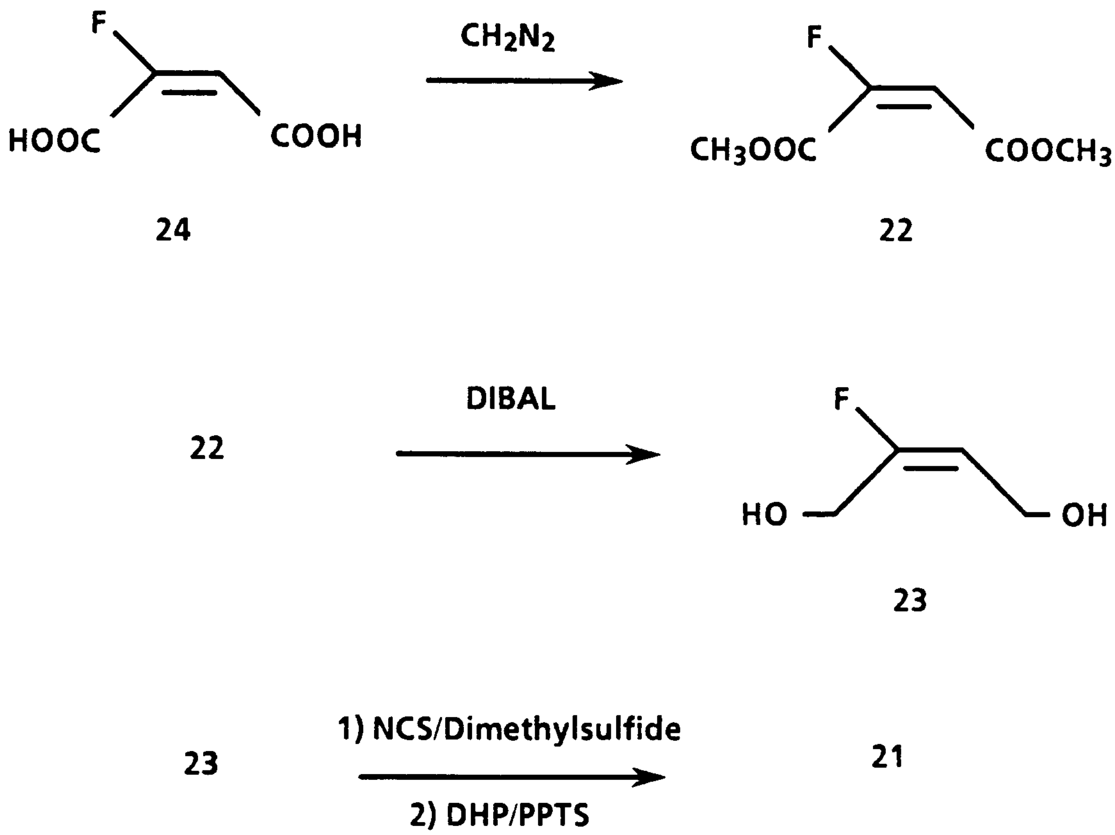


24 22

22 23

23 21

SCHEME 2

The optionally fluorinated maleic acid is converted to the corresponding dimethyl ester of formula 22 by reaction with diazomethane. Subsequent ester group reduction with excess diisobutylaluminum hydride (DIBAL) in THF at about 0 °C results in formation of the di-alcohol derivative of formula 23. Selective conversion of one of the hydroxy groups can be accomplished using a slight (10%) molar excess of N-chlorosuccinimide (NCS) and dimethylsulfide. Protection of the other hydroxy group as the THP derivative can be accomplished in the usual manner by reaction with dihydropyran (DHP) and catalytic pyridinium paratoluene sulfonate (PPTS) which results in formation of the desired formula 21 compound.

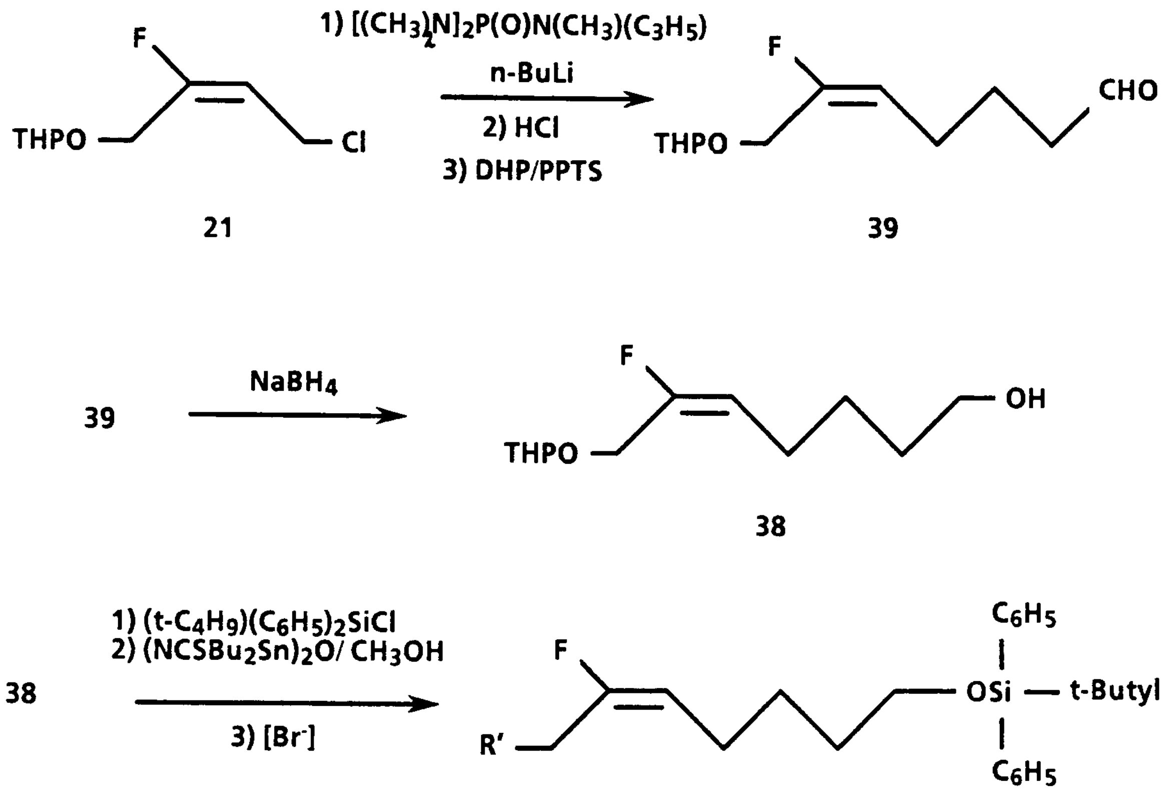


21

39

38

37 R' = THPO-
36 R' = HO-
35 R' = Br-

SCHEME C

To prepare the 6,9-difluoroarachidonic acids of formula 1, the alcohols of 36 are treated with one equivalent of 1-bromo-N,N-2-trimethylpropenylamine in a cooled (0 °C) methylene chloride solution to obtain the corresponding bromide. The bromide (35) is treated with the anion of 1,3-dithiane with n-butyl lithium in cooled (-30 °C) THF to produce an intermediate dithalane upon hydrolysis in the usual manner (i.e., addition of trimethyloxonium tetrafluoroborate in $CH_2Cl_2$. After reaction for about 1 hour at room temperature, two equivalents of calcium carbonate in aqueous acetone suspension is added and allowed to react overnight.) gives the corresponding aldehyde. This so-produced 6-F aldehyde is isolated and may be used without purification. This 6-F aldehyde is converted to the corresponding 6,9-difluoro compounds in the analogous way that the corresponding 5-fluoro aldehyde is converted to the 5,9-difluoro compounds of formula 10.

To prepare the 9-fluoro arachidonic acid derivatives of formula 1 the chemistry used and described herein may analogously be utilized. Starting with dehydro-2,3-delta valero lactone,

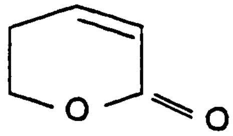

the lactone is reduced to the corresponding 2-diol with an excess of DIBAL in THF. The diol ($HOCH_2CH_2CH=CHCH_2OH$) is treated with one equivalent of N-chlorosuccinimide (NCS) in $CH_2Cl_2$ in the presence of dimethylsulfide to yield the Z-1-chloro-5-OH-2-pentene, the alcohol of which is protected as a tetrahydropyranyl ether (OTHP) using an excess of dihydropyran in the presence of catalytic amounts of PPTS. The chloride ($THPO-CH_2CH_2CH=CHCH_2Cl$) is converted to its aldehyde using analogous chemistry as described for converting 3 to 2 (i.e., $[(CH_3)_2N]_2P(O)N(CH_3)(C_3H_5)$ plus n-BuLi, (2) HCl and (3) reprotection of the alcohol with DHP/PPTS as in the conversion of 16 to 15). The aldehyde is reduced with $NaBH_4$ (analogously with 17 to 16). The alcohol is silylated using chemistry analogous to conversion of 16 to 15 and then the THP protecting group is cleaved as described on page 26 for converting compound 20 to 19. The resulting alcohol is oxidized with pyridinium chlorochromate in $CH_2Cl_2$, as previously described, to yield the aldehyde which is treated with the ylid of a fluorinated phosphonate ester using the same chemistry analogously described for converting compound 14 to 13. Following this chemical step then the same chemistry used to convert 10 to the desired arachidonic acids may be used (i.e., the $R_5$ would be H).

The compounds of structure 1 wherein X is other than C(O)OH can be readily prepared from the carboxylic acids by any procedure generally known to those skilled in the art. For example, those compounds of formula 1 wherein X is $-C(O)NH_2$, are prepared from the corresponding compound wherein X is $-CO_2H$ by reaction with about 1 molar equivalent of carbonyldiimidazole in an aprotic organic solvent, preferably dichloromethane, for a period of 1 to 7 hours, preferably about 4 hours. Then the product is reacted with a large excess of ammonium hydroxide for from 24 to 64 hours, preferably for about 48 hours. Isolation of the desired compounds of formula 1 wherein X is $CONH_2$ can be by any suitable means known to those in the art.

Alternatively, the compounds of formula 1 wherein X is $CONH_2$ can be prepared by first converting the acid into an activated derivative such as, for example, by reaction of the carboxylic acid with an acyl halide, an anhydride, a mixed anhydride, an alkyl ester, a substituted or unsubstituted phenyl ester, a thioalkyl ester, a thiophenyl ester, an acyl imidazole, and the like. The activated derivative is then reacted with ammonia or aqueous ammonia with or without a suitable water-miscible or immiscible organic solvent, for example, methanol, ethanol, dichloromethane, and the like, so as to produce the amide. The reaction is conducted at from - 30 °C to the boiling point of the solvent or solvent mixture used, for from 1 to 96 hours.

Alternatively, the amide can be made by heating together the appropriate compound of formula 1 wherein X is $CO_2H$ and ammonia, or by heating the ammonium salt of a carboxylic acid of formula 1. The reaction is conducted either in the absence of a solvent, or in the presence of a solvent such as, for example, toluene, at a temperature of from 100 °C to 300 °C, for from 1 to 12 hours.

Alternatively, the amide can be obtained by hydrolysis of a nitrile derivative (formula 1 wherein X is CN) using either inorganic or organic acids or bases, such as, for example, hydrochloric acid, sulphuric acid, p-toluenesulphonic acid, sodium hydroxide, potassium carbonate, or tetrabutylammonium hydroxide and the like. The reaction is conducted in water optionally containing from 1% to 95% of a cosolvent such as, for example, methanol, acetic acid or diglyme, at a temperature of from 0 °C to the boiling point of the solvent used, for from 1 to 96 hours. Such procedures are well known to those skilled in the art and are described, for example, in Synthetic Organic Chemistry, John Wiley and Sons, Publ., New York, 565-590 (1953) and Compendium of Organic Synthetic Methods, Vol. 1, Wiley-Interscience, New York, 203-230 (1971).

The compounds of formula 1 wherein X is a 1H-tetrazol-5-yl group can be prepared from the corresponding amide (I wherein X is $CONH_2$) via an intermediate nitrile (I wherein X is CN) derivative. To a solution of an appropriate compound of formula 1 wherein X is $CONH_2$ in a basic organic solvent, preferably pyridine, is added about 1 mole, or equivalent, of an organic sulphonyl halide, preferably p-toluenesulphonyl chloride. The mixture is reacted for 12-48 hours, preferably about 24 hours, and the solution is poured into water. The nitrile is extracted from the aqueous phase with an organic solvent, preferably ethyl ether, and the extract is purified by procedures known in the art.

The isolated nitrile is then reacted with an excess, preferably 3 moles, of an alkali metal azide, preferably sodium azide, and an excess, preferably 3 moles, of an ammonium halide, preferably ammonium chloride, in an aprotic, polar solvent, preferably dimethylformamide, at a temperature of 80 °C to 120 °C, preferably 100 °C, for 16 to 48 hours, preferably 24 hours optionally in the presence of a Lewis acid such

as, for example, boron trifluoride. In this reaction, other sources of azide ion may be used, such as aluminium azide and the azide of tri-n-butyl tin. The product is then isolated by procedures known in the art.

Alternatively, the compounds of formula 1 wherein X is a 1H-tetrazol-5-yl group can be prepared by the reaction between an iminoether derivative of formula 1 wherein X = C(NH)O($C_1$-$C_6$ alkyl) and hydrazoic acid as described in German Patent 521870. The iminoether derivative is obtained by treatment of a nitrile derivative (formula 1 wherein X = CN) with a ($C_1$-$C_6$) alkanol and a strong acid such as, for example, hydrochloric acid or p-toluenesulphonic acid. The reaction between the iminoether and hydrazoic acid is conducted in the presence of a solvent such as, for example, chloroform or dimethylformamide, at from 0 ° C to 120 ° C, for from 1 to 72 hours. Tetrazole derivatives can also be obtained by the reaction between an amidine derivative of an unsaturated fatty acid, prepared, for example, from the nitrile derivative as described in Synthetic Organic Chemistry, John Wiley and Sons, Publ., New York, 635 (1953) and nitrous acid, as described in Annalen, 263, 96 (1981), and 208, 91 (1987). The reaction is conducted in water or a mixture of water and a suitable organic solvent such as, for example, methanol or dioxane, at from 0 ° C to 100 ° C, for from 1 to 24 hours.

The esters of compounds of formula I, those wherein X is C(O)$OR^1$ wherein $R^1$ is a straight chain ($C_1$-$C_6$) alkyl group can be prepared in the usual manner by esterification of the corresponding carboxylic acid of formula 1 (X = $CO_2H$)by treatment with a solution of hydrogen chloride in the appropriate lower alkanol . Preferably the esters are prepared from the carboxylic acids via the acid chloride derivative. The acid is reacted with a thionyl or phosphoryl halide or phosphorus pentahalide, preferably thionyl chloride, dissolved in an inert organic solvent, preferably benzene, containing a trace of a tertiary organic amide, preferably dimethylformamide. The mixture is reacted for 8-32 hours, preferably for about 16 hours, at from 0 ° C to 25 ° C, then evaporated to dryness. The residue, the acid chloride, is dissolved in an inert organic solvent and the appropriate lower alkanol is added dropwise.

The acylhydroxylamine derivatives, those compounds of formula I wherein X is CONHOH, are prepared in two ways. The acid is either first converted, as described above, into the acid chloride or into a lower alkyl ester, preferably the methyl ester. The acid chloride or the lower alkyl ester is then reacted with an excess of hydroxylamine in an aqueous organic solvent, preferably aqueous methanol, at a pH of between 7 and 10, preferably at about pH 9, for from 1/4 to 6 hours, preferably about 1 hour. The acylhydroxylamine product is then isolated by means known in the art.

Acylhydroxylamines can also be prepared by the reaction between hydroxylamine and an activated derivative of an unsaturated fatty acid such as, for example, an acyl halide, an anhydride, a mixed anhydride, an alkyl ester, a substituted or unsubstituted phenyl ester, a thioalkyl ester, a thiophenyl ester, an acyl imidazole, and the like. The reaction is conducted in an aqueous organic or organic solvent such as, for example, methanol, acetonitrile or acetone, at from 0 ° C to the reflux temperature of the solvent, for from 1 to 48 hours. Alternately, acylhydroxylamines can be prepared by acid-catalyzed rearrangement of a primary nitro derivative (formula 1, X = $NO_2$) as described in Chemical Reviews, 32, 395 (1943). The reaction is conducted in an aqueous organic or organic solvent, such as, for example, methanol, ethanol and dioxan, at from 0 ° C to 100 ° C, for from 1 to 24 hours, in the presence of a strong acid such as, for example, sulphuric acid or hydrochloric acid. Acylhydroxylamine derivatives of unsaturated fatty acids can also be obtained by the oxidation of the oxime derivative of formula 1 wherein X = CHNOH using, for example, hydrogen peroxide as described in Chemical Reviews, 33, 225 (1943). The reaction is conducted in a solvent such as methanol or dichloromethane and the like, at from 0 ° C to 35 ° C for from 1 to 6 hours.

The chloro derivative, 17, is first reacted with N-allyl-N,N′,N″-pentamethylphosphoramide in tetrahydrofuran. The intermediate product is isolated and then treated with concentrated hydrochloric acid. Finally the THP (tetrahydropyranyloxy) group is reformed by reaction of the product with dihydropyran (DHP) and catalytic pyridinium paratoluene sulfonate (PPTS) to produce the aldehyde, 16. Reduction in the usual manner with sodium borohydride results in alcohol 15. Treatment of the alcohol with t-butyldiphenylsilyl chloride in the presence of a proton acceptor gives the desired 14. The THP protecting group is then removed by treatment with methanol and tetrabutyl-1,3-diisothiocyanatodistannoxane catalyst to give the alcohol 13. The alcohol is converted to the corresponding bromide, 12, by reaction with 1-bromo-N,N-2-trimethylpropenylamine in methylene chloride solution. The silylated dithialane, 7, is then produced by reaction of the bromide, 12, with the anion of 1,3-dithiane formed by reaction with n-butyl lithium in cooled (i.e. -30 ° C) tetrahydrofuran.

Isolation and purification of the compounds and intermediates described herein can be effected, if desired, by any suitable separation or purification procedure such as, for example, filtration, extraction, crystallization, column chromatography, thin-layer chromatography or thick layer chromatography, or a combination of these procedures. Specific illustrations or suitable separation and isolation procedures can be had by reference to the examples hereinbelow. However, other equivalent separation or isolation

procedures could, of course, also be used.

The pharmaceutically acceptable salts of the compounds of this invention wherein X is $CO_2H$, C(O)-NHOH or 1H-tetrazol-5-yl, are prepared by treating the carboxylic acid, acylhydroxylamine or tetrazole compound of formula 1 with at least one molar equivalent of a pharmaceutically acceptable base. Representative pharmaceutically acceptable bases are sodium hydroxide, potassium hydroxide, ammonium hydroxide, calcium hydroxide, metal alkoxides, for example, sodium methoxide, trimethylamine, lysine, caffeine, and the like. The reaction is conducted in water, alone or in combination with an inert, water-miscible organic solvent, or in a suitable organic solvent such as methanol, ethanol, and the like, at a temperature of from about 0 °C to about 100 °C, preferably at room temperature. Typical inert, water-miscible organic solvents include methanol, ethanol, or dioxane. The molar ratios of compounds of Formula 1 to base used are chosen to provide the ratio desired for any particular salt.

Salts derived from inorganic bases include sodium, potassium, lithium, ammonium, calcium, magnesium, ferrous, zinc, copper, manganous, aluminum, ferric, manganic salts and the like. Particularly preferred are the ammonium, potassium, sodium, calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like. Particularly preferred organic non-toxic bases are isopropylamine, diethylamine, ethanolamine, tromethamine, dicyclohexylamine, choline and caffeine.

The salt products are also isolated by conventional means. For example, the reaction mixtures may be evaporated to dryness, and the salts can be further purified by conventional methods. Salts of the compounds of formula 1 may be interchanged by taking advantage of differential solubilities of the salts, or by treating with the appropriately loaded ion exchange resin.

The amount of a fluorinated arachidonic acid derivative of this invention necessary to control the biosynthesis of leukotrienes prophylacticly or to treat existing allergic or inflammatory states can vary widely according to the particular dosage unit employed, the period of treatment, the age and sex of the patient treated and the nature and extent of the disorder treated. The total amount of the active ingredient to be administered will generally range from about 1 mg/kg to 150 mg/kg and preferably from 3 mg/kg to 25 mg/kg. For example, an average 70 kg human patient will require from about 70 mg to about 10 g of active compound per day. A unit dosage may contain from 25 to 500 mg of active ingredient, and can be taken one or more times per day. The active compound of formula 1 can be administered with a pharmaceutical carrier using conventional dosage unit forms either orally, parenterally, or topically.

The preferred route of administration is oral administration. For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intented to assist the break-up and dissolution of the tablet following administration such as potato starch, alginic acid, corn starch, and guar gum, lubricants intented to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intented to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers,

methylcellulose, hydroxypropylmethylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

Aerosol or spray compositions containing the compounds of this invention can be applied to the skin or mucous membranes. Such compositions may contain a micronized solid or a solution of a compound of formula 1 and may also contain solvents, buffers, surfactants, perfumes, antimicrobial agents, antioxidants, and propellants. Such compositions may be applied by means of a propellant under pressure or may be applied by means of a compressible plastic spray bottle, a nebulizer, or an atomizer without the use of a gaseous propellent. A preferred aerosol or spray composition is a nasal spray.

The active ingredient may also be administered by means of a sustained release system whereby the compound of formula 1 is gradually released at a controlled, uniform rate from an inert or bioerodible carrier by means of diffusion, osmosis, or disintegration of the carrier during the treatment period. Controlled release drug delivery systems may be in the form of a patch or bandage applied to the skin or to the buccal, sublingual, or intranasal membranes, an ocular insert placed in the cul de sac of the eye, or a gradually eroding tablet or capsule or a gastrointestinal reservoir administered orally. Administration by means of such sustained release delivery systems permits the tissues of the body to be exposed constantly for a prolonged time period to a therapeutically or prophylactically effective dosage of a compound of formula 1. The unit dosage of the compound administered by means of a sustained release system will approximate the amount of an effective daily dosage multiplied by the maximum number of days during which the carrier is to remains on or in the body of the host. The sustained release carrier may be in the form of a solid or porous matrix or reservoir and may be formed from one or more natural or synthetic polymers, including modified or unmodified cellulose, starch, gelatin, collagen, rubber, polyolefins, polyamides, polyacrylates, polyalcohols, polyethers, polyesters, polyurethanes, polysulphones, polysiloxanes, and polyimides as wells as mixtures and copolymers of these polymers. The compounds of formula 1 may be incorporated in the sustained release carrier in a pure form or may be dissolved in any suitable liquid or solid vehicle, including the polymer of which the sustained release carrier is formed.

## EXAMPLE 1

### PREPARATION OF 5,8-DIFLUOROEICOSA-5,8,14-TRIENOIC ACID

#### 1A) PREPARATION OF ETHYL 2-FLUOROTETRADECA-2,8-DIENEOATE

n-BuLi 1.7 M in hexane solution (12.9 ml) was added at -78 °C to a solution of diisopropylamine (5.5 ml) in tetrahydrofuran. The mixture was stirred for 20 min. at -78 °C. Then triethylphosphonofluoro acetate (5.3 g, 22 mmoles) in tetrahydrofuran (5 ml) was added dropwise. The mixture was stirred 5 min. at -78 °C and then 20 min. at 0 °C. Then 6-dodecenal (4 g, 22 mmoles) in tetrahydrofuran (5 mL) was added dropwise at -78 °C. The reaction mixture was stirred overnight at 0 °C. The mixture was hydrolyzed with a saturated aqueous solution of ammonium chloride and extracted with ether. The organic layer was washed with saturated brine, dried over sodium sulfate and concentrated under reduced pressure. Flash chromatography on silica gel and elution with a 8:2 mixture of hexane and ethyl acetate afforded the expected ester as an oil

(5.53 g, 93%).

1B) PREPARATION OF 2-FLUOROTETRADECA-2,8-DIENE-0L

The ester prepared in 1A (5.53 g, 20.5 mmoles) in anhydrous ether (5 ml) was added at -78°C to a mixture of DIBAL, 1 M solution in hexane, (61.4 ml, 61.5 mmoles) and ether (90 ml). The mixture was stirred 3 min. at -78°C, then overnight at room temperature. The excess of DIBAL was destroyed with methanol (10 ml) and the aluminum salts were precipitated with an aqueous saturated solution of ammonium chloride (5 ml). The mixture was filtrated and the precipitate washed with ethyl acetate. The solvents were evaporated under reduced pressure. Flash chromatography over silca gel and elution with a 8:2 mixture of hexane and ethyl acetate afforded the expected alcohol 2 as an oil (4.52 g, 92%).

1C) PREPARATION OF 1-BROMO-2-FLUOROTETRADECA-2,8-DIENE

The alcohol prepared in 1B (2 g, 8.76 mmoles) was dissolved in dry methylene chloride (20 ml). The mixture was cooled to 0°C and 1-bromo N,N-2-trimethylpropenylamine (1.9 ml, 10.7 mmoles) was added. The mixture was stirred under argon for 30 min. The methylene chloride was evaporated under reduced pressure. Flash chromatography on silica gel and elution with pentane afforded the expected bromide as an oil (2.55 g, 100%).

1D) PREPARATION OF 1(1,3-DITHIANE-2-CYCLOHEXYL)-2-FLUOROTETRADECA-2,8-DIENE

To a solution of 1,3-dithiane (1.35 g, 11.12 mmoles) in tetrahydrofuran (50 ml) cooled to -25°C was added dropwise a 1.6 M solution of n-butyllithium in hexane (5.8 ml, 9.3 mmoles). The mixture was stirred at -30°C for 30 min. Then the mixture was cooled to -40°C and the bromide prepared in 1C (2.72 g, 9.34 mmoles) in tetrahydrofuran (5 ml) was added dropwise. The reaction mixture was stirred 30 min. at -40°C and 2 hrs. at 0°C. The reaction was quenched with saturated aqueous ammonium chloride and the tetrahydrofuran was evaporated under reduced pressure. The residue was diluted with ether and washed with water. The organic layer was dried over sodium sulfate, filtered and concentrated under reduced pressure. Flash chromatography on silica gel and elution with a 8:2 mixture of hexane and ethyl acetate afforded the title dithialane as an oil (2.74 g, 90%).

1E) PREPARATION OF 3-FLUOROPENTADECA-3,9-DIENE-AL

To a solution of the dithialane prepared in 1D (1.5 g, 4.5 mmoles) in dry methylene chloride (10 ml) was added at room temperature trimethyloxonium tetrafluoroborate (1 g, 6.75 mmoles) and the mixture was stirred for 1 hr. Then a 9:1 mixture of acetone and water (20 ml) containing calcium carbonate (0.5 g) was added and the mixture was stirred overnight at room temperature. The precipitate was filtered off and after dilution with saturated brine the mixture was extracted three times with ether. The organic layer was dried over sodium sulfate, filtered and concentrated under reduced pressure to afford the expected aldehyde as an oil (650 mg, 60%) which was used without purification after drying under high vacuum.

1F) PREPARATION OF 2,5-DIFLUOROHEPTADECA-2,5,11-TRIENE OATE

n-BuLi 1.6 M in hexane (1.55 ml) was added at -78°C to a solution of diisopropyl amine (0.35 mmoles) in tetrahydrofuran. The mixture was stirred during 20 min. at -78°C. Then triethylphosphonofluoroacetate (10.6 g, 2.5 mmoles) in tetrahydrofuran (2 ml) was added dropwise. The mixture was stirred 5 min. at -78°C and then 20 min. at 0°C. Then the aldehyde prepared in 1E (0.6 g, 2.5 mmoles) in tetrahydrofuran (4 ml) was added dropwise at -78°C. The reaction mixture was stirred overnight at 0°C. The mixture was hydrolyzed with a saturated aqueous solution of ammonium chloride and extracted with ether. The organic layer was washed with saturated brine, dried over magnesium sulfate and concentrated under reduced pressure. Flash chromatography on silica gel and elution with a 8:2 mixture of hexane and ethyl acetate afforded the expected ester as an oil (0.39 g, 97%).

1G) PREPARATION OF 2,5-DIFLUOROHEPTADECA-2,5-11-TRIENE-OL

The ester prepared in 1F (0.39 g, 1.18 mmoles) in anhydrous ether (5 ml) was added at -78°C to a mixture of DIBAL, 1 M solution in hexane, (2.5 mL) and ether (20 ml). The mixture was stirred 30 min. at

-78°C, then overnight at room temperature. The excess of DIBAL was destroyed with methanol (2 ml) and the aluminum salts were precipitated with an aqueous saturated solution of ammonium chloride. The mixture was filtrated and the precipitate washed with ethyl acetate. The solvents were evaporated under reduced pressure. Flash chromatography over silica gel and elution with a 8:2 mixture of hexane and ethyl acetate afforded the expected alcohol as an oil (0.33 g, 92%).

1H) PREPARATION OF 1-CHLORO-2,5-DIFLUOROHEPTADECA-2,5,11-TRIENE

The alcohol prepared in 1G (0.33 g, 1.09 mmoles) was dissolved in dry methylene chloride (5 ml) The mixture was cooled to 0°C and 1-chloro-N,N-2-trimethylpropenylamine (0.147 g, 1.1 mmoles) was added. The mixture was stirred under argon for 15 min. Methylene chloride was evaporated under reduced pressure. Flash chromatography on silica gel and elution with pentane afforded the expected chloride as an oil (0.321 g, 90%).

1I) PREPARATION OF 5,8-DIFLUOROEICOSA-5,8,14-TRIENAL

To a solution of N-allyl-N,N',N''-pentamethyl phosphoramide (0.20 g, 1 mmoles) in tetrahydrofuran (10 ml) cooled to -78°C was added dropwise n-butyllithium 1.32 M in hexane (0.75 ml, 1 mmole). The mixture was stirred under argon at -78°C for 1 hr. To the resulting red-orange solution, the chloride prepared in 1H in tetrahydrofuran (2 ml) was added dropwise at -78°C. The mixture was stirred for 1 hr. at -78°C, then warmed to 0°C within 2 hrs. and stirred 30 min. at 0°C. The reaction was quenched with saturated aqueous ammonium chloride and tetrahydrofuran was evaporated under reduced pressure. The resulting oil was diluted with methylene chloride and washed with water. The organic layer was dried over magnesium sulfate. Filtration and concentration under reduced pressure afforded an oil. This oil was dissolved in ether (15 ml) and was stirred at room temperature during 2 hrs. with 2N aqueous solution of hydrochloric acid (15 ml). The organic layer was washed twice with water, dried over magnesium sulfate, filtered and concentrated under reduced pressure to afford an oil. Flash chromatography on silica gel and elution with a 25:75 mixture of ethyl acetate and hexane afforded the desired aldehyde (0.21 g, 69%) as an oil.

1J) PREPARATION OF 5,8-DIFLUOROEICOSA-5,8,14-TRIENOIC ACID

To a solution of the aldehyde prepared in 1I (0.2 g, 0.61 mmoles) in acetone (7 ml) cooled to 0°C was added dropwise 2.67M Jones reagent until the orange color was stable. The mixture was stirred 15 min. at 0°C. The excess of Jones reagent was reacted with isopropanol. The acetone was evaporated under reduced pressure without heating. The residue was taken with water and extracted three times with ethyl acetate. The organic layer was dried over sodium sulfate, filtered and concentrated under reduced pressure to leave an oil. Flash chromatography on silica gel and elution with a 25:75 mixture of ethyl acetate and hexane afforded the expected acid as an oil (0.125 g, 60%).

EXAMPLE 2

PREPARATION OF 5,9-DIFLUOROEICOSATETRAENOIC ACID

2A) PREPARATION OF FLUOROMALEIC ACID DIMETHYL ESTER

Fluoromaleic acid (37.14 g, 0.277 mole) were esterified in ether at 0°C by an excess of 0.5M etheral solution of diazomethane until the yellow coloration was stable. Evaporation of the solvent afforded pure diester as an oil (44.71 g, 99.5%). NMR ($H^1$, $CDCl_3$, 60 MHz): 3.78 (s, 3), 3.86 (s, 3), 6.06 (d, $J_{HF}$ = 15.5 Hz, 1).

2B) PREPARATION OF (E) 1,4-DIHYDROXY-2-FLUORO-2-BUTENE

To a solution of the diester prepared in 2A (20 g, 0.123 mole) in dry tetrahydrofuran (250 ml) cooled to -10°C was added dropwise under argon a 1.2M diisobutyl aluminium hydride (DIBAL) in hexane (568 ml) while the temperature of the reaction mixture was maintained at 0°C. The mixture was stirred at 0°C during one hour and during 1 hour at room temperature. The mixture was cooled again to 0°C and methanol (25 ml) was added dropwise to destroy the excess of DIBAL. Then the aluminium salts were precipitated with an aqueous saturated solution of ammonium chloride added until a filtrable product was obtained. The

white-grey solid was filtered and the cake was washed with ethyl acetate containing 10% of methanol. The solvents were evaporated under reduced pressure. The resulting oil was chromatographed on silicagel using pure ethyl acetate as eluent. The diol was obtained as an oil (5.4 g, 41%). NMR ($H^1$, $CD_3OD$, 360 MHz): 4.13 (dd, $J_{HH}=8$ Hz, $J_{HF}=1.5$ Hz, 2), 4.23 (d, $J_{HF}$ = 21 Hz, 2), 5.43 (dt, $J_{HF}=20$ Hz, $J_{HH}=8$ Hz, 1).

2C) PREPARATION OF (E) 1-CHLORO-3-FLUORO-4-(2-TETRAHYDROPYRANYLOXY)-2-BUTENE

To a solution of N-chlorosuccinimide (2.76 g, 18 mmoles) in methylene chloride (80 ml) was added at 0°C dimethylsulfide (1.32 ml, 18 mmoles) and the mixture was stirred for 15 min at 0°C. Then after cooling at -25°C the diol from 2B (1.74 g, 16.4 mmoles) in methylene chloride (40 ml) was added dropwise. The mixture was stirred successively for 30 min at -25°C, 3 hours at 0°C, and finally 30 min at room temperature. Dihydropyran (3 ml, 32.8 mmoles) and pyridinium paratoluenesulfonate (430 mg, 1.6 mmoles) were added. Thus the mixture was stirred overnight at room temperature. The reaction mixture was washed with water and saturated brine. The organic phase was dried over sodium sulfate. Filtration and flash chromatography on silicagel and elution with a 9:1 mixture of hexane and ethyl acetate afforded the title chloride as an oil (2.69g, 79%). NMR ($H^1$, $CDCl_3$, 60 MHz): characteristic peaks 4.15 (dd, $J_{HH}=8$ Hz, $J_{HF}=1$ Hz, 2), 4.23 (d, $J_{HF}=20$ Hz, 2), 4.68 (broad s,1), 5.55 (dt, $J_{HH}=8$ Hz $J_{HF}=20$ Hz, 1).

2D) PREPARATION OF (E) 1-(1,3-DITHIA-2-CYCLOHEXYL-3-FLUORO-4-(2-TETRAHYDROPYRANYLOXY)-2-BUTENE)

To a solution of 1,3-dithiane (1.55g, 12.9 mmole) in tetrahydrofuran (60 ml) cooled to -30°C was added dropwise a 1.32M solution of n-butyllithium in hexane (9.77 ml, 12.9 mmoles), the mixture was stirred at -30°C for 30 min. Then the mixture was cooled to -40°C and the chloride prepared in 2C (2.69g, 12.9 mmoles) in tetrahydrofuran (10 ml) was added dropwise. The reaction was stirred 30 min at -40°C and 2 hrs at 0°C. The reaction was quenched with saturated aqueous ammonium chloride and the tetrahydrofuran was evaporated under reduced pressure. The residue was diluted with ether and washed with water. The organic layer was dried over sodium sulfate, filtered and concentrated under reduced pressure. Flash chromatography on silicagel and elution with a 8:2 mixture of hexane and ethyl acetate afforded the title dithialane as an oil (3.34 g, 90%). NMR ($H^1$, $CDCl_3$, 60 MHz) characetristic peaks: 4.00 (t, $J_{HH}=7$ Hz, 1), 4.21 (d, $J_{HF}=20$ Hz, 2), 4.7 (broad peak, 1), 5.40 (dt, $J_{HF}=20$ Hz, $J_{HH}=8$ Hz, 1).

2E) PREPARATION OF (E) 1-(1,3-DITHIA-2-CYCLOHEXYL)-3-FLUORO-4-OL-2-BUTENE

The tetrahydropyranyl derivative prepared in 2D was dissolved in methanol. Pyridinium para-toluene sulfonate (0.3 g, 1.2 mmoles) was added and the mixture was refluxed for 2.5 hrs. Methanol was evaporated under reduced pressure. The residue was dissolved in ether and washed with water. The organic layer was dried over sodium sulfate, filtered and concentrated under reduced pressure. Flash chromatography on silicagel and elution with a 1:1 mixture of hexane and ethyl acetate afforded the title alcohol as white crystals (2.11 g, 91%). Recrystallization in a mixture of hexane and ether afforded analytically pure samples, m.p. = 33.5 - 34.5°C. NMR ($H^1$, $CDCl_3$, 60 MHz) characteristic peaks: 4.0 (t, $J_{HH}=7$ Hz, 1), 4.18 (d, $J_{HF}=20$ Hz, 2), 5.25 (dt, $J_{HF}=20$ Hz, $J_{HH}=8$ Hz, 1). Anal. Calc. for $C_8H_{13}FOS_2$: C, 46.13; H, 6.29. Found: C, 46.28; H, 6.01.

2F) PREPARATION OF (E) 4-CHLORO-2-(1,3-DITHIA-2-CYCLOHEXYL)-3-FLUORO-2-BUTENE

The alcohol prepared in 2E (1.9 g, 9.13 mmoles) was dissolved in dry methylene chloride (70 ml). The mixture was cooled to 0°C and 1-chloro N,N-2-trimethylpropenylamine (1.23 g, 9.2 mmoles) was added. The mixture was stirred under argon for 15 min. Methylene chloride was evaporated under reduced pressure. Flash chromatography on silicagel and elution with a 9:1 mixture of hexane and ethyl acetate afforded the expected chloride as an oil (1.98 g, 96%). NMR ($H^1$, $CDCl_3$, 60 MHz) characteristic peaks 4.05 (t, $J_{HH}=7$ Hz, 1), 4.13 (d, $J_{HF}=21$ Hz, 2), 5.36 (dt, $J_{HF}=18$ Hz $J_{HH}=8$ Hz, 1).

2G) PREPARATION OF (E) 7-(1,3-DITHIA-2-CYCLOHEXYL)-5-FLUORO-5-HEPTENAL

To a solution of N-allyl-N, N′,N″-pentamethyl phosphoramide (1.5 g, 7.32 mmoles) in tetrahydrofuran (21 ml) cooled to -78C was added dropwise n-butyllithium 1.32M in hexane (5.55 ml, 7.32 mmoles). The mixture was stirred under argon at -78°C for 1 hr. To the resulting red-orange solution, the chloride

prepared in 2F in tetrahydrofuran (10 ml) was added dropwise at -78°C. The mixture was stirred for 1 hr at -78°C, then warmed to 0°C within 2 hrs and stirred 30 min at 0°C. The reaction was quenched with saturated aqueous ammonium chloride and tetrahydrofuran was evaporated under reduced pressure. The resulting oil was diluted with methylene chloride and washed with water. The organic layer was dried over magnesium sulfate. Filtration and concentration under reduced pressure afforded an oil. This oil was dissolved in ether (36.5 ml) and was stirred at room temperature during 2 hrs with 2N aqueous solution of hydrochloric acid (36.5 ml). The organic layer was washed twice with water, dried over magnesium sulfate, filtered and concentrated under reduced pressure to afford an oil (1.45 g). Flash chromatography on silicagel and elution with a 25:75 mixture of ethyl acetate and hexane afforded the desired aldehyde (1.014 g, 56%) as an oil. NMR ($H^1$, $CDCl_3$, 60 MHz) characteristic peaks: 4.01 (t, $J_{HH}$ = 7 Hz, 1), 5.13 (dt, $J_{HF}$ = 21 Hz, $J_{HH}$ = 7 Hz, 1), 9.4 (t, $J_{HH}$ = 1 Hz, 1).

2H) PREPARATION OF (E) 7-(1,3-DITHIA-2-CYCLOHEXYL)-5-FLUORO-5-HEPTENOL

The aldehyde prepared in 2G (0.937 g, 3.77 mmoles) was dissolved in methanol (20 ml) and cooled to 0°C. Sodium borohydride (0.071 g, 1.87 mmoles) was added and the mixture was stirred 30 min. Acetone was added to react with an excess of sodium borohydride and then the reaction mixture was acidified with acetic acid. The solvents were evaporated under reduced pressure. The residue was diluted with ether and washed with water. The organic phase was dried over sodium sulfate, filtered and concentrated under reduced pressure to afford the title alcohol as an oil in a quantitative yield. NMR ($H^1$, $CDCl_3$, 60 MHz) characteristic peaks 4 (t, $J_{HH}$ = 7 Hz, 1), 5.1 (dt, $J_{HF}$ = 21 Hz, $J_{HH}$ = 8 Hz, 1).

2I) PREPARATION OF (E) 1-(t-BUTYLDIPHENYLSILYLOXY)-7-(1,3-DITHIA 2-CYCLOHEXYL)-5-FLUORO-5-HEPTENE

To a solution of the alcohol prepared in 2H (2.15 g, 9.26 mmoles) in dry methylene chloride (50 ml) was added triethylamine (2 ml, 14.3 mmoles), t-butyldiphenylchlorosilane (2.65 ml, 10.2 mmoles) and dimethylaminopyridine (45 mg). The mixture was stirred overnight at room temperature. The reaction mixture was washed once with water and then dried over sodium sulfate. Filtration and evaporation under reduced pressure afforded an oil. Flash chromatography on silicagel and elution with a 8:92 mixture of ethyl acetate and hexane afforded the desired silylether as an oil (4.12 g, 94%). NMR ($H^1$, $CDCl_3$, 60 MHz) characteristic peaks 1.06 (s, 9), 3.96 (t, $J_{HH}$ = 7 Hz, 1), 5.06 (dt, $J_{HF}$ = 21 Hz $J_{HH}$ = 8 Hz, 1), 7.23 to 7.80 (m, 10).

2J) PREPARATION OF (E) 8-(t-BUTYLDIPHENYLSILYLOXY)-4-FLUORO-3-OCTENAL

To a suspension of trimethyloxonium tetrafluoroborate (0.44 g, 2.97 mmoles) in dry methylene chloride (15 ml) was added at room temperature the dithialane prepared in 2I (1.45 g, 2.97 mmoles) and the mixture was stirred for 1 hr. Then a 9:1 mixture of acetone and water ( 5 ml) containing calcium carbonate (0.6 g, 5.94 mmoles) was added and the mixture was stirred overnight at room temperature. The precipitate was filtered off and after dilution with saturated brine the mixture was extracted three times with ether. The organic layer was dried over sodium sulfate, filtered and concentrated under reduced pressure to afford the expected aldehyde as an oil which was used after drying under high vacuum (1.19 g, 88%).

2K) PREPARATION OF ETHYL 2,6-DIFLUORO-10-DIPHENYL t-BUTYL SILYLOXY DECANE-2,5-DIENEOATE

n-BuLi 1.5 M in hexane solution (4.3 ml) was added at - 78°C to a solution of diisopropylamine (0.9 ml) in tetrahydrofuran (25 ml). The mixture was stirred during 20 min. at -78°C. Then triethylphosphonofluoro acetate (1.52 g, 6.28 mmoles) in tetrahydrofuran (3 ml) was added dropwise. The mixture was stirred 5 min. at -78°C and then 20 min. at 0°C. Then (E)-8-(t-butyldiphenylsilyloxy)-4-fluoro-3-octenal (2.5 g, 6.28 mmoles) in tetrahydrofuran (3 ml) was added dropwise at -78°C. The reaction mixture was stirred overnight at 0°C. The mixture was hydrolyzed with a saturated aqueous solution of ammonium chloride and extracted with ether. The organic layer was washed with saturated brine, dried over sodium sulfate and concentrated under reduced pressure. Flash chromatography on silica gel and elution with a 8:2 mixture of hexane and ethyl acetate afforded the expected ester as an oil (1.97 g, 61%).

2L) PREPARATION OF (E,E)-2,6-DIFLUORO-10-DIPHENYL t-BUTYLSILYLOXY DECANE-2,5-DIENE-OL

The ester prepared in 2K (1.97 g, 3.83 mmoles) in anhydrous ether (5 ml) was added at -78°C to a mixture of DIBAL, 1M solution in hexane, (7.7 ml, 7.7 mmoles) and ether (25 ml). The mixture was stirred 30 min. at -78°C, then overnight at room temperature. The excess of DIBAL was destroyed with methanol (2 ml) and the aluminum salts were precipitated with an aqueous saturated solution of ammonium chloride until a filtrable precipitate was obtained. The mixture was filtrated and the precipitate washed with ethyl acetate. The solvents were evaporated under reduced pressure. Flash chromatography over silica gel and elution with a 8:2 mixture of hexane and ethyl acetate afforded the expected alcohol as an oil (1.65 g, 91%).

2M) PREPARATION OF (E,E)-1-BROMO-2,6-DIFLUORO-10-DIPHENYL t-BUTYLSILYLOXYDECANE-2,5-DIENE

The alcohol prepared in 2L (1.65 g, 3.5 mmoles) was dissolved in dry methylene chloride (1.0 ml). The mixture was cooled to 0°C and 1-bromo N,N-2-trimethylpropenylamine (0.63 g, 3.5 mmoles) was added. The mixture was stirred under argon for 30 min. The methylene chloride was evaporated under reduced pressure. Flash chromatography on silica gel and elution with a mixture of hexane and ethyl acetate (98:2) afforded the expected bromide as an oil (1.82 g, 97%).

2N) PREPARATION OF (E,E)-1-(1,3-DITHIA-2-CYCLOHEXYL)-2,6-DIFLUORO-10-DIPHENYL t-BUTYLSILYLOXYDECANE-2,5-DIENE

To a solution of 1,3-dithiane (0.42 g, 3.5 mmoles) in tetrahydrofuran (50 ml) cooled to -25°C was added dropwise a 1.5M solution of n-butyllithium in hexane (12.2 ml, 3.4 mmoles). The mixture was stirred at -30°C for 30 min. Then the mixture was cooled to -40°C and the bromide prepared in 2M (1.82 g, 3.4 mmoles) in tetrahydrofuran (10 ml) was added dropwise. The reaction mixture was stirred 30 min at-40°C and 2 hrs. at 0°C. The reaction was quenched with saturated aqueous ammonium chloride and the tetrahydrofuran was evaporated under reduced pressure. The residue was diluted with ether and washed with water. The organic layer was dried over sodium sulfate, filtered and concentrated under reduced pressure. Flash chromatography on silica gel and elution with a 8:2 mixture of hexane and ethyl acetate afforded the title dithialane as an oil (1.58 g, 81%).

2O) PREPARATION OF (E,E)-3,7-DIFLUORO-11-DIPHENYL t-BUTYLSILYLOXYUNDECANOL-3,6-DIENE

To a suspension of trimethyloxonium tetrafluoroborate (0.41 g, 2.75 mmoles) in dry methylene chloride (75 ml) was added at room temperature the dithialane prepared in 2N (1.58 g, 2.75 mmoles) and the mixture was stirred for 1 hr. Then a 9:1 mixture of acetone and water (5 ml) containing calcium carbonate (0.6 g, 5.94 mmoles) was added and the mixture was stirred overnight at room temperature. The precipitate was filtered off and after dilution with saturated brine the mixture was extracted three times with ether. The organic layer was dried over sodium sulfate, filtered and concentrated under reduced pressure to afford an oil. The resulting oil was dissolved in ethanol (5 ml) and sodium borohydride (56 mg, 1.48 mmoles) was added. The mixture was stirred 30 min. at 0°C. The excess of sodium borohydride was reacted with acetone. The mixture was acidified with acetic acid and concentrated under reduced pressure. The residue was taken with water and extracted three times with ether. The organic layer was dried over sodium sulfate, filtered and concentrated under reduced pressure to afford an oil. Flash chromatography on silica gel and elution with a 28:72 mixture of ethyl acetate and hexane afforded the title alcohol as an oil (1.02 g, 75%).

2P) PREPARATION OF (E,E)-1-BROMO-3,7-DIFLUORO-11-DIPHENYL t-BUTYLSILYLOXYUNDECANE-3,6-DIENE

The alcohol prepared in 2O (1.02 g, 2.1 mmoles) was dissolved in dry methylene chloride (10 ml). The mixture was cooled to 0°C and 1-bromo-N,N-2-trimethylpropenylamine (0.374 g, 2.1 mmoles) was added. The mixture was stirred under argon for 15 min. Methylene chloride was evaporated under reduced pressure. Flash chromatography on silica gel and elution with a 95:5 mixture of hexane and ethyl acetate afforded the expected bromide as an oil (1.098 g, 95%).

2Q) PREPARATION OF (E,E)-3,7-DIFLUORO-11-(DIPHENYL t-BUTYLSILYLOXY)UNDECANYL-3,6-DIENE TRIPHENYLPHOSPHONIUM BROMIDE

A mixture of the bromide prepared in 2P (1.098 g, 2 mmoles) and triphenylphosphine (0.53 g, 2 mmoles) in dry acetonitrile (10 ml) were refluxed for 48 hrs. Evaporation of the solvent under reduced pressure, flash chromatography on silica gel and elution with a 9:1 mixture of methylene chloride and methanol afforded the expected phosphonium bromide as a foam (1.535 g, 90%).

2R) PREPARATION OF 5,9-DIFLUORO-1-(DIPHENYL t-BUTYLSILYLOXY)-5,8,11,14-EICOSATETRAENE

To a solution of diisopropylamine (0.25 ml, 1.8 mmoles) in tetrahydrofuran (15 ml) cooled to -78 ° C was added dropwise n-butyllithium 1.6M in hexane solution (1.12 ml, 1.8 mmoles). The mixture was warmed to -10 ° C and then cooled again to - 78 ° C. The phosphonium bromide prepared in 2Q (1.53 g, 1.8 mmoles) in tetrahydrofuran (5 ml) was added dropwise and the mixture was stirred 30 min. at -78 ° C. Hexamethylphosphonictriamide (0.8 ml) was added and the reaction mixture was warmed to -30 ° C. 2,3-Nonenal (0.252 g, 1.8 mmole) in tetrahydrofuran (3 ml) was added dropwise and the mixture was stirred 2 hrs. at -30 ° C and 30 min. at 0 ° C. Saturated aqueous solution of ammonium chloride was added and tetrahydrofuran was evaporated under reduced pressure. The residue was taken up with water and extracted three times with ether. The organic layer was washed twice with water and dried over sodium sulfate. Filtration and evaporation of the solvent afforded an oil. Flash chromatography on silica gel and elution with a 9:1 mixture of hexane and benzene afforded the expected triene (0.544 g, 51%).

2S) PREPARATION OF 5,9-DIFLUOROEICOSATETRAENOL

To a solution of the silylether prepared in 2R (0.544 g, 0.92 mmole) in tetrahydrofuran (10 ml) was added tetra-n-butylammonium fluoride trihydrate (410 mg, 1.3 mmole). The mixture was stirred at room temperature for 2 hr. The solvent was evaporated under reduced pressure. The residue was dissolved in methylene chloride, washed with water and dried over sodium sulfate. Filtration and concentration under reduced pressure afforded an oil. Flash chromatography on silica gel and elution with a 15:85 mixture of ethyl acetate and benzene afforded the expected alcohol as an oil (282 mg, 94%).

2T) PREPARATION OF 5,9-DIFLUOROEICOSATETRAENOIC ACID

To a solution of the alcohol prepared in 2S (282 mg, 0.86 mmoles) in acetone (7 ml) cooled to 0 ° C was added dropwise 2.67M Jones reagent over 15 min. until the orange color was stable. The mixture was stirred 15 min. at 0 ° C. The excess of Jones reagent was reacted with isopropanol. The acetone was evaporated under reduced pressure without heating. The residue was taken up with water and extracted three times with ethyl acetate. The organic layer was dried over sodium sulfate, filtered and concentrated under reduced pressure to leave an oil. Flash chromatography on silica gel and elution with a 15:85 mixture of ethyl acetate and benzene gave pure acid (180 mg, 61%).

The dithiane 1 and the aldehyde 2 are described during the synthesis of 6-fluoroarachidonic acid.

EXAMPLE 3

PREPARATION OF 6,9-DIFLUOROEICOSATETRAENOIC ACID

3A) PREPARATION OF (E) 6-FLUORO-7-(2-TETRAHYDROPYRANYLOXY)-5-HEPTENAL

To a solution of N-allyl-N,N'N''-pentamethylphosphoramide (2.50 g, 11.99 mmoles) in tetrahydrofuran (30 ml) cooled to - 78 ° C was added dropwise n-butyllithium 1.55 M in hexane (7.74 ml, 11.93 mmoles). The mixture was stirred under argon at - 78 ° C for 1 hr. To the resulting red-orange solution the chloride prepared in 2C in tetrahydrofuran (15 ml) was added dropwise at -78 ° C. The mixture was stirred for 1 hr at -78 ° C, then warmed up to 0 ° C within 2 hrs and stirred 1 hr at 0 ° C. The reaction was quenched with saturated aqueous ammonium chloride and the tetrahydrofuran was evaporated under reduced pressure. The resulting oil was diluted with methylene chloride and washed with water. The organic layer was dried over magnesium sulfate. Filtration and concentration under reduced pressure afforded an oil. This oil was dissolved in ether (60 ml) and was stirred at room temperature for 2 hrs with a 2N aqueous solution of hydrochloric acid (60 ml). The organic layer was washed twice with water, dried over magnesium sulfate,

filtered and concentrated under reduced pressure to afford an oil (2.10 g). The NMR of the crude mixture showed that the THP has been mostly cleaved. To a solution of the crude oil in methylene chloride (100 ml) was added dihydropyran (2.1 ml) and pyridinium paratoluene sulfonate (0.236 g) and the mixture was stirred overnight at room temperature. The reaction mixture was washed with water. The organic layer was dried over sodium sulfate. Filtration and concentration under reduced pressure afforded an oil (3 g). Flash chromatography on silicagel and elution with a 25:75 mixture of ethyl acetate and hexane afforded the aldehyde (1.74 g, 64%) as an oil. NMR ($H^1$, $CDCl_3$, 60 MHz) characteristic peaks: 4.18 (AB part of an ABX system,

$$J_{H_AH_B}=13\ Hz,\ J_{H_AF}=20.5\ Hz,\ J_{H_BF}=24.6\ Hz,\ 2),$$

4.68 (t, $J_{HH}$ = 3.4 Hz, 1), 5.25 (dt, $J_{HH}$ = 8.2 Hz, $J_{HF}$ = 20.4 Hz, 1), 9.77 (t, $J_{HH}$ = 1.5 Hz, 1).

3B) PREPARATION OF (E) 6-FLUORO-7,12-TETRAHYDROPYRANYLOXY-5-HEPTANOL

The aldehyde prepared in 3A (1.34 g, 7.56 mmoles) was dissolved in methanol (20 ml) and cooled to 0 ° C. Sodium borohydride (0.143 g, 3.78 mmoles) was added and the mixture was stirred 30 min. Acetone was added to react with the excess of sodium borohydride. The solvents were evaporated under reduced pressure. The residue was diluted with ether and washed with water. The organic phase was dried over sodium sulfate, filtered and concentrated under reduced pressure to afford pure alcohol as an oil (1.66 g) which was used to the next step without purification.

3C) PREPARATION OF (E) 1-(t-BUTYLDIPHENYLSILYLOXY)-6-FLUORO-7-(2-TETRAHYDROPYRANYLOXY)-5-HEPTENE

To a solution of the alcohol prepared in 3B (1.66 g, 7.15 mmoles) in dry methylene chloride (50 ml) was added triethylamine (1.7 ml, 11.34 mmoles), t-butyldiphenylchlorosilane (1.7 ml, 8.31 mmoles) and dimethylaminopyridine (40 mg). The mixture was stirred overnight at room temperature. The reaction mixture was washed once with water and then dried over sodium sulfate. Filtration and evaporation under reduced pressure afforded an oil. Flash chromatography on silicagel and elution with a 10:90 mixture of ethyl acetate and hexane afforded the silylether as an oil (2.87 g).

3D) PREPARATION OF (E) 1-(t-BUTYLDIPHENYLSILYLOXY)-6-FLUORO-5-HEPTENE-7-OL

The tetrahydropyranyl derivative prepared in 3C (2.26 g, 4.8 mmoles) was dissolved in methanol. Tetrabutyl-1,3-diisothiocyanatodistannoxane (30 mg) was added and the mixture was refluxed for 24 hrs. Methanol was evaporated under reduced pressure. The residue was dissolved in ether and washed with water. The organic layer was dried over sodium sulfate, filtered and concentrated under reduced pressure. Flash chromatography on silicagel and elution with a 8:2 mixture of hexane and ethyl acetate afforded the alcohol as an oil (1.65 g, 92%).

3E) PREPARATION OF (E) 7-BROMO-1-(t-BUTYLDIPHENYLSILYLOXY)-6-FLUORO-5-HEPTENE

The alcohol prepared in 3D (1.1 g, 2.85 mmoles) was dissolved in dry methylene chloride (20 ml). The mixture was cooled to 0 ° C and 1-bromo-N,N-2-trimethylpropenylamine (0.51 g, 2.85 mmoles) was added. The mixture was stirred under argon for 15 min. Methylene chloride was evaporated under reduced pressure. Flash chromatography on silicagel and elution with a 95:5 mixture of hexane and ethyl acetate afforded the expected bromide as an oil (1.24 g, 98%). NMR ($H^1$, $CDCl_3$, 60 MHz) characteristic peaks: 1.05 (s, 9), 3.65 (m, 2), 3.91 (d, $J_{HF}$ = 22 Hz, 2), 5.23 (dt, $J_{HF}$ = 19 Hz, $J_{HH}$ = 7.5 Hz, 1), 7.26 to 7.78 (m, 10).

3F) PREPARATION OF (E)-1-(t-BUTYLDIPHENYLSILYLOXY)-7-(1,3-DITHIA-2-CYCLOHEXYL)-6-FLUORO-5-HEPTENE

To a solution of dithialane (0.365 g, 3.04 mmole) in tetrahydrofuran (50 ml) cooled to -30 ° C was added dropwise a 1.5M solution of n-butyllithium in hexane (2 ml, 3 mmoles) and the mixture was stirred at -30 ° C for 30 min. Then the mixture was cooled to -40 ° C and the bromide prepared in 3E (1.24 g, 2.76 mmoles) in tetrahydrofuran (10 ml) was added dropwise. The reaction mixture was stirred 30 min at -40 ° C and 2 hrs at

0°C and then quenched with saturated aqueous ammonium chloride and the tetrahydrofuran was evaporated under reduced pressure. The residue was diluted with ether and washed with water. The organic layer was dried over sodium sulfate, filtered and concentrated under reduced pressure. Flash chromatography on silicagel and elution with a 95:5 mixture of hexane and ethyl acetate afforded the desired dithialane as an oil (0.524 g, 40%). NMR ($H^1$, $CDCl_3$, 60 MHz) characteristic peaks: 1.03 (s, 9), 3.61 (m, 2), 4.23 (t, $J_{HH}$ = 7.5 Hz, 1), 5.3 (dt, $J_{HF}$ = 21 Hz, $J_{HH}$ = 7.5 Hz, 1), 7.16 to 7.83 (m, 10).

3G) PREPARATION OF (E) 8-(t-BUTYLDIPHENYLSILYLOXY)-3-FLUORO-3-OCTENAL

To a solution of the dithialane prepared in 3F (0.424 g, 0.86 mmoles) in dry methylene chloride (4 ml) was added at room temperature trimethyloxonium tetrafluoroborate (0.125 g, 0.86 mmoles) and the mixture was stirred for 1 hr. Then a 9:1 mixture of acetone and water (2 ml) containing calcium carbonate (0.172 g, 1.72 mmoles) was added and the mixture was stirred overnight at room temperature. The precipitate was filtered off and after dilution with saturated brine the mixture was extracted three times with ether. The organic layer was dried over sodium sulfate, filtered and concentrated under reduced pressure to afford the expected aldehyde as an oil which was used after drying under high vacuum (0.366 g, 92%).

3H) PREPARATION OF ETHYL 2,5-DIFLUORO-10-DIPHENYL t-BUTYL SILYLOXYDECANE-2,5-DIENEOATE

n-BuLi 1.5 M in hexane solution (4.3 ml) was added at - 78°C to a solution of diisopropylamine (0.9 ml) in tetrahydrofuran (25 ml). The mixture was stirred during 20 min. at -78°C. Then triethylphosphonofluoro acetate (1.52 g, 6.28 mmoles) in tetrahydrofuran (3 ml) was added dropwise. The mixture was stirred 5 min. at -78°C and then 20 min. at 0°C. Then (E) 8-(t-butyldiphenylsilyloxy)-3-fluoro-3-octenal (2.5 g, 6.28 mmoles) in tetrahydrofuran (3 ml) was added dropwise at -78°C. The reaction mixture was stirred overnight at 0°C. The mixture was hydrolyzed with a saturated aqueous solution of ammonium chloride and extracted with ether. The organic layer was washed with saturated brine, dried over sodium sulfate and concentrated under reduced pressure. Flash chromatography on silica gel and elution with a 8:2 mixture of hexane and ethyl acetate afforded the expected ester as an oil (1.97 g, 61%).

3I) PREPARATION OF (E,E)-2,5-DIFLUORO-10-DIPHENYL t-BUTYLSILYLOXYDECANE-2,5-DIENEOL

The ester prepared in 3H (1.97 g, 3.83 mmoles) in anhydrous ether (5 ml) was added at -78°C to a mixture of DIBAL, 1M solution in hexane, (7.7 ml, 7.7 mmoles) and ether (25 ml). The mixture was stirred 30 min. at -78°C, then overnight at room temperature. The excess of DIBAL was destroyed with methanol (2 ml) and the aluminum salts were precipitated with an aqueous saturated solution of ammonium chloride until a filtrable precipitate was obtained. The mixture was filtrated and the precipitate washed with ethyl acetate. The solvents were evaporated under reduced pressure. Flash chromatography over silica gel and elution with a 8:2 mixture of hexane and ethyl acetate afforded the expected alcohol 3 as an oil (1.65 g, 91%).

3J) PREPARATION OF (E,E)-1-BROMO-2,5-DIFLUORO-10-DIPHENYL t-BUTYLSILYLOXYDECANE-2,5-DIENE

The alcohol prepared in 3I (1.65 g, 3.5 mmoles) was dissolved in dry methylene chloride (1.0 ml). The mixture was cooled to 0°C and 1-bromo-N,N-2-trimethylpropenylamine (0.63 g, 3.5 mmoles) was added. The mixture was stirred under argon for 30 min. The methylene chloride was evaporated under reduced pressure. Flash chromatography on silica gel and elution with a mixture of hexane and ethyl acetate (98:2) afforded the expected bromide as an oil (1.82 g, 97%).

3K) PREPARATION OF (E,E)-1-(1,3-DITHIA-2-CYCLOHEXYL)-2,5-DIFLUORO-10-DIPHENYL t-BUTYLSILYLOXYDECANE-2,5-DIENE

To a solution of 1,3-dithiane (0.42 g, 3.5 mmoles) in tetrahydrofuran (50 ml) cooled to -25°C was added dropwise a 1.5M solution of n-butyllithium in hexane (12.2 ml, 3.4 mmoles). The mixture was stirred at -30°C for 30 min. Then the mixture was cooled to -40°C and the bromide prepared in 3J (1.82 g, 3.4 mmoles) in tetrahydrofuran (10 ml) was added dropwise. The reaction mixture was stirred 30 min at-40°C and 2 hrs. at 0°C. The reaction was quenched with saturated aqueous ammonium chloride and the tetrahydrofuran was evaporated under reduced pressure. The residue was diluted with ether and washed

with water. The organic layer was dried over sodium sulfate, filtered and concentrated under reduced pressure. Flash chromatography on silica gel and elution with a 8:2 mixture of hexane and ethyl acetate afforded the title dithialane as an oil (1.58 g, 81%).

3L) PREPARATION OF (E,E)-3,6-DIFLUORO-11-DIPHENYL t-BUTYLSILYLOXYUNDECANOL-3,6-DIENE

To a suspension of trimethyloxonium tetrafluoroborate (0.41 g, 2.75 mmoles) in dry methylene chloride (75 ml) was added at room temperature the dithialane prepared in 3K (1.58 g, 2.75 mmoles) and the mixture was stirred for 1 hr. Then a 9:1 mixture of acetone and water (5 ml) containing calcium carbonate (0.6 g, 5.94 mmoles) was added and the mixture was stirred overnight at room temperature. The precipitate was filtered off and after dilution with saturated brine the mixture was extracted three times with ether. The organic layer was dried over sodium sulfate, filtered and concentrated under reduced pressure to afford an oil. The resulting oil was dissolved in ethanol and sodium borohydride (56 mg, 1.48 mmoles) was added. The mixture was stirred 30 min. at 0 °C. The excess of sodium borohydride was reacted with acetone. The mixture was acidified with acetic acid and concentrated under reduced pressure. The residue was taken with water and extracted three times with ether. The organic layer was dried over sodium sulfate, filtered and concentrated under reduced pressure to afford an oil. Flash chromatography on silica gel and elution with a 28:72 mixture of ethyl acetate and hexane afforded the title alcohol as an oil (1.02 g, 75%).

3M) PREPARATION OF (E,E)-1-BROMO-3,6-DIFLUORO-11-(DIPHENYL t-BUTYLSILYLOXY)UNDECANE-3,6-DIENE

The alcohol prepared in 3L (1.02 g, 2.1 mmoles) was dissolved in dry methylene chloride (10 ml). The mixture was cooled to 0 °C and 1-bromo-N,N-2-trimethylpropenylamine (0.374 g, 2.1 mmoles) was added. The mixture was stirred under argon for 15 min. Methylene chloride was evaporated under reduced pressure. Flash chromatography on silica gel and elution with a 95:5 mixture of hexane and ethyl acetate afforded the expected bromide as an oil (1.098 g, 95%).

3N) PREPARATION OF (E,E)-3,6-DIFLUORO-11-(DIPHENYL t-BUTYLSILYLOXY)UNDECANYL-3,6-DIENE TRIPHENYLPHOSPHONIUM BROMIDE

A mixture of the bromide prepared in 3M (1.098 g, 2 mmoles) and triphenylphosphine (0.53 g, 2 mmoles) in dry acetonitrile (10 ml) were refluxed for 48 hrs. Evaporation of the solvent under reduced pressure, flash chromatography on silica gel and elution with a 9:1 mixture of methylene chloride and methanol afforded the expected phosphonium bromide as a foam (1.535 g, 90%).

3O) PREPARATION OF 6,9-DIFLUORO-1-(DIPHENYL t-BUTYLSILYLOXY)-5,8,11,14-EICOSATETRAENE

To a solution of diisopropylamine (0.25 ml, 1.8 mmoles) in tetrahydrofuran (15 ml) cooled to -78 °C was added dropwise n-butyllithium 1.6M in hexane solution (1.12 ml, 1.8 mmoles). The mixture was warmed to -10 °C and then cooled again to -78 °C. The phosphonium bromide prepared in 3N (1.53 g, 1.8 mmoles) in tetrahydrofuran (5 ml) was added dropwise and the mixture was stirred 30 min. at -78 °C. Hexamethylphosphonictriamide (0.8 ml) was added and the reaction mixture was warmed to -30 °C. 2,3-Nonenal (0.252 g, 1.8 mmole) in tetrahydrofuran (3 ml) was added dropwise and the mixture was stirred 2 hrs. at -30 °C and 30 min. at 0 °C. Saturated aqueous solution of ammonium chloride was added and tetrahydrofuran was evaporated under reduced pressure. The residue was taken up with water and extracted three times with ether. The organic layer was washed twice with water and dried over sodium sulfate. Filtration and evaporation of the solvent afforded an oil. Flash chromatography on silica gel and elution with a 9:1 mixture of hexane and benzene afforded the expected triene (0.544 g, 51%).

3P) PREPARATION OF 6,9-DIFLUOROEICOSATETRAENOL

To a solution of the silylether prepared in 3O (10.544 g, 0.92 mmole) in tetrahydrofuran (10 ml) was added tetra-n-butylamonium fluoride trihydrate (410 mg, 1.3 mmole). The mixture was stirred at room temperature for 2 hr. The solvent was evaporated under reduced pressure. The residue was dissolved in methylene chloride, washed with water and dried over sodium sulfate. Filtration and concentration under reduced pressure afforded an oil. Flash chromatography on silica gel and elution with a 15:85 mixture of ethyl acetate and benzene afforded the expected alcohol as an oil (282 mg, 94%).

3Q) PREPARATION OF 6,9-DIFLUOROEICOSATETRAENOIC ACID

To a solution of the alcohol prepared in 3P (282 mg, 0.86 mmoles) in acetone (7 ml) cooled to 0 ° C was added dropwise 2.67M Jones reagent over 15 min. until the orange color was stable. The mixture was stirred 15 min. at 0 ° C. The excess of Jones reagent was reacted with isopropanol. The acetone was evaporated under reduced pressure without heating. The residue was taken up with water and extracted three times with ethyl acetate. The organic layer was dried over sodium sulfate, filtered and concentrated under reduced pressure to leave an oil. Flash chromatography on silica gel and elution with a 15:85 mixture of ethyl acetate and benzene gave pure acid (180 mg, 61%).

**Claims**

**Claims for the following Contracting States : AT, BE, CH, DE, FR, GB, IT, LI, LU, NL, SE**

**1.** A fluorinated arachidonic acid derivative of the formula

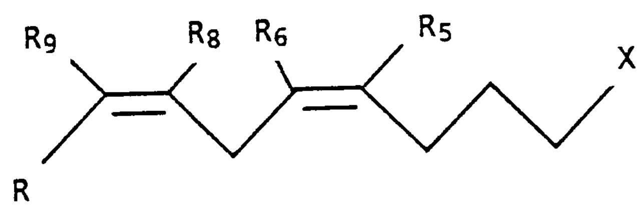

wherein

one of $R_5$ and $R_6$ is a fluoro group and the other is a hydrogen or both $R_5$ and $R_6$ individually are a hydrogen;

one of $R_8$ and $R_9$ is a fluoro group and the other is a hydrogen;

X is a C(O)OR' group wherein R' is a hydrogen, a straight chain $(C_1\text{-}C_6)$alkyl group, or

X is a group of the formula $-C(O)OCH_2CH(OR'')CH_2(OR''')$ wherein R'' is a long chain fatty acid residue and wherein R''' is a hydrogen or a long chain fatty acid residue, or

X is a $-C(O)NH_2$ or -C(O)NH(OH) group, or

X is a 1H-tetrazol-5-yl group; and

R is a group of one of the structural formulae

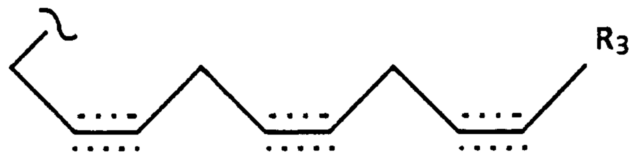

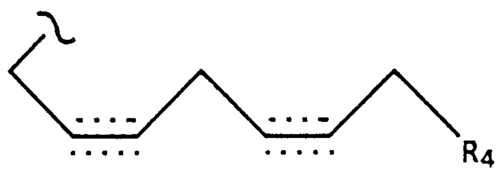

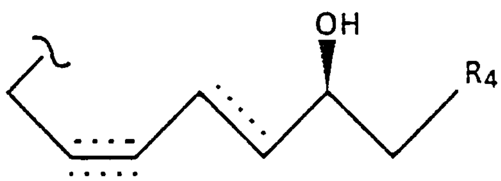

wherein

$R_3$ is a hydrogen or a straight chain ($C_1$-$C_4$)alkyl and $R_4$ is a hydrogen or a straight chain ($C_1$-$C_6$)-alkyl and wherein a dotted line indicates an optional double or triple bond or a pharmaceutically acceptable salt thereof.

**2.** A fluorinated arachidonic acid derivative of claim 1 wherein X is a $CO_2H$ group.

**3.** A fluorinated arachidonic acid derivative of one of claims 1 or 2 wherein X is a group of the formula $-C(O)OCH_2CH(OR'')CH_2(OR''')$ wherein R'' is a long chain, fatty acid residue and wherein R''' is a hydrogen or a long chain, fatty acid residue.

**4.** A fluorinated arachidonic acid derivative of one of claims 1 or 2 wherein R is a group of the structural formula

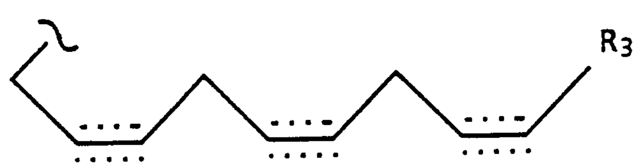

wherein

$R_3$ is a hydrogen or a straight chain ($C_1$-$C_4$)alkyl and wherein a dotted line indicates an optional double or triple bond.

**5.** A fluorinated arachidonic acid derivative or claim 4 wherein $R_3$ is an ethyl group.

**6.** A fluorinated arachidonic acid derivative of one of claims 1 or 2 wherein R is a group of the structural formula

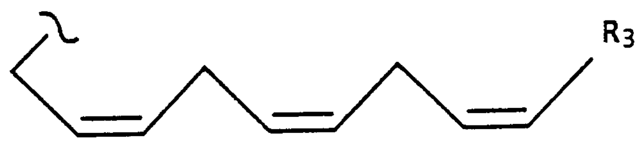

or

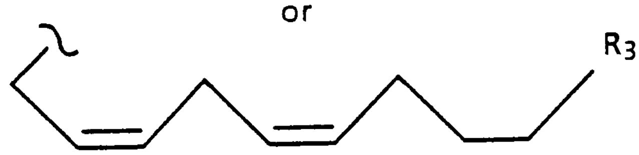

wherein

$R_3$ is a hydrogen or a straight chain $(C_1\text{-}C_4)$alkyl.

**7.** A fluorinated arachidonic acid derivative of claim 6 wherein $R_3$ is an ethyl group.

**8.** The use of fluorinated arachidonic acid derivatives according to claim 1 to 7 for the preparation of a medicine.

**9.** The use of fluorinated arachidonic acid derivative according to claims 1 to 7 for the preparation of a 5-lipoxygenase inhibitor.

**10.** The use of fluorinated arachidonic acid derivatives according to claims 1 to 7 for the preparation of an antiasthmatic.

**11.** Pharmaceutical composition containing a fluorinated arachidonic acid derivative as defined in claims 1 to 7 in combination with a pharmaceutically acceptable carrier.

**Claims for the following Contracting State : ES**

**1.** A method for the preparation of fluorinated arachidonic acid derivatives of the formula

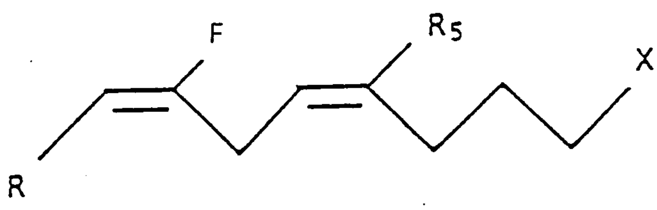

1

in which

$R_5$ is a hydrogen or a fluoro group ;

X is a -COOH group ; and

R is a group of one of the structural formulae

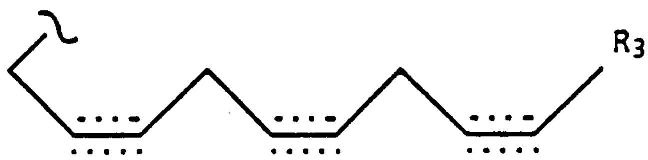

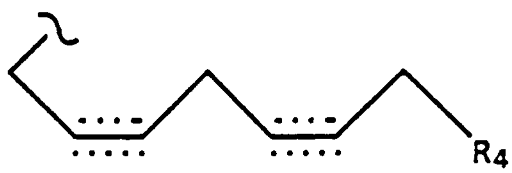

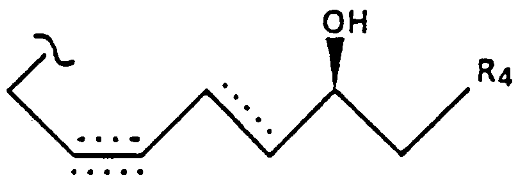

in which
$R_3$ is hydrogen or a straight chain ($C_1$-$C_4$) alkyl and $R_4$ is hydrogen or a straight chain ($C_1$-$C_6$) alkyl and a dotted line indicates an optional double or triple bond,
or of a pharmaceutically acceptable salt thereof,
which comprises :

a) condensing the ylid of the fluorinated phosphonate $(C_2H_5O)_2P(O)CHFCO_2(C_2H_5)$ with an aldehyde of the formula RCHO in which R is as defined above to give the ester of formula

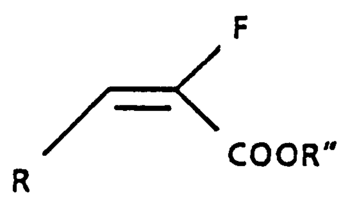

9

in which R is as defined above and R" is a lower alkyl, phenyl or benzyl group ;

b) reducing the ester 9 in an ethereal solvent with an aluminum hydride reducing agent to give the alcohol of formula

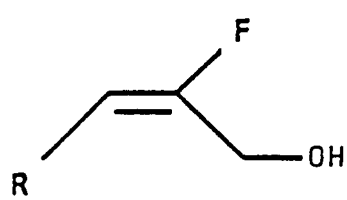

8a

in which R is as defined above ;

c) converting the alcohol 8a to the corresponding halide of formula

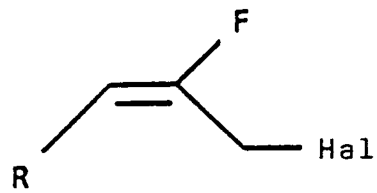

8

in which R is as defined above and Hal is a bromo or chloro group ;

d) treating the halide 8 with the anion of 1,3-dithiane to give the dithiane of formula

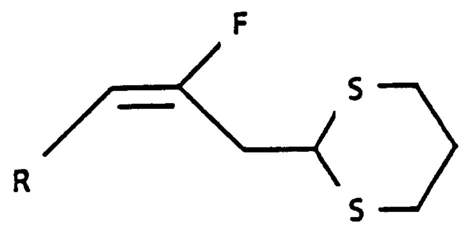

7

in which R is as defined above ;

e) hydrolyzing the dithiane 7 to form the aldehyde of formula

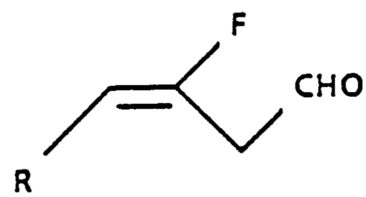

6

in which R is as defined above ;

f) reacting the aldehyde 6 with the ylid of triethylphospono fluoroacetate or triethylphosphono acetate to give the ester of formula

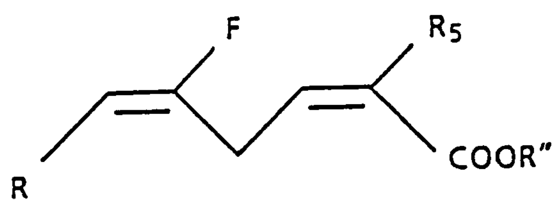

5

in which R and $R_5$ are as defined above and R'' is an alkyl or benzyl group ;

g) reducing the ester 5 in an ethereal solvent with an aluminum hydride reducing agent to form the alcohol of formula

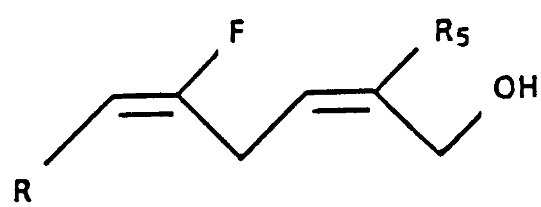

4

in which R and $R_5$ are as defined above ;

h) converting the alcohol 4 to the corresponding halide of formula

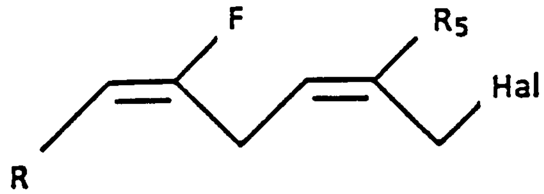

3

in which R and $R_5$ are as defined above and Hal is a chloro or bromo group ;

i) treating the halide 3 with N-allyl-N,N',N"-pentamethylphosphoramide to form the condensation product of formula

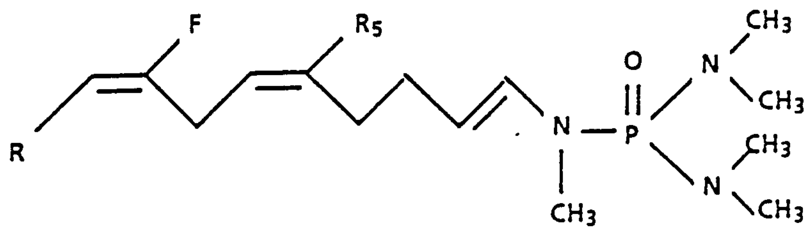

3a

in which R and $R_5$ are as defined above ;

j) hydrolyzing condensation product 3a in a mild acidic medium to give the aldehyde of formula

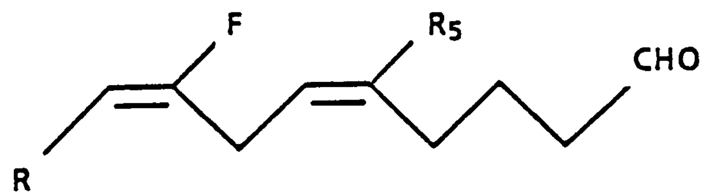

2

in which R and $R_5$ are as defined above ;

k) oxidizing the aldehyde 2 to obtain the desired compound 1 ; and

l) optionally, converting compound 1 to one of its pharmaceutically acceptable salts.

**2.** A method for the preparation of fluorinated arachidonic acid derivatives of the formula

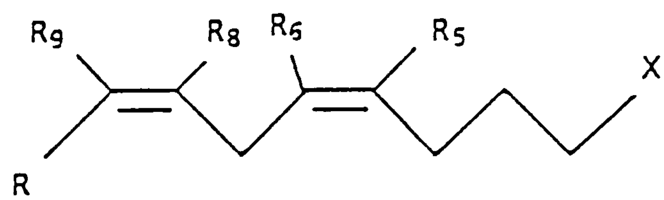

1

in which

one of $R_5$ and $R_6$ is a fluoro group and the other is a hydrogen or both $R_5$ and $R_6$ individually are a hydrogen;

one of $R_8$ and $R_9$ is a fluoro group and the other is a hydrogen ;

X is a C(O)OR' group wherein R' is a hydrogen, a straight chain ($C_1$-$C_6$)alkyl group, or

X is a group of the formula

$-C(O)OCH_2CH(OR'')CH_2(OR''')$ wherein R" is a long chain fatty acid residue and wherein R''' is a hydrogen or a long chain fatty acid residue, or

X is a $-C(O)NH_2$ or $-C(O)NH(OH)$ group, or

X is a 1H-tetrazol-5-yl group ; and
R has the following formula :

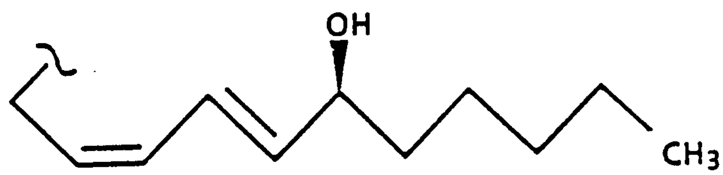

,

or of
a pharmaceutically acceptable salt thereof, which comprises :

a) treating D-Arabinose to give the dithioacetal of formula

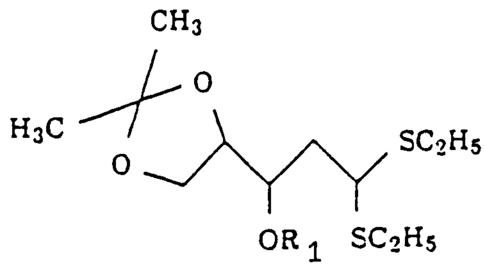

26

in which $R_1$ is $(t\text{-}C_4H_9)Si(C_6H_5)_2$ ;

b) reacting the dithioacetal 26 with mercuric oxide and calcium carbonate to give the silyloxy aldehyde of formula

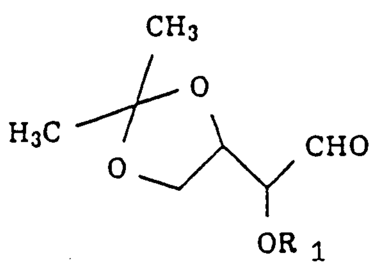

27

in which $R_1$ is $(t\text{-}C_4H_9)Si(C_6H_5)_2$ ;

c) reacting the silyloxy aldehyde 27 with the ylid of n-propylbromide and triphenylphosphine to give the silyloxyolefin of formula

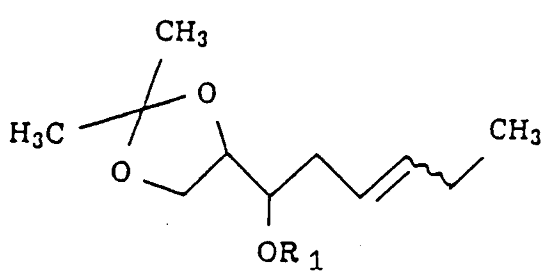

29

in which $R_1$ is $(t\text{-}C_4H_9)Si(C_6H_5)_2$ ;

d) reducing the silyloxyolefin 29 to give the silyloxy compound of formula

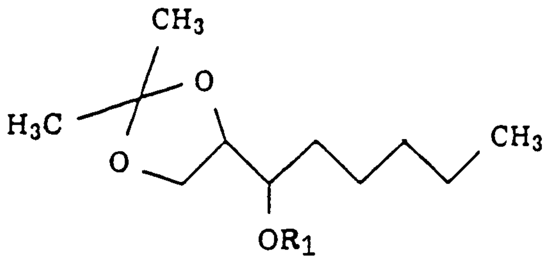

30

in which $R_1$ is $(t\text{-}C_4H_9)Si(C_6H_5)_2$ ;

e) converting the silyloxy compound 30 to the unsaturated aldehyde of formula

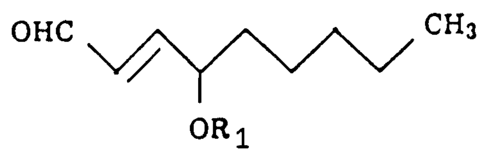

33

in which $R_1$ is $(t\text{-}C_4H_9)Si(C_6H_5)_2$, via the diol and the aldehyde analogs ;

f) reacting the aldehyde 33 with the ylid of the acetone ketal of 3,4-dihydroxy-iodobutane to give the diolefinketal of formula

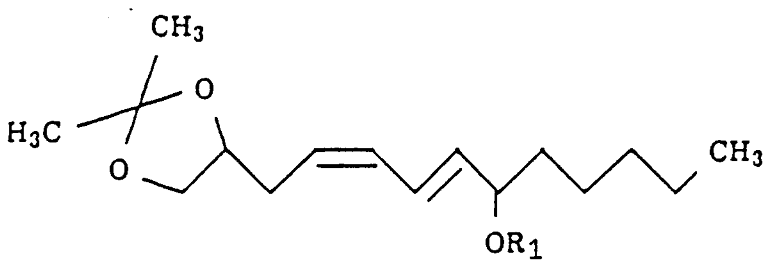

34

in which $R_1$ is $(t\text{-}C_4H_9)Si(C_6H_5)_2$ ;

g) hydrolysing then oxidizing the diolefinketal 34 to give a silyl ether derivative, and removing the silyl group of said silyl ether derivative to yield the aldehyde of formula

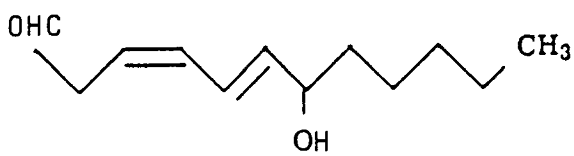

25,

i.e. an aldehyde of formula RCHO in which R is as defined above ;

h) repeating steps a) to l) set forth in claim 1.

3. A method for the preparation of fluorinated arachidonic acid derivatives of the formula

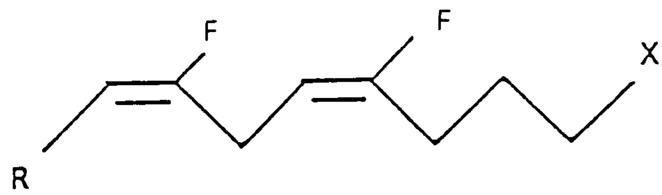

1

in which
X is a -COOH group ; and
R is as defined in claim 1,
or of a pharmaceutically acceptable salt thereof, which comprises :

a) reacting a chloro derivative of formula

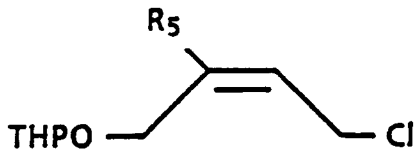

21

in which $R_5$ is a fluoro group ;
with the anion of 1,3-dithiane to give the dithialane derivative of formula

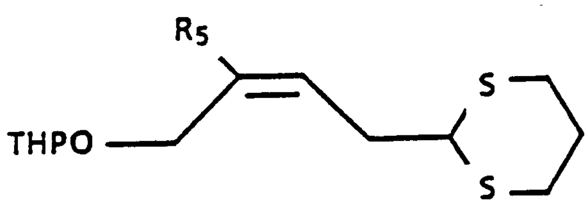

20

in which $R_5$ is a fluoro group ;

b) removing the THP-protecting group of compound 20 to give the alcohol of formula

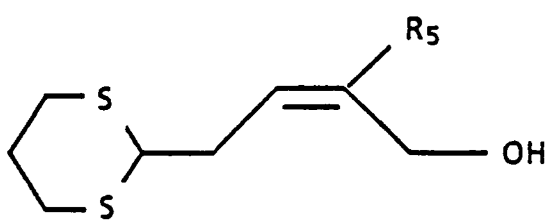

19

in which $R_5$ is a fluoro group ;

c) converting the alcohol 19 to the corresponding chloro derivative of formula

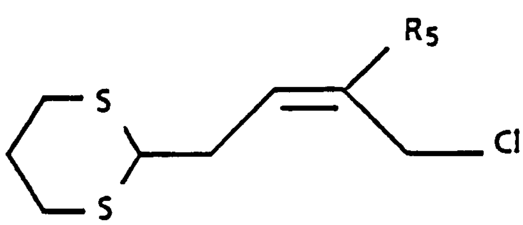

18

in which $R_5$ is a fluoro group ;

d) transforming the chlorinated compound 18 with N-allyl-N,N',N"-pentamethylphosphoramide into the aldehyde of formula

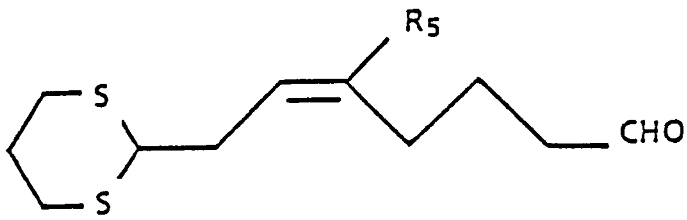

17

in which $R_5$ is a fluoro group ;

e) reducing the aldehyde 17 to give the alcohol of formula

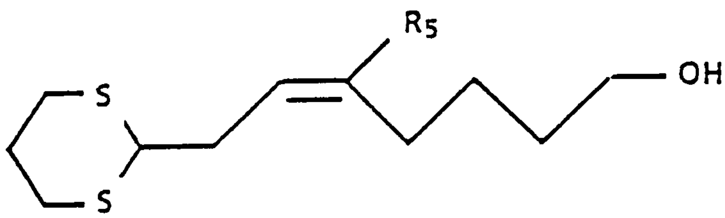

16

in which $R_5$ is a fluoro group ;

f) treating the alcohol 16 with t-butyldiphenylsilyl chloride to give the silylated dithialane of formula

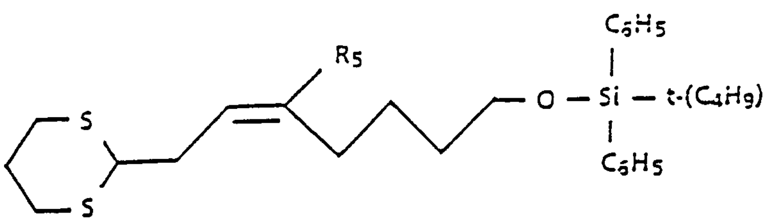

15

in which $R_5$ is a fluoro group ;

g) adding the 1,3-dithialane derivative 15 to a suspension of trimethyloxinium tetrafluoroborate to give the aldehyde of formula

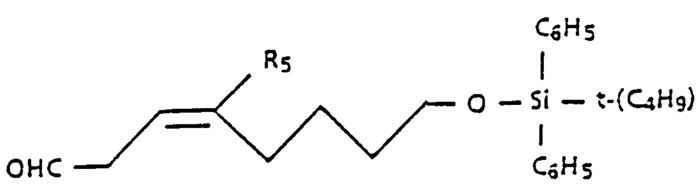

14

in which $R_5$ is a fluoro group ;

h) reacting the aldehyde 14 with the ylid of $(C_2H_5O)_2P(O)CHFCO_2C_2H_5$ to give the ester of formula

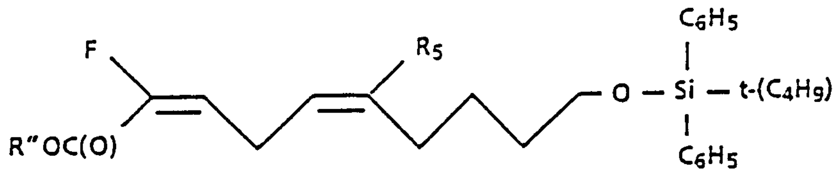
13

in which $R_5$ is a fluoro group and R'' is a lower alkyl, phenyl or benzyl group ;
i) reducing the ester 13 in an ethereal solvent with an aluminum hydride reducing agent to give the alcohol of formula

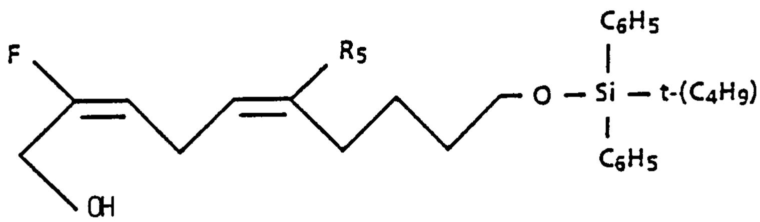
12a

in which $R_5$ is a fluoro group ;
j) converting the alcohol 12a to the corresponding halide of formula

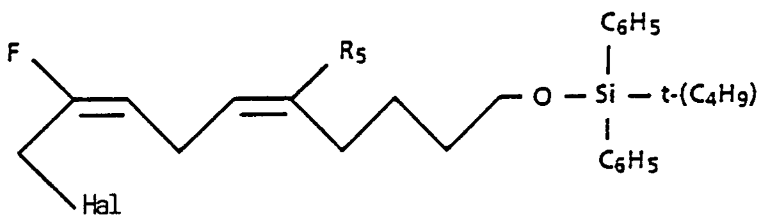
12

in which $R_5$ is a fluoro group and Hal is a chloro or bromo group ;
k) treating the halide 12 with the anion of 1,3-dithiane, hydrolyzing the intermediate dithialane compound, adding calcium carbonate to the resulting compound to isolate an intermediate aldehyde and reducing said aldehyde to yield the alcohol of formula

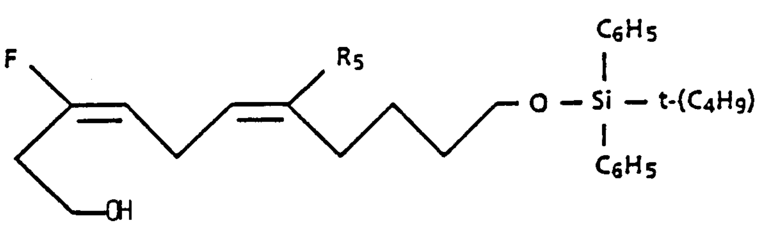
11a

in which $R_5$ is a fluoro group ;

l) converting the alcohol 11a to the corresponding halide of formula

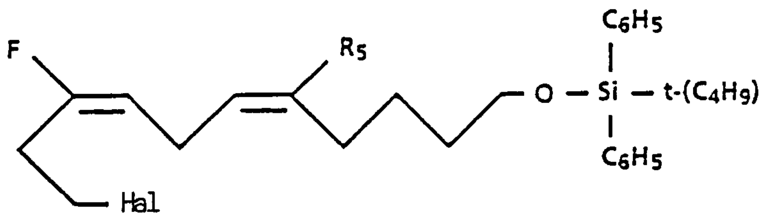

11

in which $R_5$ is a fluoro group and Hal is a chloro or bromo group ;
m) reacting the ylid of compound 11 with the appropriate aldehyde to give the alcohol of formula

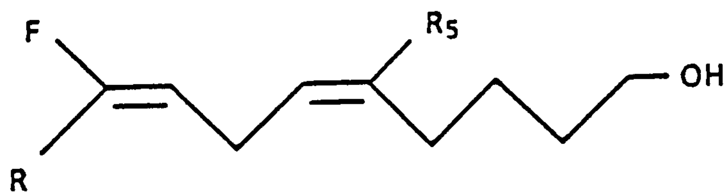

10

in which $R_5$ is a fluoro group and R is as defined above;
n) oxidizing the alcohol 10 to obtain the desired compound 1 ;
o) optionally, converting the compound 1 thus obtained to one of its pharmaceutically acceptable salts.

**4.** A method of preparation of fluorinated arachidonic acid derivatives of the formula

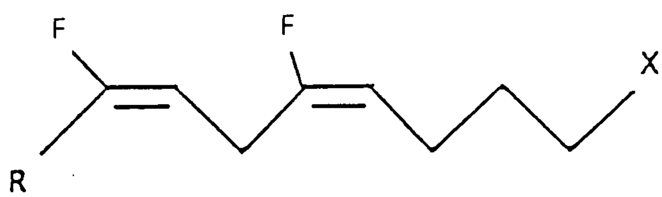

1

in which
X is a -COOH group and R is as defined in claim 1, or of a pharmaceutically acceptable salt thereof, which comprises :
a) reacting the chlorinated derivative of formula

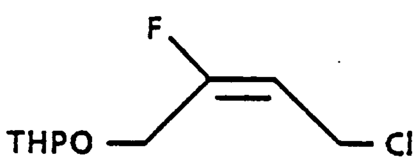

21

with N-allyl-N,N',N''-pentamethylphosphoramide to give the aldehyde of formula

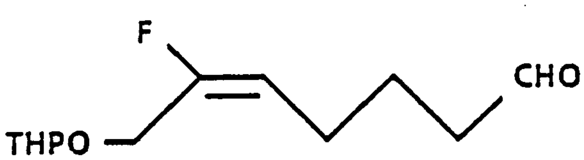

39 ;

b) reducing the aldehyde 39 to give the alcohol of formula

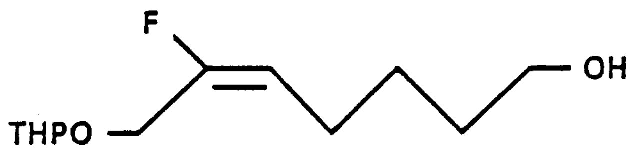

38 ;

c) treating the alcohol 38 with diphenyl-t-butyl silyl chloride and cleaving the THP protecting group to give the alcohol of formula

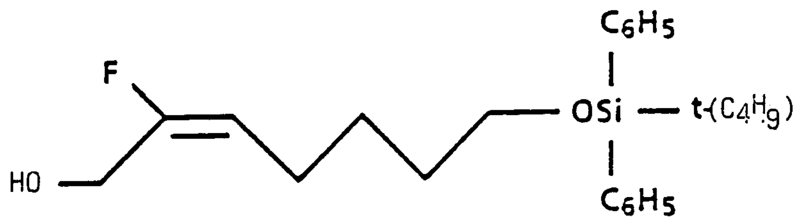

36 ;

d) converting the alcohol 36 to the corresponding bromide of formula

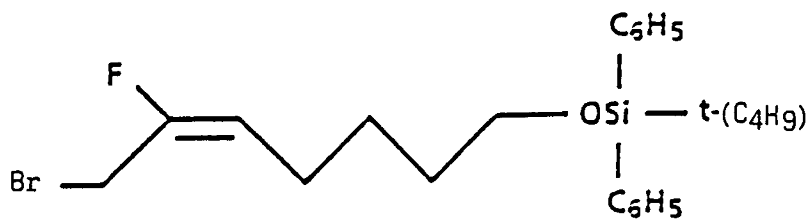

35 ;

e) treating the bromide 35 with the anion of 1,3-dithiane, hydrolizing the resulting compound and adding calcium carbonate to give the aldehyde of formula

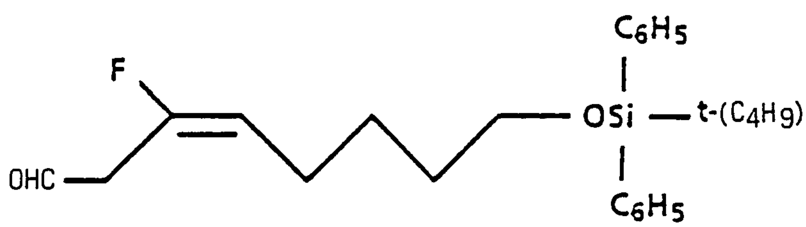

;

f) repeating steps h) to o) set forth in claim 3 starting from the aldehyde obtained above, to give the desired compound 1 or one of its pharmaceutically acceptable salts.

**5.** A method of preparation of fluorinated arachidonic acid derivatives of the formula

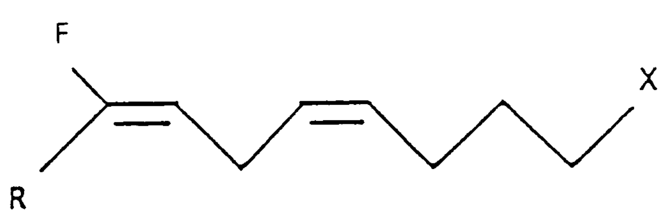

1

in which

X is a -COOH group and R is as defined in claim 1, or of a pharmaceutically acceptable salt thereof, which comprises :

a) reducing the lactone of formula

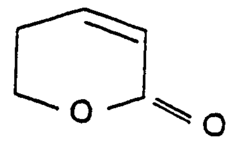

to the corresponding 2-diol ;

b) converting the diol ($HOCH_2CH_2CH=CHCH_2OH$) to the Z-1-chloro-5-OH-2-pentene and protecting the hydroxy group with a THP protecting group ;

c) treating the chloride (THPO-$CH_2CH_2CH=CHCH_2Cl$) with N-allyl-N,N',N''-pentamethylphosphoramide to give the aldehyde (THPO-$CH_2CH_2CH=CH(CH_2)_3CHO$) ;

d) reducing the aldehyde obtained above to give the alcohol (THPO-$CH_2CH_2CH=CH(CH_2)_3CH_2OH$) ;

e) treating the alcohol obtained above with diphenyl-t-butylsilyl chloride and cleaving the THP protecting group to give the alcohol ($HOCH_2CH_2CH=CH(CH_2)_3CH_2OSi(C_6H_5)_2(t\text{-}C_4H_9)$) ;

f) oxidizing the alcohol obtained above to give the aldehyde (OHC-$CH_2CH=CH(CH_2)_3CH_2OSi(C_6H_5)$-$_2(t\text{-}C_4H_9)$) ;

g) repeating steps h) to o) set forth in claim 3 to obtain the desired compound 1 or one of its pharmaceutically acceptable salts, starting from the aldehyde obtained above.

**Claims for the following Contracting State : GR**

**1.** A fluorinated arachidonic acid derivative of the formula

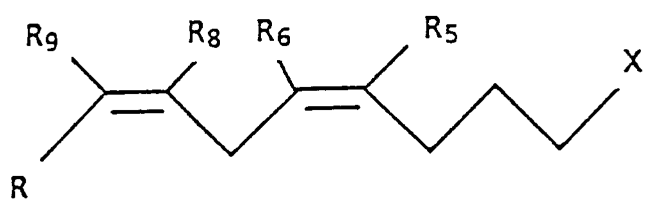

wherein

one of $R_5$ and $R_6$ is a fluoro group and the other is a hydrogen or both $R_5$ and $R_6$ individually are a hydrogen;

one of $R_8$ and $R_9$ is a fluoro group and the other is a hydrogen;

X is a C(O)OR' group wherein R' is a hydrogen, a straight chain ($C_1$-$C_6$)alkyl group, or

X is a group of the formula

-C(O)O$CH_2CH(OR'')CH_2(OR''')$ wherein R'' is a long chain fatty acid residue and wherein R''' is a hydrogen or a long chain fatty acid residue, or

X is a -C(O)$NH_2$ or -C(O)NH(OH) group, or

X is a 1H-tetrazol-5-yl group; and

R is a group of one of the structural formulae

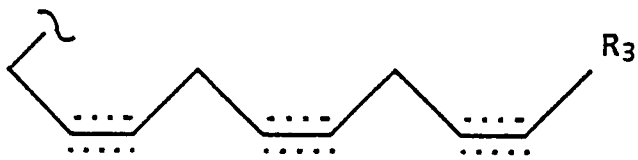

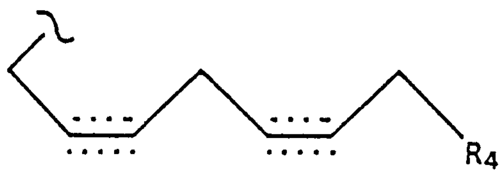

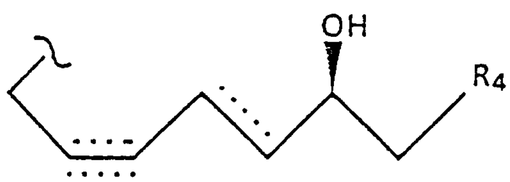

wherein

$R_3$ is a hydrogen or a straight chain ($C_1$-$C_4$)alkyl and $R_4$ is a hydrogen or a straight chain ($C_1$-$C_6$)-alkyl and wherein a dotted line indicates an optional double or triple bond or a pharmaceutically acceptable salt thereof.

**2.** A fluorinated arachidonic acid derivative of claim 1 wherein X is a $CO_2H$ group.

**3.** A fluorinated arachidonic acid derivative of one of claims 1 or 2 wherein X is a group of the formula $-C(O)OCH_2CH(OR'')CH_2(OR''')$ wherein R'' is a long chain, fatty acid residue and wherein R''' is a hydrogen or a long chain, fatty acid residue.

**4.** A fluorinated arachidonic acid derivative of one of claims 1 or 2 wherein R is a group of the structural formula

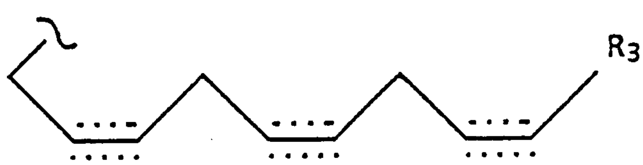

wherein

$R_3$ is a hydrogen or a straight chain ($C_1$-$C_4$)alkyl and wherein a dotted line indicates an optional double or triple bond.

**5.** A fluorinated arachidonic acid derivative of claim 4 wherein $R_3$ is an ethyl group.

**6.** A fluorinated arachidonic acid derivative of one of claims 1 or 2 wherein R is a group of the structural formula

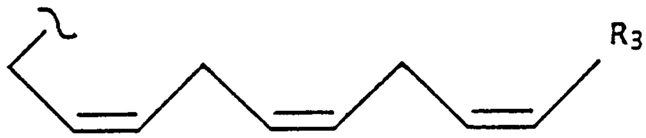

or

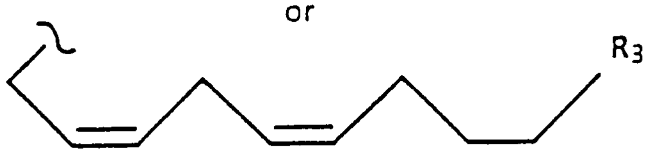

wherein

$R_3$ is a hydrogen or a straight chain $(C_1\text{-}C_4)$alkyl.

**7.** A fluorinated arachidonic acid derivative of claim 6 wherein $R_3$ is an ethyl group.

**8.** The use of fluorinated arachidonic acid derivatives according to claim 1 to 7 for the preparation of a medicine.

**9.** The use of fluorinated arachidonic acid derivative according to claims 1 to 7 for the preparation of a 5-lipoxygenase inhibitor.

**10.** The use of fluorinated arachidonic acid derivatives according to claims 1 to 7 for the preparation of an antiasthmatic.

**11.** A method for the preparation of a pharmaceutical composition useful for inhibiting 5-lipoxygenase which consists in mixing a fluorinated arachidonic acid derivative according to claims 1 to 7 with a pharmaceutically acceptable carrier.

**Patentansprüche**
**Patentansprüche für folgende Vertragsstaaten : AT, BE, CH, DE, FR, GB, IT, LI, LU, NL, SE**

**1.** Fluoriertes Arachidonsäurederivat der Formel

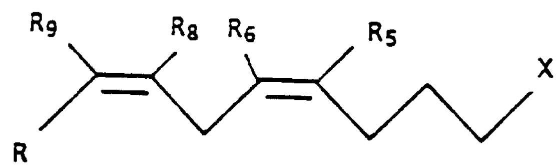

in der

einer der Reste $R_5$ und $R_6$ ein Fluoratom und der andere ein Wasserstoffatom ist oder sowohl $R_5$ als auch $R_6$ ein Wasserstoffatom bedeuten;

einer der Reste $R_8$ und $R_9$ ein Fluoratom und der andere ein Wasserstoffatom ist;

X einen C(O)OR'-Rest bedeutet, worin R' ein Wasserstoffatom oder ein geradkettiger $(C_1\text{-}C_6)$Alkylrest ist,

oder

X ein Rest der Formel $-C(O)OCH_2CH(OR'')CH_2(OR''')$ ist, in der R'' ein langkettiger Fettsäurerest ist und R''' ein Wasserstoffatom oder ein langkettiger Fettsäurerest ist,

oder

X eine $-C(O)NH_2$- oder -C(O)NH(OH)-Gruppe ist, oder

X eine 1H-Tetrazol-5-yl-Gruppe ist; und

R ein Rest einer der folgenden Strukturformeln ist,

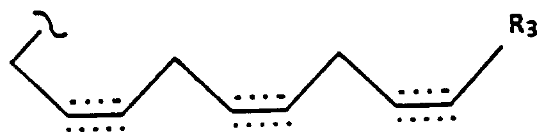

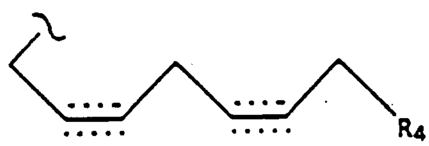

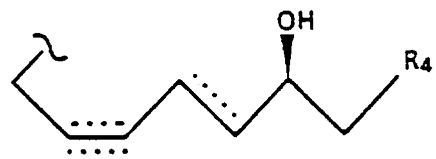

wobei $R_3$ ein Wasserstoffatom oder einen geradkettigen ($C_1$-$C_4$)Alkylrest bedeutet und $R_4$ ein Wasserstoffatom oder ein geradkettiger ($C_1$-$C_6$)Alkylrest ist, und wobei eine gepunktete Linie eine fakultative Doppel- oder Dreifachbindung anzeigt,
oder ein pharmazeutisch verträgliches Salz davon.

**2.** Fluoriertes Arachidonsäurederivat nach Anspruch 1, wobei X eine $CO_2H$-Gruppe ist.

**3.** Fluoriertes Arachidonsäurederivat nach einem der Ansprüche 1 oder 2, wobei X ein Rest der Formel $-C(O)OCH_2CH(OR'')CH_2(OR''')$ ist, in der R'' ein langkettiger Fettsäurerest ist und R''' ein Wasserstoffatom oder einen langkettigen Fettsäurerest bedeutet.

**4.** Fluoriertes Arachidonsäurederivat nach einem der Ansprüche 1 oder 2, wobei R ein Rest der folgenden Strukturformel ist

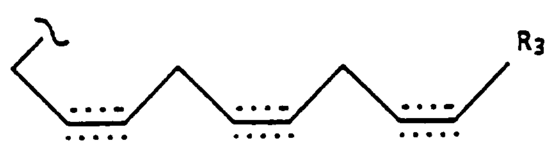

in der
$R_3$ ein Wasserstoffatom oder einen geradkettigen ($C_1$-$C_4$)Alkylrest bedeutet und wobei eine gepunktete Linie eine fakultative Doppel- oder Dreifachbindung anzeigt.

**5.** Fluoriertes Arachidonsäurederivat nach Anspruch 4, wobei $R_3$ eine Ethylgruppe ist.

**6.** Fluoriertes Arachidonsäurederivat nach einem der Ansprüche 1 oder 2, wobei R ein Rest der folgenden Strukturformel ist

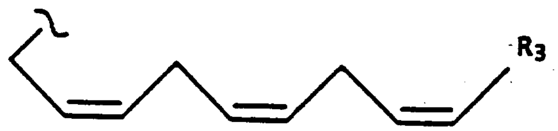

oder

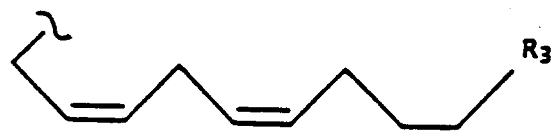

in der
$R_3$ ein Wasserstoffatom oder einen geradkettigen ($C_1$-$C_4$)Alkylrest bedeutet.

**7.** Fluoriertes Arachidonsäurederivat nach Anspruch 6, wobei $R_3$ eine Ethylgruppe ist.

**8.** Verwendung von fluorierten Arachidonsäurederivaten nach den Ansprüchen 1 bis 7 für die Herstellung eines Medikaments.

**9.** Verwendung von fluorierten Arachidonsäurederivaten nach den Ansprüchen 1 bis 7 für die Herstellung eines 5-Lipoxygenaseinhibitors.

**10.** Verwendung von fluorierten Arachidonsäurederivaten nach den Ansprüchen 1 bis 7 für die Herstellung eines Antiasthmatikums.

**11.** Arzneimittel, umfassend ein fluoriertes Arachidonsäurederivat nach den Ansprüchen 1 bis 7 in Kombination mit einem pharmazeutisch verträglichen Träger.

**Patentansprüche für folgenden Vertragsstaat : ES**

**1.** Verfahren zur Herstellung von fluorierten Arachidonsäurederivaten der Formel

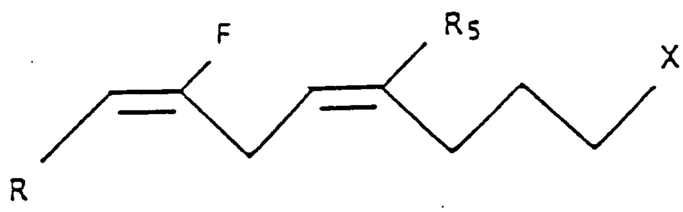

1

in der
$R_5$ ein Wasserstoffatom oder ein Fluoratom bedeutet,
X eine -COOH-Gruppe bedeutet, und
R einen Rest einer der folgenden Strukturformeln darstellt

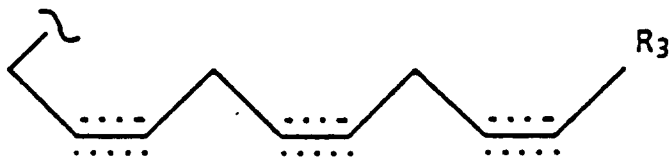

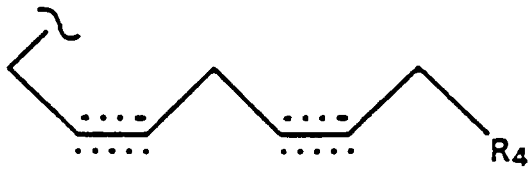

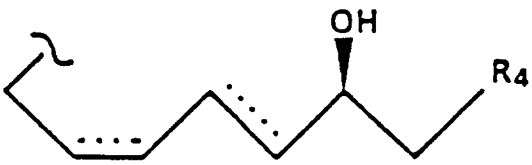

wobei
$R_3$ ein Wasserstoffatom oder einen geradkettigen ($C_1$-$C_4$)Alkylrest bedeutet und $R_4$ ein Wasserstoffatom oder ein geradkettiger ($C_1$-$C_6$)Alkylrest ist, und wobei eine gepunktete Linie eine fakultative Doppel- oder Dreifachbindung anzeigt,
oder deren pharmazeutisch verträglicher Salze, umfassend:

a) Kondensation des Ylids des fluorierten Phosphonats $(C_2H_5O)_2P(O)CHFCO_2(C_2H_5)$ mit einem Aldehyd der Formel RCHO, in der R die vorstehend angegebene Bedeutung hat, wobei man den Ester der folgenden Formel erhält

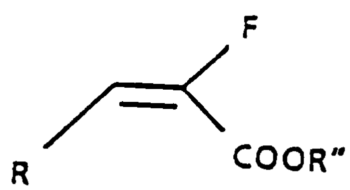

9

in der R die vorstehend angegebene Bedeutung hat und R" ein Niederalkylrest, eine Phenylgruppe oder eine Benzylgruppe ist;

b) Reduktion des Esters 9 in einem etherischen Lösungsmittel mit einem Aluminiumhydrid-Reduktionsmittel, wobei man den Alkohol der folgenden Formel erhält

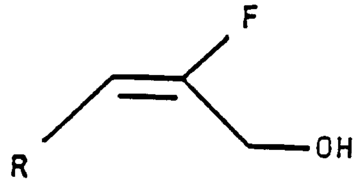

8a

in der R die vorstehend angegebene Bedeutung hat,

c) Umwandlung des Alkohols 8a in das entsprechende Halogenid der folgenden Formel

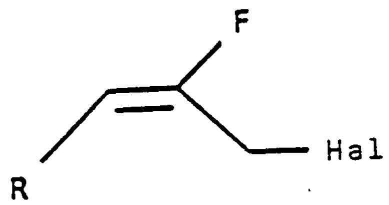

8

in der R die vorstehend angegebene Bedeutung hat und Hal ein Brom- oder Chloratom bedeutet,

d) Behandlung des Halogenids 8 mit dem Anion von 1,3-Dithian, wobei man das Dithian der folgenden Formel erhält

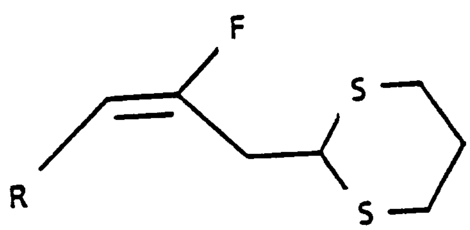

7

in der R die vorstehend angegebene Bedeutung hat,

e) Hydrolyse des Diothians 7, wobei man den Aldehyd der folgenden Formel erhält

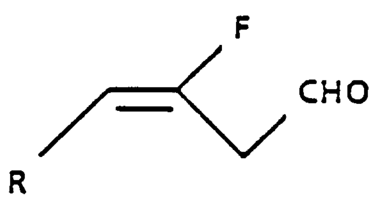

6

in der R die vorstehend angegebene Bedeutung hat,

f) Umsetzung des Aldehyds 6 mit dem Ylid von Triethylphosphonofluoracetat oder Triethylphosphonoacetat, wobei man den Ester der folgenden Formel erhält

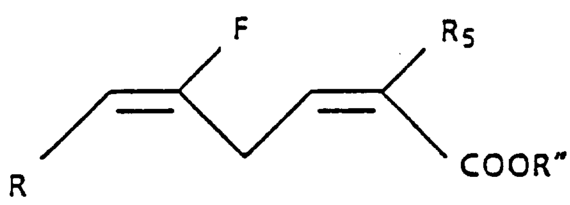

5

in der R und $R_5$ die vorstehend angegebene Bedeutung haben und R'' ein Alkylrest oder eine Benzylgruppe ist,

g) Reduktion des Esters 5 in einem etherischen Lösungsmittel mit einem Aluminiumhydrid-Reduktionsmittel, wobei man den Alkohol der folgenden Formel erhält

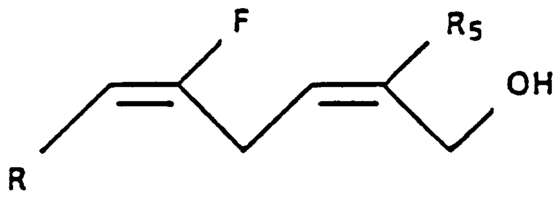

4

in der R und $R_5$ die vorstehend angegebene Bedeutung haben,

h) Umwandlung des Alkohols 4 in das entsprechende Halogenid der folgenden Formel

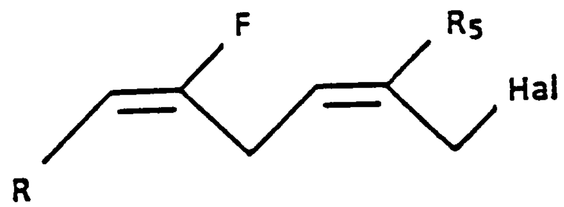

3

in der R und $R_5$ die vorstehend angegebene Bedeutung haben und Hal ein Chlor- oder Bromatom bedeutet,

i) Behandlung des Halogenids 3 mit N-Allyl-N,N',N''-Pentamethylphosphoramid, wobei man das Kondensationsprodukt der folgenden Formel erhält

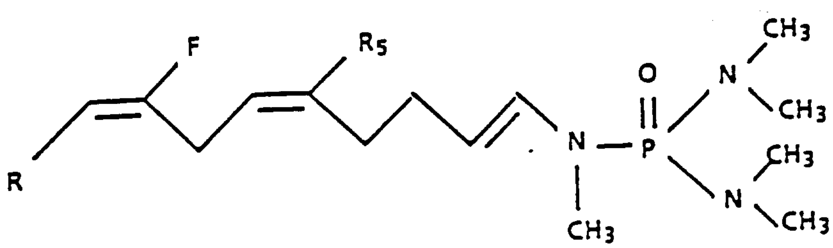

3a

in der R und $R_5$ die vorstehend angegebene Bedeutung haben,

j) Hydrolyse des Kondensationsprodukts 3a in einem schwach sauren Medium, wobei man den Aldehyd der folgenden Formel erhält

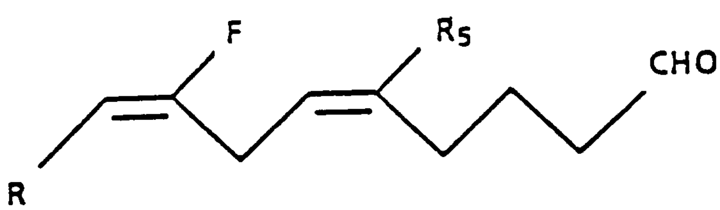

2

in der R und $R_5$ die vorstehend angegebene Bedeutung haben,

k) Oxidation des Aldehyds 2, wobei man die gewünschte Verbindung 1 erhält, und

l) gegebenenfalls Umwandlung der Verbindung 1 in eines ihrer pharmazeutisch verträglichen Salze.

**2.** Verfahren zur Herstellung von fluorierten Arachidonsäurederivaten der Formel

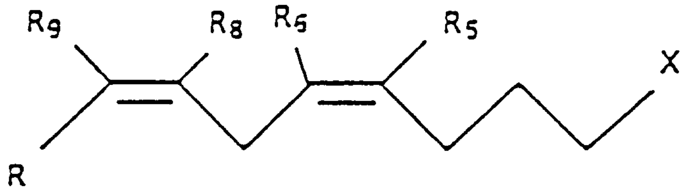

1

in der
einer der Reste $R_5$ und $R_6$ ein Fluoratom und der andere ein Wasserstoffatom ist oder sowohl $R_5$ als auch $R_6$ ein Wasserstoffatom bedeuten;
einer der Reste $R_8$ und $R_9$ ein Fluoratom und der andere ein Wasserstoffatom ist;
X einen C(O)OR'-Rest bedeutet, wobei R' ein Wasserstoffatom oder ein geradkettiger $(C_1\text{-}C_6)$Alkylrest ist,
oder
X ein Rest der Formel $-C(O)OCH_2CH(OR'')CH_2(OR''')$ ist, in der R'' ein langkettiger Fettsäurerest ist und R''' ein Wasserstoffatom oder ein langkettiger Fettsäurerest ist,
oder
X eine $-C(O)NH_2$- oder -C(O)NH(OH)-Gruppe ist, oder
X eine 1H-Tetrazol-5-yl-Gruppe ist; und
R die folgende Formel hat:

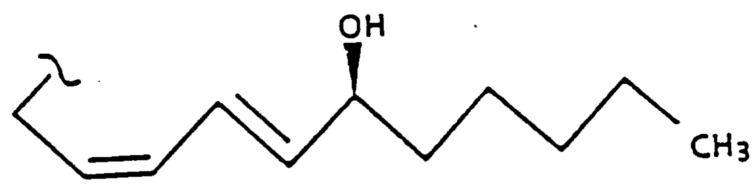

oder eines pharmazeutisch verträglichen Salzes davon, umfassend:

a) Behandlung von D-Arabinose, wobei man das Dithioacetal der folgenden Formel erhält

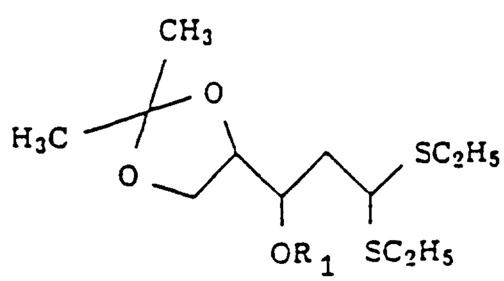

26

in der $R_1$ eine $(t\text{-}C_4H_9)Si(C_6H_5)_2$-Gruppe ist,

b) Umsetzung des Dithioacetals 26 mit Quecksilber(II)-oxid und Calciumcarbonat, wobei man den Silyloxyaldehyd der folgenden Formel erhält

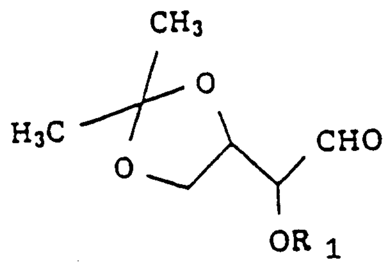

27

in der $R_1$ eine $(t\text{-}C_4H_9)Si(C_6H_5)_2$-Gruppe ist,

c) Umsetzung des Silyloxyaldehyds 27 mit dem Ylid von n-Propylbromid und Triphenylphosphin, wobei man das Silyloxyolefin der folgenden Formel erhält

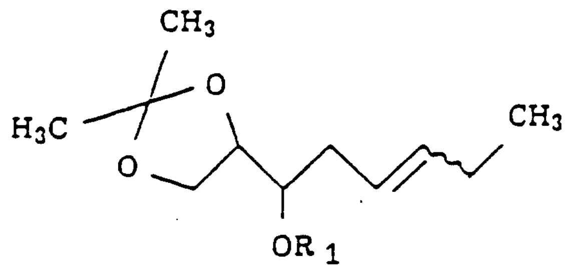

29

in der $R_1$ eine $(t\text{-}C_4H_9)Si(C_6H_5)_2$-Gruppe ist,

d) Reduktion des Silyloxyolefins 29, wobei man die Silyloxyverbindung der folgenden Formel erhält

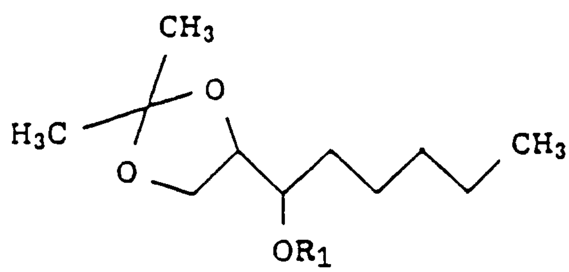

30

in der $R_1$ eine $(t\text{-}C_4H_9)Si(C_6H_5)_2$-Gruppe ist,

e) Umwandlung der Silyloxyverbindung 30 in den ungesättigten Aldehyd der folgenden Formel

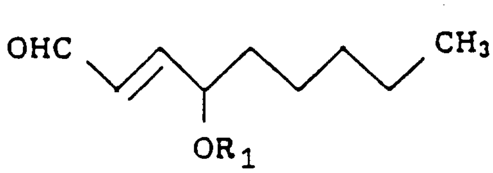

33

in der $R_1$ eine $(t\text{-}C_4H_9)Si(C_6H_5)_2$-Gruppe ist, über das Diol und die Aldehyd-Analoga,

f) Umsetzung des Aldehyds 33 mit dem Ylid des Acetonketals von 3,4-Dihydroxy-jodbutan, wobei man das Diolefinketal der folgenden Formel erhält

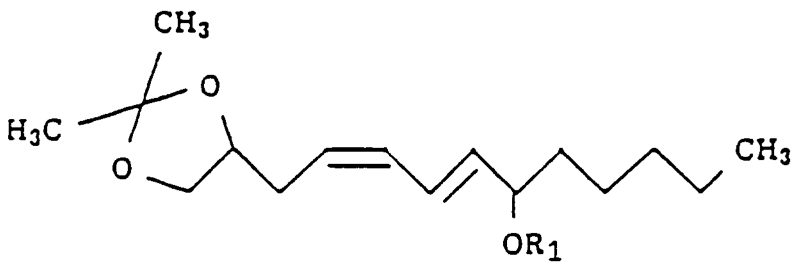

34

in der $R_1$ eine $(t\text{-}C_4H_9)Si(C_6H_5)_2$-Gruppe ist,

g) Hydrolyse und Oxidation des Diolefinketals 34, wobei man ein Silyletherderivat erhält, und Entfernung der Silylgruppe von dem Silyletherderivat, wobei man den Aldehyd der folgenden Formel erhält

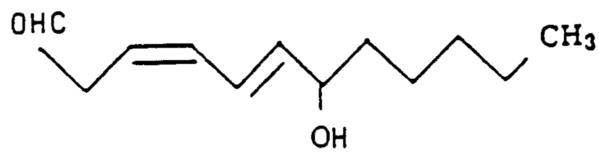

25

d.h. einen Aldehyd der Formel RCHO, in dem R die vorstehend angegebene Bedeutung hat,

h) Wiederholung der Schritte a) bis l) gemäß Anspruch 1.

**3.** Verfahren zur Herstellung von fluorierten Arachidonsäurederivaten der Formel

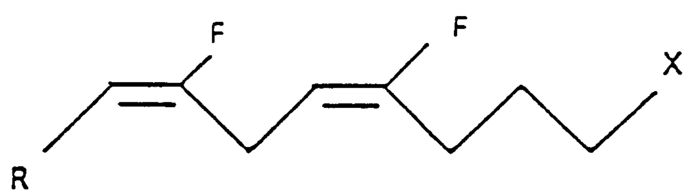

1

in der

X eine -COOH-Gruppe bedeutet, und

R die in Anspruch 1 angegebene Bedeutung hat,

oder von pharmazeutisch verträglichen Salzen davon,

umfassend:

a) Umsetzung eines Chlorderivats der folgenden Formel

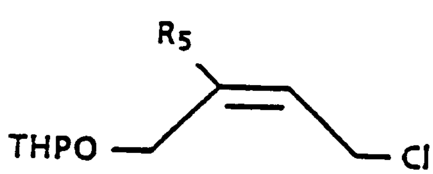

21

in der $R_5$ ein Fluoratom bedeutet, mit dem Anion von 1,3-Dithian, wobei man das Dithialanderivat der folgenden Formel erhält

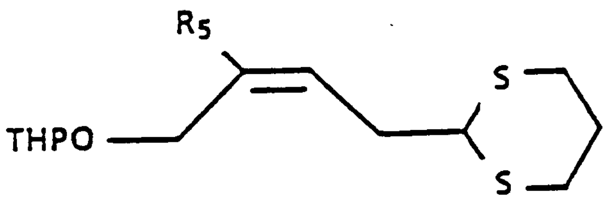

20

in der $R_5$ ein Fluoratom bedeutet,

b) Entfernung der THP-Schutzgruppe von Verbindung 20, wobei man den Alkohol der folgenden Formel erhält

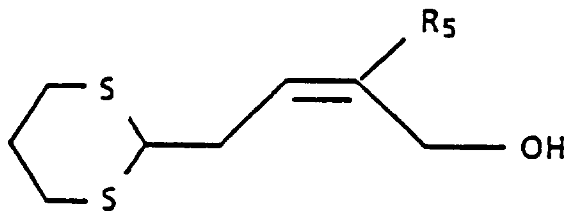

19

in der $R_5$ ein Fluoratom bedeutet,

c) Umwandlung des Alkohols 19 in das entsprechende Chlorderivat der folgenden Formel

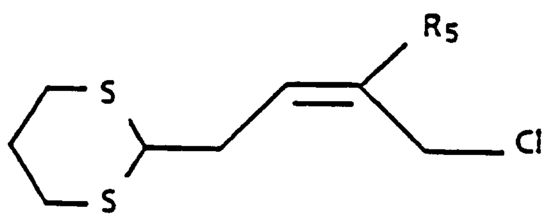

18

in der $R_5$ ein Fluoratom bedeutet,

d) Transformation der chlorierten Verbindung 18 mit N-Allyl-N,N',N''-pentamethylphosphoramid in den Aldehyd der folgenden Formel

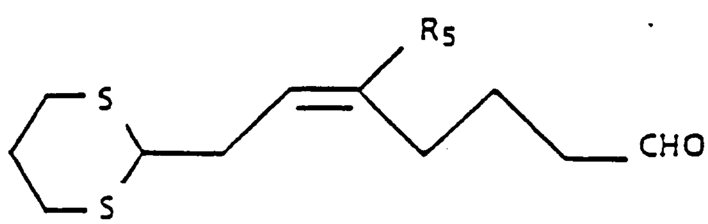

17

in der $R_5$ ein Fluoratom bedeutet,

e) Reduktion des Aldehyds 17, wobei man den Alkohol der folgenden Formel erhält

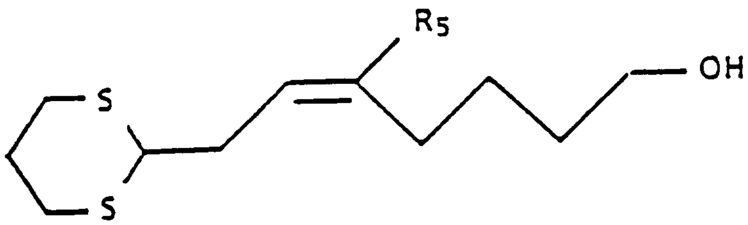

16

in der $R_5$ ein Fluoratom bedeutet,
f) Behandlung des Alkohols 16 mit t-Butyldiphenylsilylchlorid, wobei man das silylierte Dithialan der folgenden Formel erhält

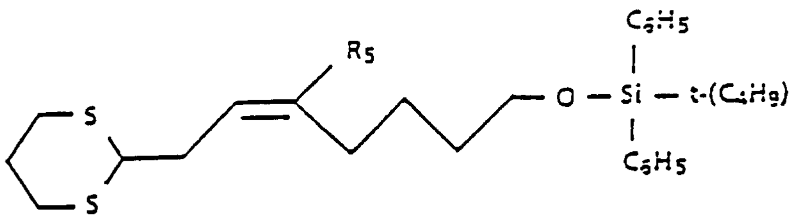

15

in der $R_5$ ein Fluoratom bedeutet,
g) Zugabe des 1,3-Dithialanderivats 15 zu einer Suspension von Trimethyloxoniumtetrafluorborat, wobei man den Aldehyd der folgenden Formel erhält

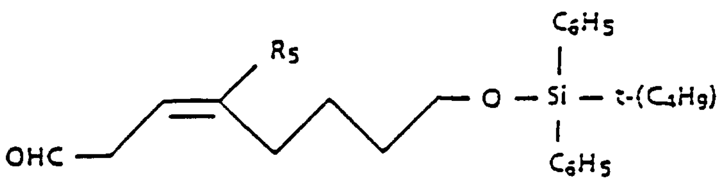

14

in der $R_5$ ein Fluoratom bedeutet,
h) Umsetzung des Aldehyds 14 mit dem Ylid von $(C_2H_5O)_2P(O)CHFCO_2C_2H_5$, wobei man den Ester der folgenden Formel erhält

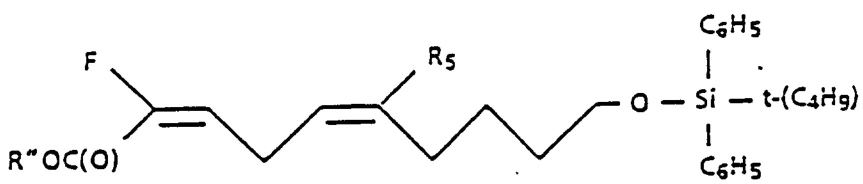

13

in der $R_5$ ein Fluoratom und R" ein Niederalkylrest, eine Phenylgruppe oder eine Benzylgruppe bedeutet,
i) Reduktion des Esters 13 in einem etherischen Lösungsmittel mit einem Aluminiumhydrid-Reduktionsmittel, wobei man den Alkohol der folgenden Formel erhält

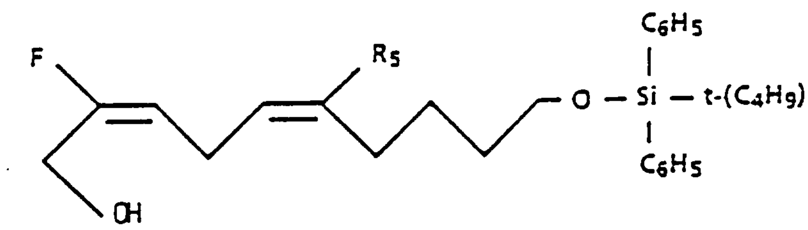

12a

in der $R_5$ ein Fluoratom bedeutet,

j) Umwandlung des Alkohols 12a in das entsprechende Halogenid der folgenden Formel

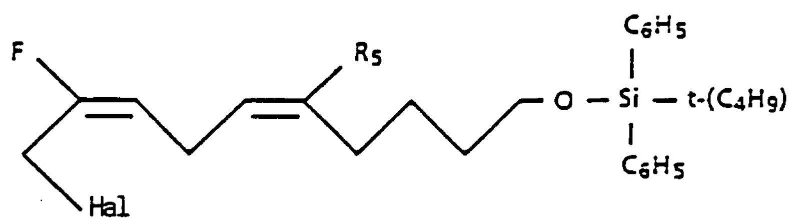

12

in der $R_5$ ein Fluoratom und Hal ein Chlor- oder Bromatom bedeutet,

k) Behandlung des Halogenids 12 mit dem Anion von 1,3-Dithian, Hydrolyse der als Zwischenprodukt erhaltenen Dithialan-Verbindung, Zugabe von Calciumcarbonat zur erhaltenen Verbindung, um als Zwischenprodukt einen Aldehyd zu isolieren und Reduktion des Aldehyds, wobei man den Alkohol der folgenden Formel erhält,

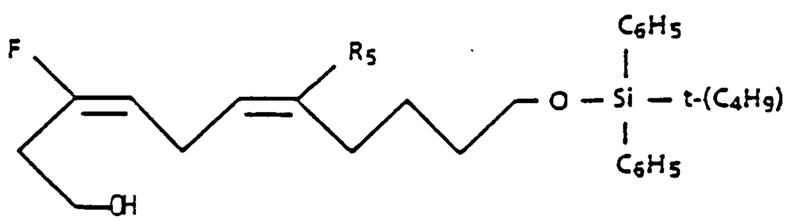

11a

in der $R_5$ ein Fluoratom bedeutet,

l) Umwandlung des Alkohols 11a in das entsprechende Halogenid der folgenden Formel

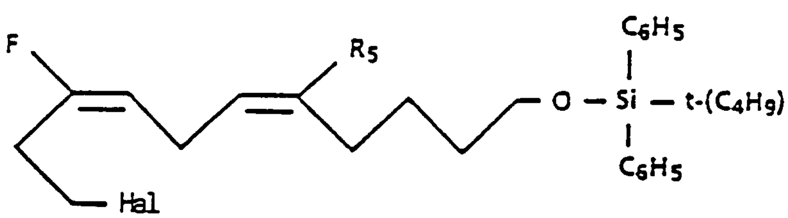

11

in der $R_5$ ein Fluoratom bedeutet und Hal ein Chlor- oder Bromatom darstellt,

m) Umsetzung des Ylids von Verbindung 11 mit dem geeigneten Aldehyd, wobei man den Alkohol der folgenden Formel erhält

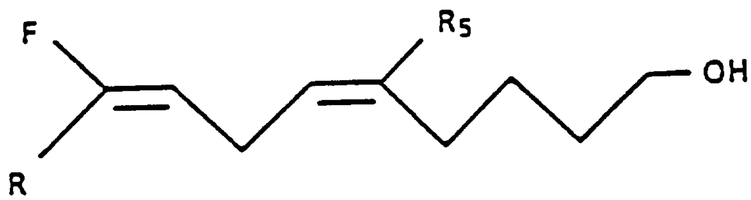

10

in der $R_5$ ein Fluoratom bedeutet und R die vorstehend angegebene Bedeutung hat,
n) Oxidation des Alkohols 10, wobei man die gewünschte Verbindung 1 erhält,
o) gegebenenfalls Umwandlung der so erhaltenen Verbindung 1 in eines ihrer pharmazeutisch verträglichen Salze.

**4.** Verfahren zur Herstellung von fluorierten Arachidonsäurederivaten der Formel

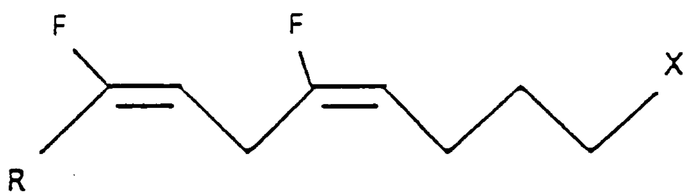

1

in der
X eine -COOH-Gruppe bedeutet und R die in Anspruch 1 angegebene Bedeutung hat,
oder eines pharmazeutisch verträglichen Salzes davon, umfassend:
a) Umsetzung des chlorierten Derivats der folgenden Formel

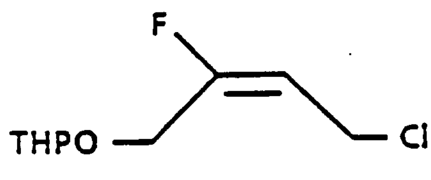

21

mit N-Allyl-N,N',N''-Pentamethylphosphoramid, wobei man den Aldehyd der folgenden Formel erhält

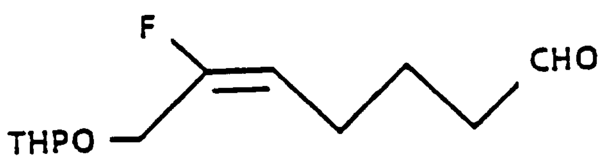

39

b) Reduktion des Aldehyds 39, wobei man einen Alkohol der folgenden Formel erhält

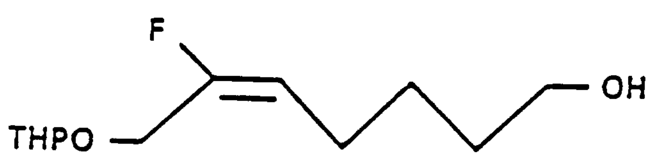

38

c) Behandlung des Alkohols 38 mit Diphenyl-t-butylsilylchlorid und Abspaltung der THP-Schutzgruppe, wobei man den Alkohol der folgenden Formel erhält

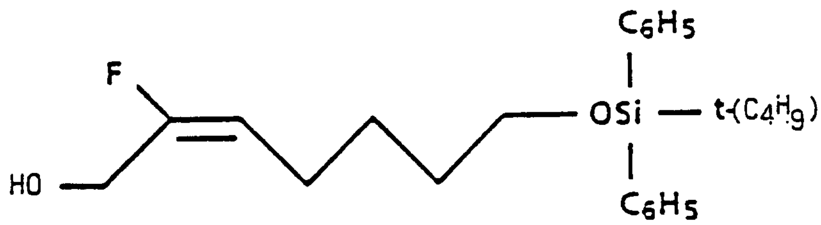

36

d) Umwandlung des Alkohols 36 in das entsprechende Bromid der folgenden Formel

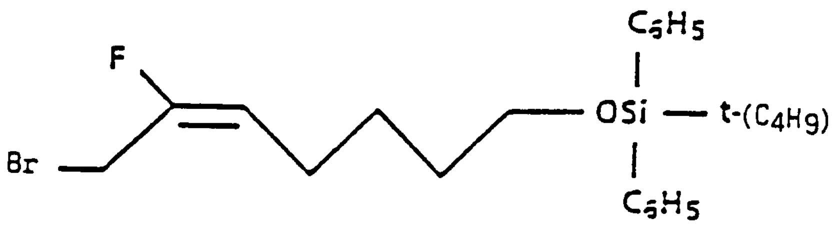

35

e) Behandlung des Bromids 35 mit dem Anion von 1,3-Dithian, Hydrolyse der erhaltenen Verbindung und Zugabe von Calciumcarbonat, wobei man den Aldehyd der folgenden Formel erhält

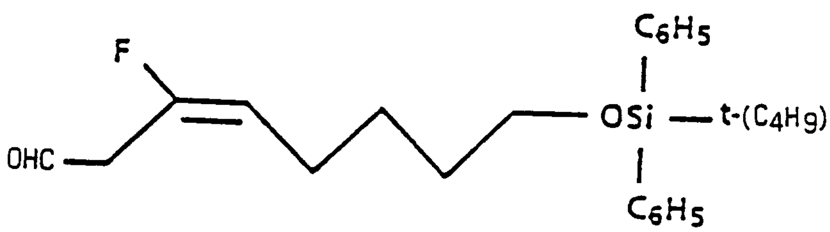

f) Wiederholung der Schritte h) bis o) gemäß Anspruch 3, ausgehend von dem vorstehend erhaltenen Aldehyd, wobei man die gewünschte Verbindung 1 oder eines ihrer pharmazeutisch vertraglichen Salze erhält.

**5.** Verfahren zur Herstellung von fluorierten Arachidonsäurederivaten der Formel

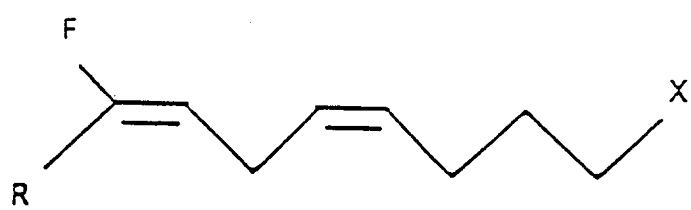

1

in der
X eine -COOH-Gruppe bedeutet und R die in Anspruch 1 angegebene Bedeutung hat,

oder eines pharmazeutisch verträglichen Salzes davon, umfassend:

a) Reduktion des Lactons der Formel

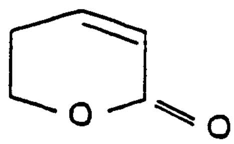

zum entsprechenden 2-Diol,

b) Umwandlung des Diols ($HOCH_2CH_2CH=CHCH_2OH$) zum Z-1-Chlor-5-OH-2-penten und Schutz der Hydroxygruppe mit einer THP-Schutzgruppe,

c) Behandlung des Chlorids (THPO-$CH_2CH_2CH=CHCH_2Cl$) mit N-Allyl-N,N',N''-pentamethylphosphoramid, wobei man den Aldehyd (THPO-$CH_2CH_2CH=CH(CH_2)_3CHO$) erhält,

d) Reduktion des vorstehend erhaltenen Aldehyds, wobei man den Alkohol (THPO-$CH_2CH_2CH=CH$-$(CH_2)_3CH_2OH$) erhält,

e) Behandlung des vorstehend erhaltenen Alkohols mit Diphenyl-t-butylsilylchlorid und Abspaltung der THP-Schutzgruppe, wobei man den Alkohol ($HOCH_2CH_2CH=CH(CH_2)_3CH_2OSi(C_6H_5)_2(t\text{-}C_4H_9)$) erhält,

f) Oxidation des vorstehend erhaltenen Alkohols, wobei man den Aldehyd (OHC-$CH_2CH=CH(CH_2)$-$_3CH_2OSi(C_6H_5)_2(t\text{-}C_4H_9)$) erhält,

g) Wiederholung der Schritte h) bis o) gemäß Anspruch 3, wobei man die gewünschte Verbindung 1 oder eines ihrer pharmazeutisch verträglichen Salze erhält, ausgehend von dem vorstehend erhaltenen Aldehyd.

**Patentansprüche für folgenden Vertragsstaat : GR**

1. Fluoriertes Arachidonsäurederivat der Formel

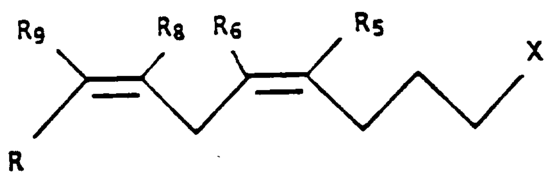

in der

einer der Reste $R_5$ und $R_6$ ein Fluoratom und der andere ein Wasserstoffatom ist oder sowohl $R_5$ als auch $R_6$ ein Wasserstoffatom bedeuten;

einer der Reste $R_8$ und $R_9$ ein Fluoratom und der andere ein Wasserstoffatom ist;

X einen C(O)OR'-Rest bedeutet, worin R' ein Wasserstoffatom oder ein geradkettiger ($C_1$-$C_6$)Alkylrest ist,

oder

X ein Rest der Formel -$C(O)OCH_2CH(OR'')CH_2(OR''')$ ist, in der R'' ein langkettiger Fettsäurerest ist und R''' ein Wasserstoffatom oder ein langkettiger Fettsäurerest ist,

oder

X eine -$C(O)NH_2$- oder -C(O)NH(OH)-Gruppe ist, oder

X eine 1H-Tetrazol-5-yl-Gruppe ist; und

R ein Rest einer der folgenden Strukturformeln ist,

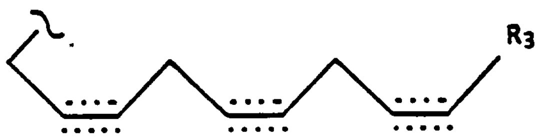

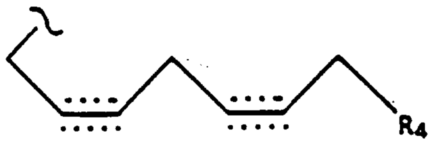

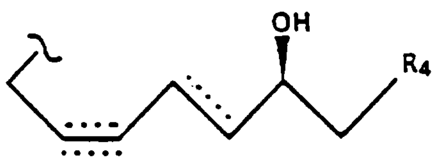

wobei $R_3$ ein Wasserstoffatom oder einen geradkettigen $(C_1\text{-}C_4)$Alkylrest bedeutet und $R_4$ ein Wasserstoffatom oder ein geradkettiger $(C_1\text{-}C_6)$Alkylrest ist, und wobei eine gepunktete Linie eine fakultative Doppel- oder Dreifachbindung anzeigt,
oder ein pharmazeutisch verträgliches Salz davon.

**2.** Fluoriertes Arachidonsäurederivat nach Anspruch 1, wobei X eine $CO_2H$-Gruppe ist.

**3.** Fluoriertes Arachidonsäurederivat nach einem der Ansprüche 1 oder 2, wobei X ein Rest der Formel $-C(O)OCH_2CH(OR'')CH_2(OR''')$ ist, in der R'' ein langkettiger Fettsäurerest ist und R''' ein Wasserstoffatom oder einen langkettigen Fettsäurerest bedeutet.

**4.** Fluoriertes Arachidonsäurederivat nach einem der Ansprüche 1 oder 2, wobei R ein Rest der folgenden Strukturformel ist

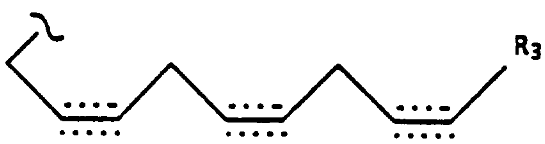

in der
$R_3$ ein Wasserstoffatom oder einen geradkettigen $(C_1\text{-}C_4)$Alkylrest bedeutet und wobei eine gepunktete Linie eine fakultative Doppel- oder Dreifachbindung anzeigt.

**5.** Fluoriertes Arachidonsäurederivat nach Anspruch 4, wobei $R_3$ eine Ethylgruppe ist.

**6.** Fluoriertes Arachidonsäurederivat nach einem der Ansprüche 1 oder 2, wobei R ein Rest der folgenden Strukturformel ist

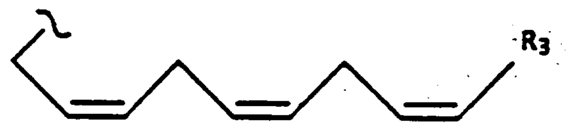

oder

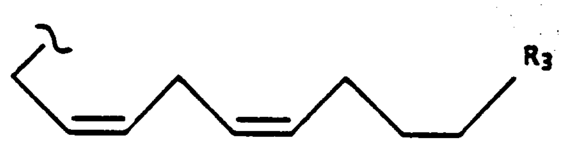

in der
$R_3$ ein Wasserstoffatom oder einen geradkettigen ($C_1$-$C_4$)Alkylrest bedeutet.

7. Fluoriertes Arachidonsäurederivat nach Anspruch 6, wobei $R_3$ eine Ethylgruppe ist.

8. Verwendung von fluorierten Arachidonsäurederivaten nach den Ansprüchen 1 bis 7 für die Herstellung eines Medikaments.

9. Verwendung von fluorierten Arachidonsäurederivaten nach den Ansprüchen 1 bis 7 für die Herstellung eines 5-Lipoxygenaseinhibitors.

10. Verwendung von fluorierten Arachidonsäurederivaten nach den Ansprüchen 1 bis 7 für die Herstellung eines Antiasthmatikums.

11. Verfahren zur Herstellung eines Arzneimittels, das nützlich ist für die Hemmung der 5-Lipoxygenase, umfassend das Mischen eines fluorierten Arachidonsäurederivats nach den Ansprüchen 1 bis 7 mit einem pharmazeutisch verträglichen Träger.

**Revendications**

**Revendications pour les Etats contractants suivants : AT, BE, CH, DE, FR, GB, IT, LI, LU, NL, SE**

1. Dérivé fluoré de l'acide arachidonique, de formule :

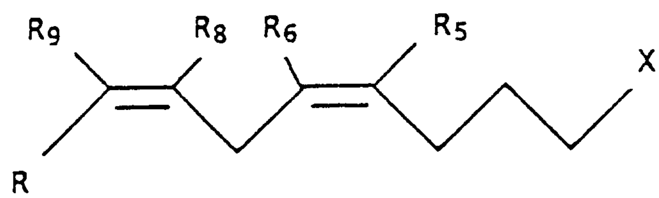

dans laquelle l'un des symboles $R_5$ et $R_6$ représente un atome de fluor et l'autre représente un atome d'hydrogène ou bien les deux symboles $R_5$ et $R_6$ représentent individuellement un atome d'hydrogène ;
l'un des symboles $R_8$ et $R_9$ représente un atome de fluor et l'autre représente un atome d'hydrogène ;
X représente un groupe C(O)OR' dans lequel R' est un atome d'hydrogène, un groupe alkyle en $C_1$-$C_6$ à chaîne linéaire, ou
X est un groupe de formule $-C(O)OCH_2CH(OR'')CH_2(OR''')$ dans laquelle R'' est un résidu d'acide gras à longue chaîne et dans laquelle R''' est un atome d'hydrogène ou un résidu d'acide gras à longue chaîne, ou
X est un groupe $-C(O)NH_2$ ou -C(O)NH(OH), ou
X est un groupe 1H-tétrazol-5-yle ; et
R est un groupe répondant à l'une des formules structurales

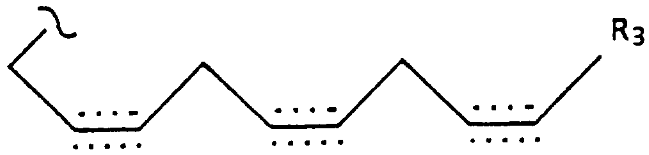

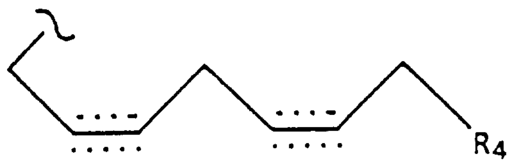

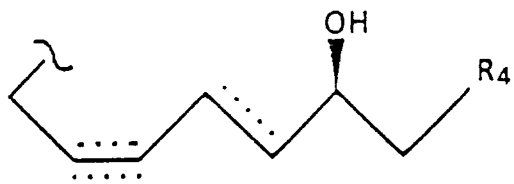

dans lesquelles $R_3$ est un atome d'hydrogène ou un groupe alkyle en $C_1$-$C_4$ à chaîne linéaire et $R_4$ est un atome d'hydrogène ou un groupe alkyle en $C_1$-$C_6$ à chaîne linéaire et dans lesquelles les pointillés représentent des doubles ou triples liaisons facultatives
ou l'un de ses sels pharmaceutiquement acceptables.

**2.** Dérivé fluoré de l'acide arachidonique selon la revendication 1, dans lequel X est un groupe $CO_2H$.

**3.** Dérivé fluoré de l'acide arachidonique selon l'une des revendications 1 ou 2, dans lequel X est un groupe de formule $-C(O)OCH_2CH(OR'')CH_2(OR''')$ dans laquelle R" est un résidu d'acide gras à longue chaîne et dans laquelle R'" est un atome d'hydrogène ou un résidu d'acide gras à longue chaîne.

**4.** Dérivé fluoré de l'acide arachidonique selon l'une des revendications 1 ou 2, dans lequel R est un groupe de formule structurale

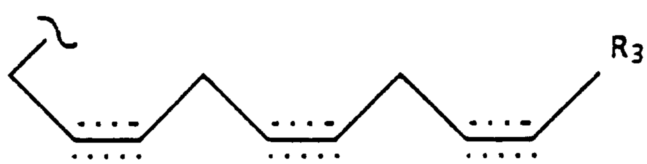

dans laquelle $R_3$ est un atome d'hydrogène ou un groupe alkyle en $C_1$-$C_4$ à chaîne linéaire et dans laquelle les pointillés représentent des doubles ou triples liaisons facultatives.

**5.** Dérivé fluoré de l'acide arachidonique selon la revendication 4, dans lequel $R_3$ est un groupe éthyle.

**6.** Dérivé fluoré de l'acide arachidonique selon l'une des revendications 1 ou 2, dans lequel R est un groupe de formule structurale :

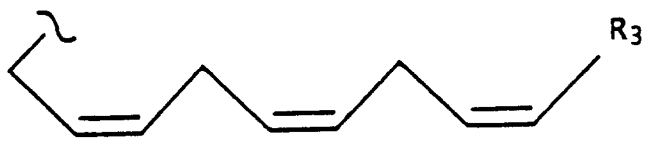

ou

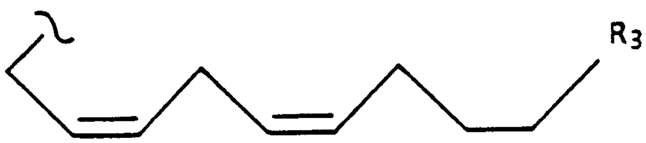

où $R_3$ est un atome d'hydrogène ou un groupe alkyle en $C_1$-$C_4$ à chaîne linéaire.

7. Dérivé fluoré de l'acide arachidonique selon la revendication 6, dans lequel $R_3$ est un groupe éthyle.

8. Utilisation des dérivés fluorés de l'acide arachidonique selon les revendications 1 à 7 pour la préparation d'un médicament.

9. Utilisation des dérivés fluorés de l'acide arachidonique selon les revendications 1 à 7 pour la préparation d'un inhibiteur de la 5-lipoxygénase.

10. Utilisation des dérivés fluorés de l'acide arachidonique selon les revendications 1 à 7 pour la préparation d'un antiasthmatique.

11. Composition pharmaceutique contenant un dérivé fluoré de l'acide arachidonique selon les revendications 1 à 7 en combinaison avec un véhicule pharmaceutiquement acceptable.

**Revendications pour l'Etat contractant suivant : ES**

1. Méthode de préparation d'un dérive fluoré de l'acide arachidonique, de formule :

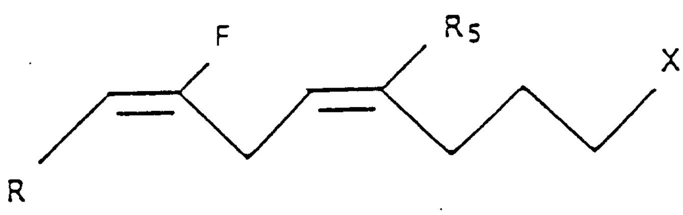

<u>1</u>

dans laquelle $R_5$ représente un atome de fluor ou un atome d'hydrogène ;
X représente un groupe -COOH ; et
R est un groupe répondant à l'une des formules structurales

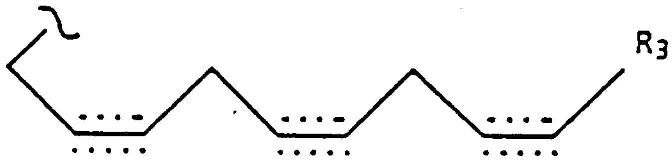

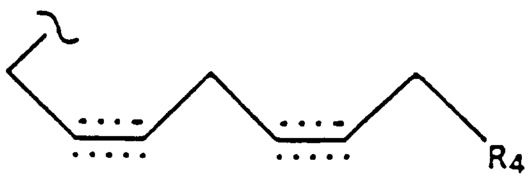

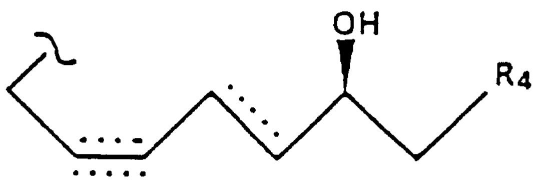

dans lesquelles $R_3$ est un atome d'hydrogène ou un groupe alkyle en $C_1$-$C_4$ à chaîne linéaire et $R_4$ est un atome d'hydrogène ou un groupe alkyle en $C_1$-$C_6$ à chaîne linéaire et dans lesquelles les pointillés représentent des doubles ou triples liaisons facultatives, ou d'un de ses sels pharmaceutiquement acceptables, qui consiste à :

a) condenser l'yure du phosphonate fluoré $(C_2H_5O)_2P(O)CHFCO_2(C_2H_5)$ avec un aldéhyde de formule RCHO dans laquelle R est tel que défini ci-dessus pour donner l'ester de formule

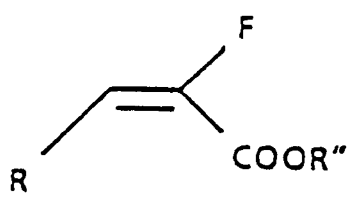

9

dans laquelle R est tel que défini ci-dessus et R" est un alkyle inférieur, un groupe phényle ou benzyle ;

b) réduire l'ester 9 dans un solvant éthéré avec un agent réducteur à base d'hydrure d'aluminium pour donner l'alcool de formule

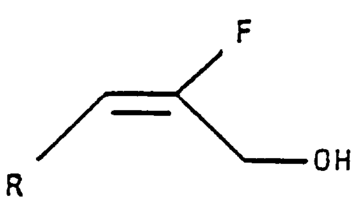

8a

dans laquelle R est tel que défini ci-dessus ;

c) convertir l'alcool 8a en l'halogénure correspondant de formule

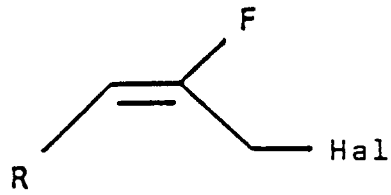

8

dans laquelle R est tel que défini ci-dessus et Hal représente un atome de brome ou de chlore ;

d) traiter l'halogénure 8 avec l'anion du 1,3-dithiane pour donner le dithiane de formule

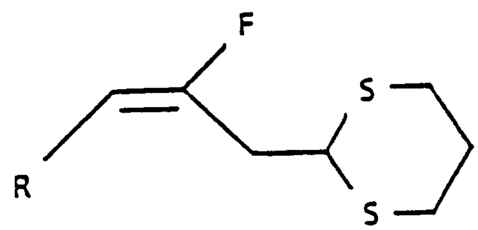

7

dans laquelle R est tel que défini ci-dessus ;

e) hydrolyser le dithiane 7 pour former l'aldéhyde de formule

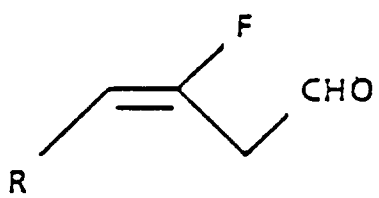

6

dans laquelle R est tel que défini ci-dessus ;

f) faire réagir l'aldéhyde 6 avec l'ylure du triéthylphosphonofluoroacétate ou du triéthylphosphonoacétate pour donner l'ester de formule

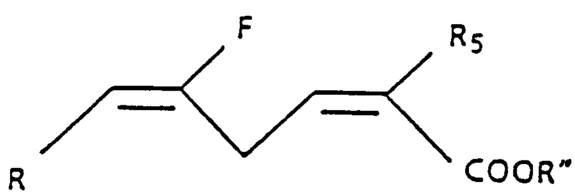

5

dans laquelle R et $R_5$ sont tels que définis ci-dessus et R'' est un groupe alkyle ou benzyle ;

g) réduire l'ester 5 dans un solvant éthéré avec un agent réducteur à base d'hydrure d'aluminium pour former l'alcool de formule

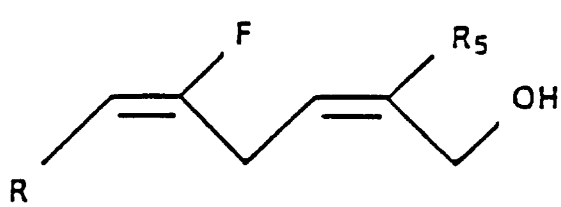

4

dans laquelle R et $R_5$ sont tels que définis ci-dessus ;

h) convenir l'alcool 4 en l'halogénure correspondant de formule

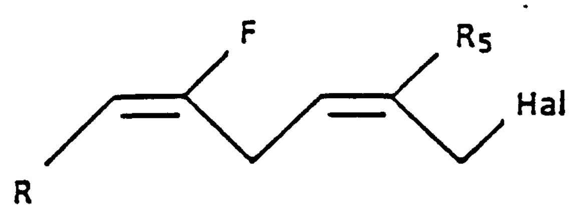

3

dans laquelle R et $R_5$ sont tels que définis ci-dessus et Hal est un atome de chlore ou de brome ;
i) traiter l'halogénure 3 avec le N-allyl-N,N',N"-pentaméthylphosphoramide pour former le produit de condensation de formule

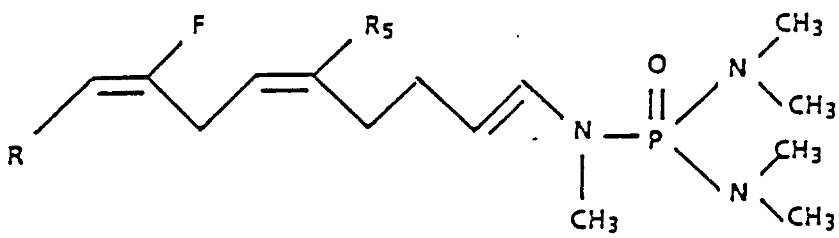

3a

dans laquelle R et $R_5$ sont tels que définis ci-dessus ;
j) hydrolyser le produit de condensation 3a en milieu acide à l'aide d'un acide doux pour donner l'aldéhyde de formule

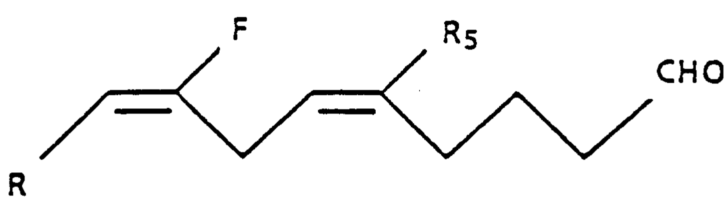

2

dans laquelle R et $R_5$ sont tels que définis ci-dessus ;
k) oxyder l'aldéhyde 2 pour obtenir le composé 1 désiré ; et
l) éventuellement, convenir le composé 1 en l'un de ses sels pharmaceutiquement acceptables.

**2.** Méthode de préparation d'un dérivé fluoré de l'acide arachidonique, de formule :

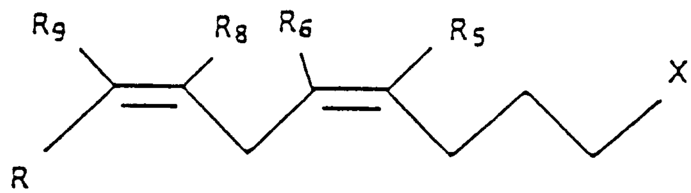

1

dans laquelle l'un des symboles $R_5$ et $R_6$ représente un atome de fluor et l'autre représente un atome d'hydrogène ou bien les deux symboles $R_5$ et $R_6$ représentent individuellement un atome d'hydrogène ;
l'un des symboles $R_8$ et $R_9$ représente un atome de fluor et l'autre représente un atome d'hydrogène ;
X représente un groupe C(O)OR' dans lequel R' est un atome d'hydrogène, un groupe alkyle en $C_1$-$C_6$ à chaîne linéaire, ou
X est un groupe de formule $-C(O)OCH_2CH(OR'')CH_2(OR''')$ dans laquelle R" est un résidu d'acide gras à longue chaîne et dans laquelle R''' est un atome d'hydrogène ou un résidu d'acide gras à longue

chaîne, ou
X est un groupe $-C(O)NH_2$ ou $-C(O)NH(OH)$, ou
X est un groupe 1H-tétrazol-5-yle ; et
R est un groupe répondant à la formule :

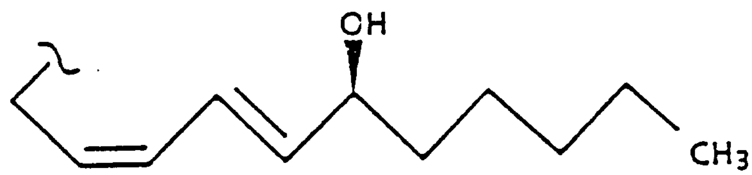

ou d'un de ses sels pharmaceutiquement acceptables, qui consiste à :

a) traiter le D-arabinose pour donner le dithioacétal de formule

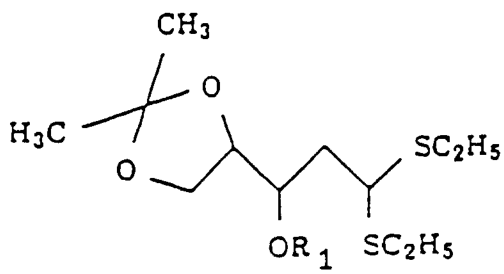

26

dans laquelle $R_1$ représente le groupe $(t\text{-}C_4H_9)Si(C_6H_5)_2$ ;

b) faire réagir le dithioacétal 26 avec de l'oxyde mercurique et du carbonate de calcium pour donner le silyloxyaldéhyde de formule

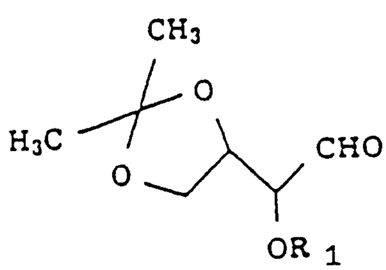

27

dans laquelle $R_1$ représente le groupe $(t\text{-}C_4H_9)Si(C_6H_5)_2$ ;

c) faire réagir le silyloxyaldéhyde 27 avec l'ylure du bromure de n-propyle et de la triphénylphosphine pour donner la silyloxyoléfine de formule

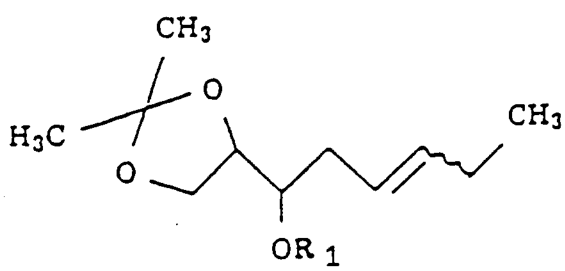

29

dans laquelle $R_1$ représente le groupe $(t\text{-}C_4H_9)Si(C_6H_5)_2$ ;

d) réduire la silyloxyoléfine 29 pour donner le composé silyloxy de formule

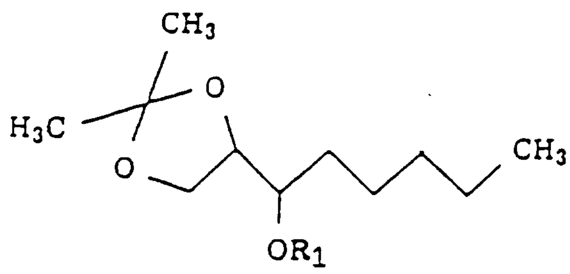

30

dans laquelle $R_1$ représente le groupe $(t\text{-}C_4H_9)Si(C_6H_5)_2$ ;
e) convertir le composé silyloxy 30 en l'aldéhyde insaturé de formule

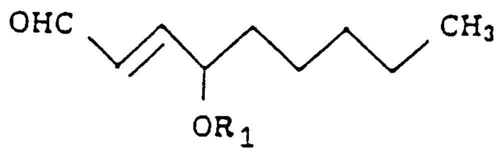

33

dans laquelle $R_1$ représente le groupe $(t\text{-}C_4H_9)Si(C_6H_5)_2$, par l'intermédiaire des analogues diol et aldéhyde ;
f) faire réagir l'aldéhyde 33 avec l'ylure de l'acétal de la cétone du 3,4-dihydroxyiodobutanepour donner l'acétal de dioléfine de formule

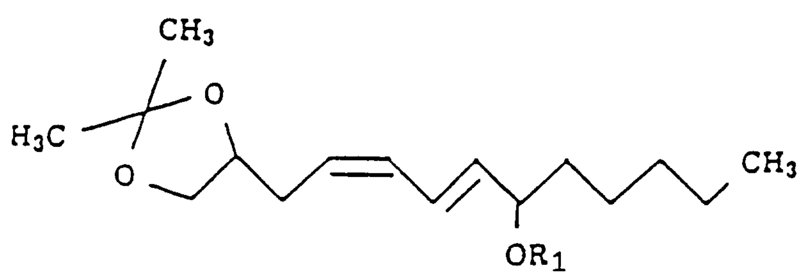

34

dans laquelle $R_1$ représente le groupe $(t\text{-}C_4H_9)Si(C_6H_5)_2$ ;
g) hydrolyser puis oxyder l'acétal de dioléfine 34 pour donner un dérivé silyléthéré, et éliminer le groupe silyl dudit dérivé silyléthéré pour donner l'aldéhyde de formule

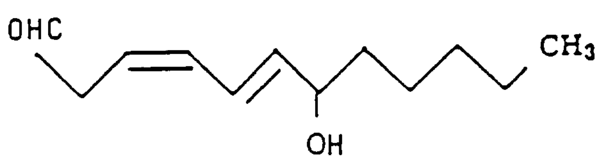

25

c'est à dire un aldéhyde de la formule RCHO dans laquelle R est tel que défini ci-dessus ;
h) répéter les étapes a) à l) décrites dans la revendication 1.

**3.** Méthode de préparation d'un dérivé fluoré de l'acide arachidonique, de formule :

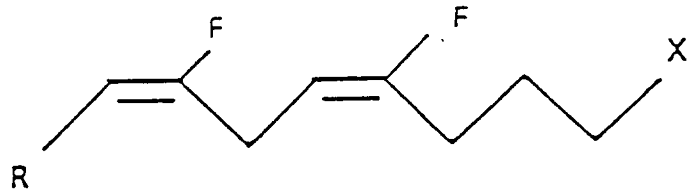

1

dans laquelle X représente un groupe -COOH et R est tel que défini dans la revendication 1, ou d'un de ses sels pharmaceutiquement acceptables, qui consiste à :

a) faire réagir un dérivé chloré de formule

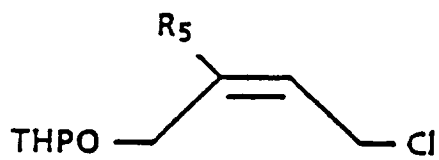

21

dans laquelle $R_5$ est un atome de fluor ,
avec l'anion du 1,3-dithiane pour donner le dérivé dithialane de formule

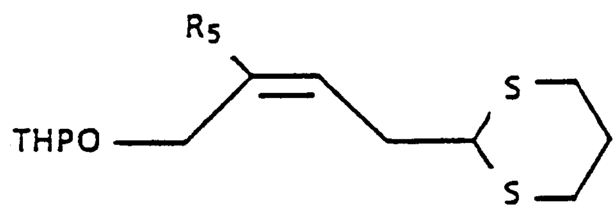

20

dans laquelle R5 est un atome de fluor ;

b) éliminer le groupe protecteur THP du composé 20 pour donner l'alcool de formule

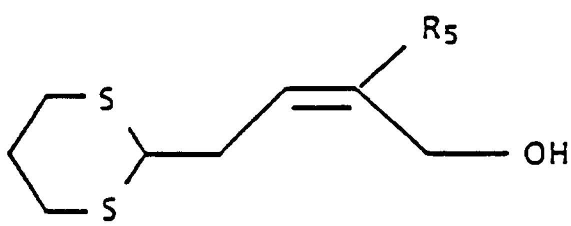

19

dans laquelle $R_5$ est un atome de fluor ;

c) convertir l'alcool 19 en le dérivé chloré correspondant de formule

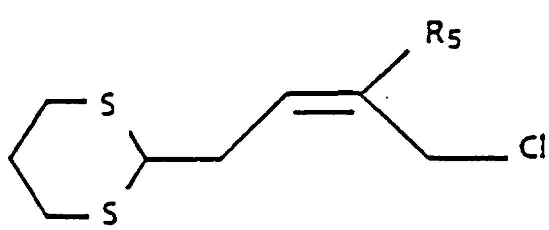

18

dans laquelle $R_5$ est un atome de fluor ;

d) transformer le composé chloré 18, à l'aide du N-allyl-N,N',N''-pentaméthylphosphoramide, en aldéhyde de formule

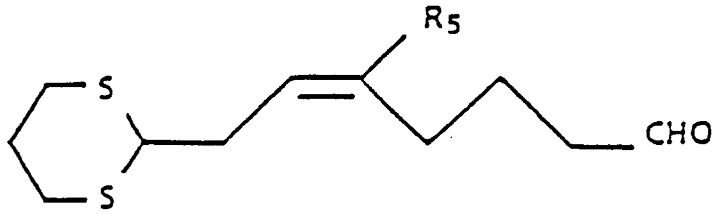

17

dans laquelle $R_5$ est un atome de fluor ;

e) réduire l'aldéhyde 17 pour donner l'alcool de formule

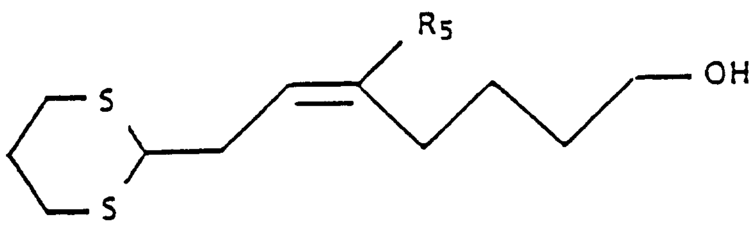

16

dans laquelle $R_5$ est un atome de fluor ;

f) traiter l'alcool 16 avec le chlorure de t-butyldiphénylsilyl pour donner le dithialane silylé de formule

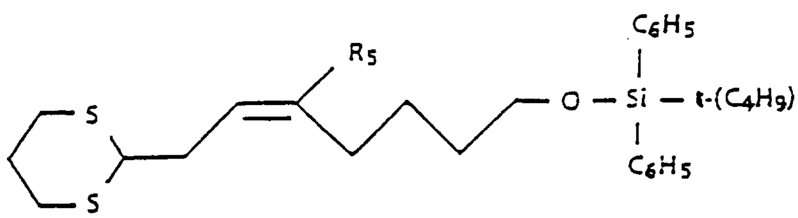

15

dans laquelle $R_5$ est un atome de fluor ;

g) ajouter le dérivé 1,3-dithialane 15 à une suspension de tétrafluoroborate de triméthyloxonium pour donner l'aldéhyde de formule

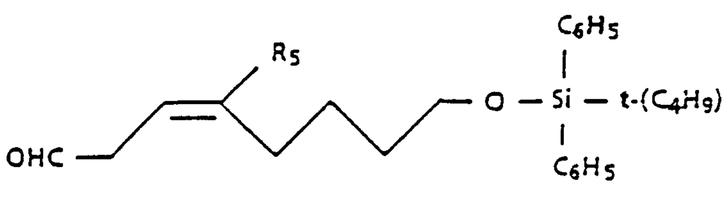

14

dans laquelle $R_5$ est un atome de fluor ;

h) faire réagir l'aldéhyde 14 avec l'ylure de $(C_2H_5O)_2P(O)CHFCO_2C_2H_5$ pour donner l'ester de formule

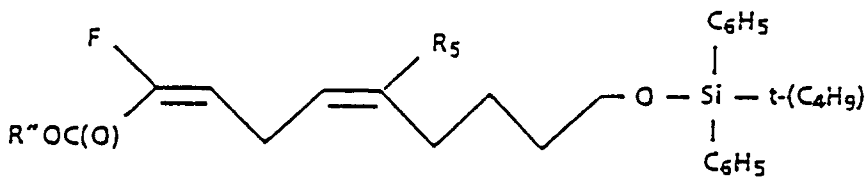

13

dans laquelle $R_5$ est un atome de fluor et R'' est un alkyle inférieur, un groupe phényle ou benzyle ;
i) réduire l'ester 13 dans un solvant éthéré avec un agent réducteur à base d'hydrure d'aluminium pour donner l'alcool de formule

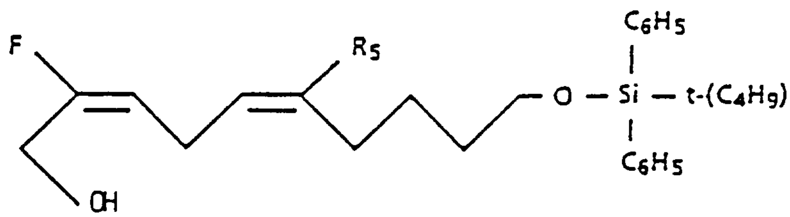

12a

dans laquelle $R_5$ est un atome de fluor ;
j) convertir l'alcool 12a en l'halogénure correspondant de formule

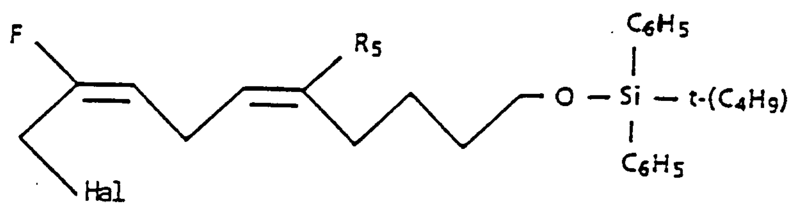

12

dans laquelle $R_5$ est un atome de fluor et Hal représente un atome de chlore ou de brome ;
k) traiter l'halogénure 12 avec l'anion du 1,3-dithiane, hydrolyser le composé dithialane intermédiaire, ajouter du carbonate de calcium au composé résultant pour isoler un aldéhyde intermédiaire et réduire ledit aldéhyde pour donner l'alcool de formule

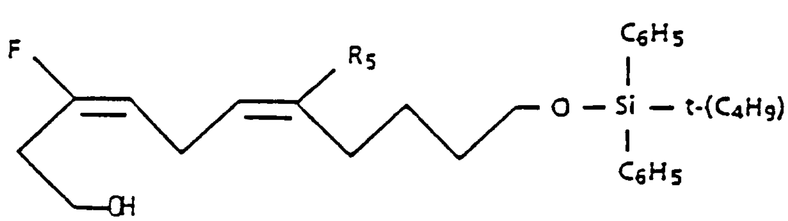

11a

dans laquelle $R_5$ est un atome de fluor ;
l) convertir l'alcool 11a en l'halogénure correspondant de formule

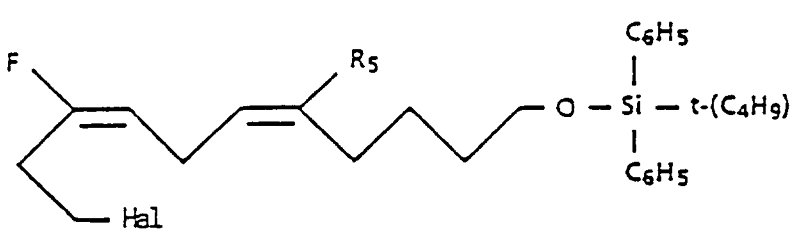

11

dans laquelle $R_5$ est un atome de fluor et Hal représente un atome de chlore de brome ;
m) faire réagir l'ylure du composé 11 avec l'aldéhyde approprié pour donner l'alcool de formule

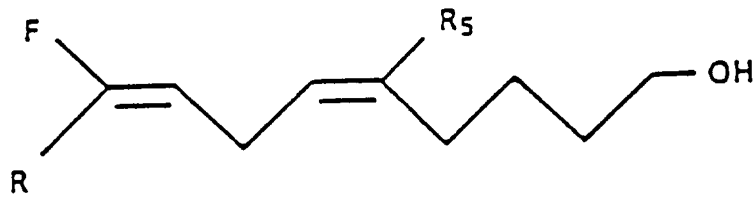

10

dans laquelle $R_5$ est un atome de fluor et R est tel que défini ci-dessus ;
n) oxyder l'alcool 10 pour obtenir le composé 1 désiré ; et
o) éventuellement, convertir le composé 1 ainsi obtenu en l'un de ses sels pharmaceutiquement acceptables.

**4.** Méthode de préparation d'un dérivé fluoré de l'acide arachidonique, de formule :

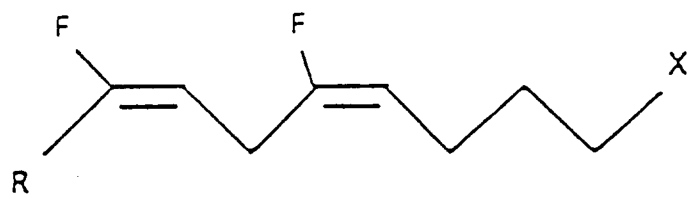

1

dans laquelle X représente un groupe -COOH et R est tel que défini dans la revendication 1, ou d'un de ses sels pharmaceutiquement acceptables, qui consiste à :
a) faire réagir le dérivé chloré de formule

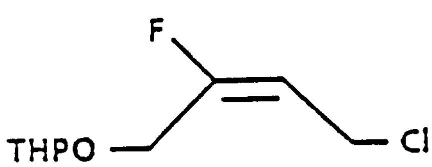

21

avec le N-allyl-N,N',N''-pentaméthylphosphoramide pour donner l'aldéhyde de formule

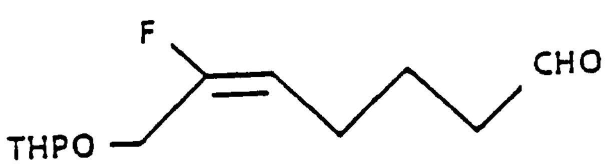

39

b) réduire l'aldéhyde 39 pour donner l'alcool de formule

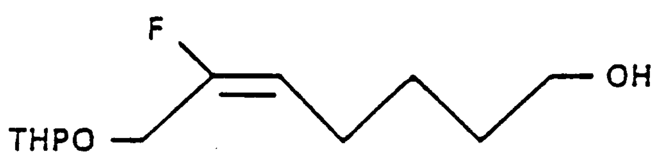

38

c) traiter l'alcool 38 avec le chlorure de t-butyldiphénylsilyl et cliver le groupe protecteur THP pour donner l'alcool de formule

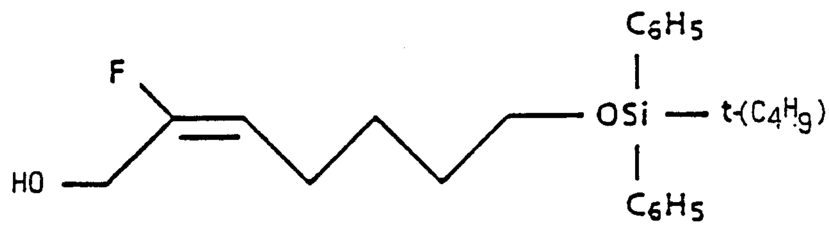

36

d) convertir l'alcool 36 en le bromure correspondant de formule

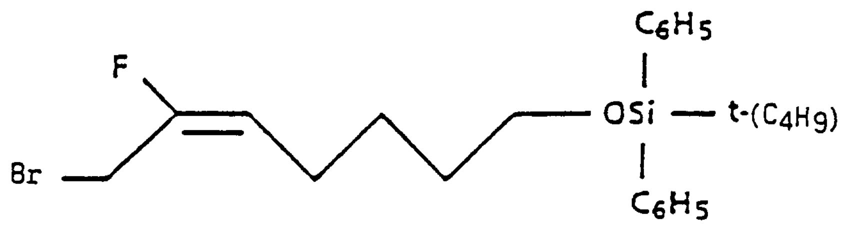

35

e) traiter le bromure 35 avec l'anion du 1,3-dithiane, hydrolyser le composé résultant et ajouter du carbonate de calcium pour donner l'aldéhyde de formule

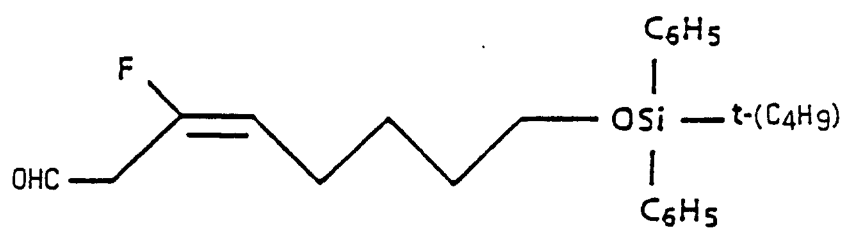

f) répéter les étapes h) à o) décrites dans la revendication 3 en partant de l'aldéhyde obtenu ci-dessus, pour obtenir le composé 1 désiré ou l'un de ses sels pharmaceutiquement acceptables.

**5.** Méthode de préparation d'un dérivé fluoré de l'acide arachidonique, de formule :

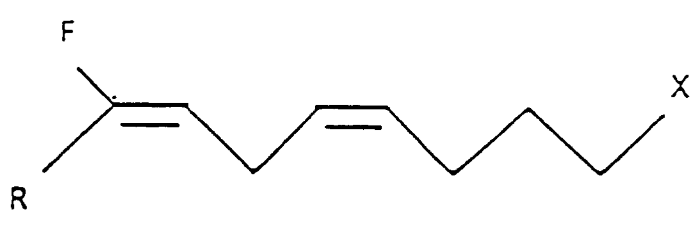

1

dans laquelle X représente un groupe -COOH et R est tel que défini dans la revendication 1, ou d'un de ses sels pharmaceutiquement acceptables, qui consiste à :

a) réduire la lactone de formule

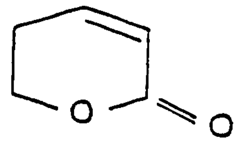

en le 2-diol correspondant ;
b) convertir le diol ($HOCH_2CH_2CH=CHCH_2OH$) en le (Z)-1-chloro-5-OH-2-pentène et protéger le groupe hydroxyle à l'aide d'un groupe protecteur THP ;
c) traiter le chlorure (THPO-$CH_2CH_2CH=CHCH_2C1$) avec le N-allyl-N,N',N"-pentaméthylphosphoramide pour donner l'aldéhyde (THPO-$CH_2CH_2CH=CH(CH_2)_3CHO$) ;
d) réduire l'aldéhyde obtenu ci-dessus pour donner l'alcool (THPO-$CH_2CH_2CH=CH(CH_2)_3CH_2OH$) ;
e) traiter l'alcool obtenu ci-dessus avec le chlorure de t-butyldiphénylsilyl et cliver le groupe protecteur THP pour donner l'alcool ($HOCH_2CH_2CH=CH(CH_2)_3CH_2OSi(C_6H_5)_2$(t-$C_4H_9$)) ;
f) oxyder l'alcool obtenu ci-dessus pour donner l'aldéhyde (OHC-$CH_2CH=CH(CH_2)_3CH_2OSi(C_6H_5)_2$-(t-$C_4H_9$)) ;
g) répéter les étapes h) à o) décrites dans la revendication 3, en partant de l'aldéhyde obtenu ci-dessus, pour obtenir le composé 1 désiré ou l'un de ses sels pharmaceutiquement acceptables.

**Revendications pour l'Etat contractant suivant : GR**

**1.** Dérivé fluoré de l'acide arachidonique, de formule :

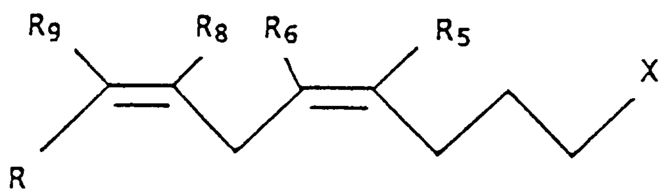

dans laquelle l'un des symboles $R_5$ et $R_6$ représente un atome de fluor et l'autre représente un atome d'hydrogène ou bien les deux symboles $R_5$ et $R_6$ représentent individuellement un atome d'hydrogène ;
l'un des symboles $R_8$ et $R_9$ représente un atome de fluor et l'autre représente un atome d'hydrogène ;
X représente un groupe C(O)OR' dans lequel R' est un atome d'hydrogène, un groupe alkyle en $C_1$-$C_6$ à chaîne linéaire, ou
X est un groupe de formule -C(O)$OCH_2CH$(OR")$CH_2$(OR"') dans laquelle R" est un résidu d'acide gras à longue chaîne et dans laquelle R"' est un atome d'hydrogène ou un résidu d'acide gras à longue chaîne, ou
X est un groupe -C(O)$NH_2$ ou -C(O)NH(OH), ou
X est un groupe 1H-tétrazol-5-yle ; et
R est un groupe répondant à l'une des formules structurales

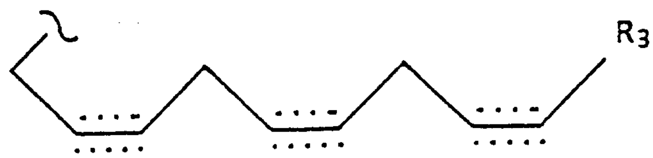

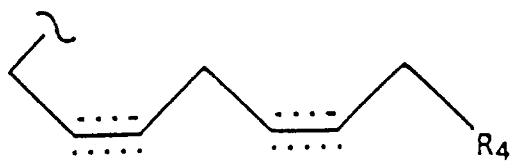

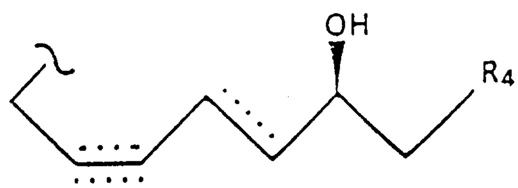

dans lesquelles $R_3$ est un atome d'hydrogène ou un groupe alkyle en $C_1$-$C_4$ à chaîne linéaire et $R_4$ est un atome d'hydrogène ou un groupe alkyle en $C_1$-$C_6$ à chaîne linéaire et dans lesquelles les pointillés représentent des doubles ou triples liaisons facultatives
ou l'un de ses sels pharmaceutiquement acceptables.

**2.** Dérivé fluoré de l'acide arachidonique selon la revendication 1, dans lequel X est un groupe $CO_2H$.

**3.** Dérivé fluoré de l'acide arachidonique selon l'une des revendications 1 ou 2, dans lequel X est un groupe de formule $-C(O)OCH_2CH(OR'')CH_2(OR''')$ dans laquelle R'' est un résidu d'acide gras à longue chaîne et dans laquelle R''' est un atome d'hydrogène ou un résidu d'acide gras à longue chaîne.

**4.** Dérivé fluoré de l'acide arachidonique selon l'une des revendications 1 ou 2, dans lequel R est un groupe de formule structurale

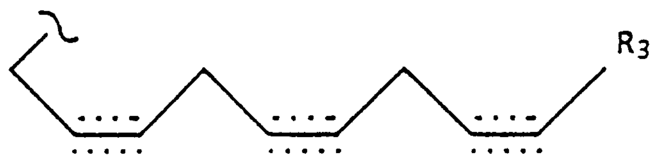

dans laquelle $R_3$ est un atome d'hydrogène ou un groupe alkyle en $C_1$-$C_4$ à chaîne linéaire et dans laquelle les pointillés représentent des doubles ou triples liaisons facultatives.

**5.** Dérivé fluoré de l'acide arachidonique selon la revendication 4, dans lequel $R_3$ est un groupe éthyle.

**6.** Dérivé fluoré de l'acide arachidonique selon l'une des revendications 1 ou 2, dans lequel R est un groupe de formule structurale :

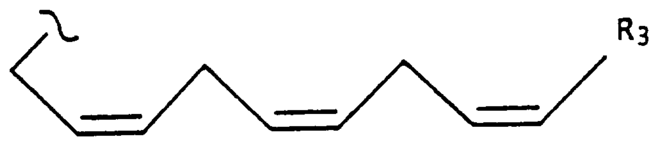

ou

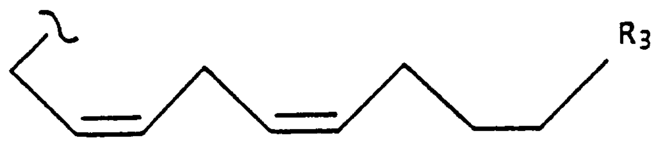

où $R_3$ est un atome d'hydrogène ou un groupe alkyle en $C_1$-$C_4$ à chaîne linéaire.

**7.** Dérivé fluoré de l'acide arachidonique selon la revendication 6, dans lequel $R_3$ est un groupe éthyle.

**8.** Utilisation des dérivés fluorés de l'acide arachidonique selon les revendications 1 à 7 pour la préparation d'un médicament.

**9.** Utilisation des dérivés fluorés de l'acide arachidonique selon les revendications 1 à 7 pour la préparation d'un inhibiteur de la 5-lipoxygénase.

**10.** Utilisation des dérivés fluorés de l'acide arachidonique selon les revendications 1 à 7 pour la préparation d'un antiasthmatique.

**11.** Méthode de préparation d'une composition pharmaceutique utile pour inhiber la 5-lipoxygénase, qui consiste à mélanger un dérivé fluoré de l'acide arachidonique selon les revendications 1 à 7 avec un véhicule pharmaceutiquement acceptable.